# EUROPÄISCHE PATENTSCHRIFT

(11) **EP 1 558 930 B1**
(45) Veröffentlichungstag und Bekanntmachung des Hinweises auf die Patenterteilung: **11.07.2007**
(21) Anmeldenummer: 02754606.8
(22) Anmeldetag: 31.05.2002
(51) Int. Cl.: G01N 33/53, C12Q 1/68, G01N 33/543

(54) **VERFAHREN ZUR BESTIMMUNG EINES ANALYTEN**
METHOD FOR DETERMINING AN ANALYTE
PROCEDE DE DETERMINATION D'UN ANALYTE

(30) Priorität: 01.06.2001 DE 10126798; 09.02.2002 DE 10205418
(43) Veröffentlichungstag der Anmeldung: 03.08.2005
(73) Patentinhaber: Arrowhead Research Corporation, Pasadena, CA 91101 (US)
(72) Erfinder: BLANK, Kerstin, 80939 München (DE); SPINNLER, Katrin, 81379 München (DE); MAI, Thao, 81927 München (DE); OESTERHELT, Filipp, 42115 Wuppertal (DE); GILBERT, Ilka, 80686 München (DE); STEIPE, Boris, Toronto, Ontario M6G 3K3 (CA); GAUB, Hermann, 83727 Neuhaus (DE); ALBRECHT, Christian, 81371 München (DE); BRINK, Gunnar, D-80339 München (DE)
(74) Vertreter: Eisenführ, Speiser & Partner
(86) Internationale Anmeldenummer: PCT/EP2002/006003
(87) Internationale Veröffentlichungsnummer: WO 2002/099423

(56) Entgegenhaltungen:
- WO-A-99/36577
- DE-A- 19 808 884
- MOY V T ET AL: "INTERMOLECULAR FORCES AND ENERGIES BETWEEN LIGANDS AND RECEPTORS" SCIENCE, AMERICAN ASSOCIATION FOR THE ADVANCEMENT OF SCIENCE,, US, Bd. 266, 1994, Seiten 257-259, XP002911770 ISSN: 0036-8075

## Beschreibung

Die vorliegende Erfindung betrifft Verfahren zur Bestimmung von Analyten und zum qualitativen und/oder quantitativen Nachweis von Analyten in einer Probe, insbesondere Assays in Form von hochparallelisierten Proteinarrays.

Es existieren zahlreiche Assays, um Substanzen in Proben nachzuweisen oder ihre Konzentration zu bestimmen. Nach einer Methode werden beispielsweise spezifische Antikörper auf einer Oberfläche immobilisiert, die Probenzusammensetzung wird dann mit der Antikörper-dekorierten Oberfläche in Kontakt gebracht, schließlich wird bestimmt, wie viel der nachzuweisenden Probensubstanz an der Oberfläche gebunden ist. Der Nachweis kann dabei prinzipiell auf zwei verschiedene Arten erfolgen. Entweder wird die gesamte Probenzusammensetzung selbst direkt markiert oder es erfolgt eine sekundäre Markierung durch ein Molekül, das spezifisch die nachzuweisende Probensubstanz erkennt.

Eine wesentliche Weiterentwicklung von Ligandenassays bestand in der Einführung von hochparallelen Mikroarrays von Nucleinsäuren oder Proteinen oder anderen als Rezeptor dienenden Molekülen. Eine Übersicht gibt Ekins, Clinical Chemistry, 1998, Vol. 44:9, 2015-2030. Ein Proteinarray beispielsweise unterscheidet sich von herkömmlichen Immuntests durch die hochgradige Parallelisierung einzelner Nachweise und die damit einhergehende Miniaturisierung und Verkürzung der durchschnittlichen Testdauer. Der Array besteht aus einer Vielzahl verschiedener Rezeptoren (Antikörper, Peptide, andere Scaffold-Proteine, DNA, RNA, RNA-Aptamere, usw.), die jeweils auf einem eigenen kleinen Bereich immobilisiert sind, wobei die Bereiche in einem Raster auf der Oberfläche verteilt sind. Gibt man eine Probezusammensetzung auf den Array, kommt es zur spezifischen Bindung der Probemoleküle an die jeweiligen Rezeptoren.

Die Bindung von Probemolekülen auf verschiedenen Bereichen kann direkt oder indirekt nachgewiesen werden. Beim direkten Nachweis wird das Probenmolekül mit einer Markierung versehen. Als Beispiele sind die kovalente Anbindung von Fluorophoren, die Markierung der Probenzusammensetzung durch radioaktive Isotope oder im Fall von Aptamer-Chips die Anfärbung der gesamten an der Oberfläche gebundenen Proteinsubstanz mit Coomassie-Blue oder Silver-Staining zu nennen. Ein großer Nachteil hierbei ist der Umstand, dass alle in der Probe enthaltenen Substanzen, nachzuweisende Substanzen wie auch andere Stoffe, markiert werden und damit jede unspezifisch gebundene oder an der Oberfläche adsorbierte Substanz zum Hintergrund des Auslesesignals beiträgt.

Beim indirekten Nachweis wird ein zweiter (sekundärer) Rezeptor, hier als Sonde bezeichnet, gegen die am immobilisierten (primären) Rezeptor spezifisch gebundene Probensubstanz gerichtet. Man spricht hier auch vom Sandwichformat. Diese Sonde ist zum Beispiel durch Fluorophore oder Radioaktivität markiert oder mit einem Reporterenzym versehen (ELISA-Microarray). Hierbei wird nur noch die gesuchte Probensubstanz aus einer Probenzusammensetzung markiert. Das Signal-zu-Hintergrund-Verhältnis des Auslesesignals ist damit deutlich besser als bei der direkten Markierung. Allerdings tritt auch hier das Problem der unspezifischen Bindung auf, da nicht unterschieden werden kann, ob die Sonde spezifisch gebunden oder unspezifisch adsorbiert wurde.

Die Sonde kann für ein bestimmtes Protein selektiv sein oder aber ein Epitop binden, das allen Probemolekülen gemeinsam ist. Ein Beispiel für eine nicht-selektive Sonde ist die Verwendung eines Anti-His-Tag-Antikörpers. Dieser kommt zum Einsatz, wenn es sich bei den Probemolekülen um rekombinante Proteine einer cDNA-Bank handelt, die mit einem His-Tag fusioniert wurden. Als Beispiel siehe Büssow et al. Nucleic Acids Research, 1998, Vol. 26, No. 21, 5007-5008. Als weiteres Beispiel ist der "one side Assay"-ELISA zu nennen, bei dem Proteine als Rezeptoren an die Unterlage gebunden sind und in der Probenzusammensetzung spezifische Antikörper zu diesen Proteinen nachgewiesen werden sollen. Die Markierung der an den Proteinen gebundenen Antikörper erfolgt dann über sekundäre Antikörper-Enzym Komplexe, welche spezifisch an die konstanten Domänen der gebundenen Antikörper binden. Das Enzym erzeugt dann einen Farbstoff oder einen Fluorophor, welcher das eigentliche Detektionssignal gibt. (Siehe: Joos et al. Electrophoresis, 2000, Vol 21, 2641-2650).

Die nicht-selektive indirekte Markierung ist jedoch in den Fällen, in denen die Probesubstanzen kein gemeinsames Epitop besitzen, z.B. beim Nachweis beliebiger nichtrekombinanter Proteine, nicht anwendbar. In diesem Fall muss jedes nachzuweisende Probemolekül mit einer eigenen selektiven Sonde nachgewiesen werden.

Um die Spezifität eines Nachweistests zu erhöhen, können selektive Sonden zur indirekten Markierung verwendet werden.

Ein wichtiger Vorteil der Verwendung selektiver Sonden zur indirekten Markierung besteht in der Erhöhung der Spezifität des gesamten Tests. Unspezifisch an den Rezeptoren gebundene Probesubstanzen, d.h. Liganden, die zu anderen Rezeptoren gehören, werden durch die selektive Sonde nicht markiert. Auf einem Array werden bei einer selektiven Markierung genauso viele Sonden benötigt wie Rezeptoren auf dem Array vorhanden sind. Dies bringt allerdings mehrere Probleme mit sich:

Die relative Konzentration der zu einem bestimmten Liganden bzw. Rezeptor gehörenden Sonde nimmt mit zunehmender Anzahl der verwendeten Sonden ab, die Anzahl der Sonden, die nicht spezifisch binden können, aber dennoch zum Hintergrund durch Adsorption und/oder unspezifische Bindung beitragen, nimmt dagegen zu.

Unspezifisch, d.h. nicht am spezifischen Rezeptor und damit am falschen Ort gebundene Probesubstanzen werden dabei genauso wie spezifisch gebundene markiert, da auf jedem einzelnen Rezeptorbereich jede Sonde vorhanden ist. Dies führt zu falsch-positiven Ergebnissen.

Ebenso wird Probesubstanz, die irgendwo auf der Oberfläche, statt an ihrem spezifischen Ort adsorbiert ist, markiert. Dadurch kann es auch zu falsch-positiven Ergebnissen kommen.

Weiterhin ist für jeden Liganden eine durch die gegebene Affinität der Sonde vorgegebene minimale Konzentration an Sonde notwendig, die nicht unterschritten werden kann. Durch die hohe Parallelisierung und die damit verbundene große Zahl an verschiedenen Sonden steigt auch die Gesamtkonzentration der Sonden an. Eine Erhöhung der Sondenkonzentration führt aber wiederum zu einem stark erhöhten Hintergrundsignal durch Adsorption und unspezifische Bindung auf jedem einzelnen Rezeptorbereich.

Ein weiterer Störfaktor, der bei einer hohen Parallelisierung von Nachweisen bzw. der zunehmenden Anzahl nachzuweisender Substanzen auf einem Chip an Bedeutung gewinnt, ist die Kreuzreaktivität. Mit der Anzahl der nachzuweisenden Analyten bzw. ihrer Rezeptoren steigt auch die Wahrscheinlichkeit, dass eine Sonde mit anderen als den für sie bestimmten Analyten reagiert oder dass Analyten an anderen, als den ihnen zugehörigen Rezeptoren "hängen bleiben".

Durch die verschiedenen unspezifischen Wechselwirkungen und die Adsorption von Analyten und Sonden kommt es zu einem starken Hintergrundsignal und damit einem niedrigen Verhältnis von Signal/Hintergrund und damit auch zu ungünstigen Nachweisbedingungen, so dass in geringer Konzentration vorliegende Moleküle bereits nicht mehr nachgewiesen werden können. Dies führt zu falsch-negativen Ergebnissen.

Weiterhin kann es durch die unerwünschte Kreuzreaktivität und unspezifische Bindung zu "falsch-positiven" Nachweisen bei der Bindung von unerwünschten Substanzen der Probe an Rezeptormolekülen kommen.

Um dieses Problem zu überwinden, wurde bereits vorgeschlagen, die einzelnen Reaktionen auf einem Protein-Chip in einzelnen Vertiefungen durchzuführen, so dass sich die verschiedenen Reaktionslösungen und Rezeptoren bzw. Liganden und Sonden nicht vermischen können. Ein solcher Testaufbau ist jedoch zu aufwändig, um von praktischem Interesse zu sein.

So wird ein Assay unter Verwendung eines Mikroarrays z.B. in WO 97/43447 beschrieben. Dazu wird auf einem Chip eine Vielzahl von Vertiefungen angeordnet, die über Transferelemente gefüllt werden können. Auf diese Weise ist es möglich, dass auf einem Chip eine Vielzahl von Vertiefungen gleichzeitig mit vielen verschiedenen Reagenzien bzw. Proben gefüllt werden. Außerdem ist es auch möglich, mit Hilfe einer Art von Stempel auf einer Oberfläche mit einem vorbestimmten Muster eine Vielzahl von Substanzen anzuordnen. Über eine kleine Walze kann dann ein Reagenz auf die Transferelemente aufgebracht werden. Auf diese Weise kann auf dem Chip direkt eine Reaktion der einzelnen Substrate mit einem Reagenz stattfinden. Das Dosieren von Substanzen in einzelne Vertiefungen ist aber sehr aufwändig. Außerdem können beispielsweise durch die Walze oder andere Übertragselemente Substanzen verschleppt und dadurch Substrate kontaminiert werden.

Weiterhin ist aus EP-A 0 865 323 die Herstellung eines Chips bekannt, mit dem biologische Moleküle in der richtigen Orientierung auf einer Oberfläche adsorbiert oder immobilisiert werden können. Dazu werden die Moleküle unter Verwendung von mit ihnen spezifisch bindefähigen Substanzen und eines Stempels von einer Oberfläche auf eine andere übertragen, so dass sie dann auf der Übertragsfläche wiederum die richtige Orientierung haben. Der Chip, auf dem die Moleküle in der richtigen Orientierung vorhanden sind, kann dann für die üblichen Tests eingesetzt werden.

EP-A 09 62 759 beschreibt eine Methode zur Messung von Bindungskräften, die auf der Verformung eines elastischen Stempels beruht. Der mit einer Probesubstanz beschichtete Stempel wird mit einer Oberfläche in Kontakt gebracht, die mit einer Sonde beschichtet ist. Beim Wieder-Ablösen des Stempels von der Oberfläche können Trennkräfte gemessen werden, die der Identifikation des Analyten dienen können. Dieses Verfahren eignet sich zur Messung von Kräften zwischen einzelnen Molekülen, nicht aber für parallelisierte Tests, bei denen an vielen Molekülen gleichzeitig Bindungskräfte bestimmt bzw. verglichen werden sollen. Außerdem ist bei der Methode von EP-A 09 62 759 mit kooperativen Effekten zu rechnen, d.h. die Ereignisse sind nicht unabhängig von der Zahl der gleichzeitig mit Zugkraft belasteten Bindungen.

Ein Verfahren unter Anwendung eines AFM wird in US-A 59 92 226 beschrieben. Hier wird eine Probe statt auf einer flachen Oberfläche, wie es für AFM-Messungen üblich ist, zwischen einem spitz auslaufenden Vorsprung und der AFM-Spitze gebunden. Auch dieses Verfahren eignet sich nicht zur Parallelisierung der Messungen.

Aufgabe der vorliegenden Erfindung war es nun, ein Verfahren zur Bestimmung eines, bevorzugt aber vieler Analyten in einer Probe bereitzustellen, mit dem Analyten mit höherer Empfindlichkeit nachgewiesen werden können. Weiterhin sollte ein Verfahren zur Verfügung gestellt werden, das für den Anwender einfach und ohne aufwändige Geräte durchführbar ist, das keine speziellen Instrumente mit störanfälligen Teilen erfordert und auch von Personal ohne Spezialausbildung durchgeführt werden kann.

Diese Aufgabe wird gelöst mit dem erfindungsgemäßen Verfahren zur Bestimmung eines Analyten in einer Probe. Das Verfahren ist dadurch gekennzeichnet, dass
man eine Zusammensetzung bzw. Probe, die möglicherweise den zu bestimmenden Analyten enthält, mit einer ersten Oberfläche (= Unterlage) in Kontakt bringt, so dass der möglicherweise in der Probe enthaltene Analyt an die Unterlage gebunden wird;
eine zweite Oberfläche (= Stempel), an die eine Sonde, die an den Analyten binden kann, gebunden ist, an die Unterlage annähert, so dass Sonde und Analyt interagieren können, wobei die Bindung der Sonde an den Analyten und die Bindung des Analyten an die Unterlage unter einer von außen angelegten Zugkraft stabiler ist, als die Bindung der Sonde an den Stempel;
Stempel und Unterlage voneinander trennt; und
bestimmt, ob die Sonde an die Unterlage gebunden ist und/oder wieviel Sonde an die Unterlage gebunden ist.

Mit dem erfindungsgemäßen Verfahren ist es möglich, viele verschiedene Substanzen unter Verwendung eines Chips nachzuweisen, bei dem die Empfindlichkeit gegenüber bekannten Verfahren erhöht ist, da am jeweiligen "Bestimmungsort" die lokale Konzentration der Substanzen, die miteinander reagieren sollen, optimiert ist und die Menge an unspezifischen Bindungen, die den Nachweis stören könnten, minimiert. Bei steigender Sondenkonzentration werden gleichzeitig die bei anderen Verfarhen relevanten Probleme der Kreuzreaktivität und unspezifischen Bindungen vermieden. Dieser Vorteil erhöht sich noch bei steigender Parallelisierung.

Zur Beschreibung der vorliegenden Erfindung werden einige Begriffe verwendet, die im Folgenden definiert werden.

Der Begriff "Analyt" bezieht sich auf alle Arten von Molekülen, Substanzen oder Materialien, die in einer Probe nachgewiesen werden sollen. In Betracht kommen hier alle Moleküle oder Komplexe, ob organisch, anorganisch oder mit organischen und anorganischen Anteilen, die mindestens zwei spezifisch bindefähige Stellen aufweisen. Analyten werden in der vorliegenden Beschreibung auch als Probensubstanzen oder Probemoleküle bezeichnet.

Die Bezeichnung "Bestimmung" wird hier sowohl für den qualitativen als auch den quantitativen Nachweis eines Analyten verwendet und kann die Bestimmung der Menge und/oder Konzentration des Analyten umfassen. Umfasst von diesem Begriff sind auch die Identifizierung und/oder jegliche Charakterisierung eines Analyten anhand physikalischer Parameter.

Der Begriff "Rezeptor" soll im Rahmen der vorliegenden Erfindung jede Substanz oder jedes Molekül bezeichnen, das für einen Analyten eine molekulare Erkennungsstelle aufweist und den Analyten in spezifischer Weise binden kann.

Der Begriff "Sonde" soll im Rahmen der vorliegenden Erfindung jede Substanz oder jedes Molekül bezeichnen, das für einen Analyten eine molekulare Erkennungsstelle aufweist und den Analyten in spezifischer Weise binden kann und darüberhinaus eine Markierung mit dem Analyten in Verbindung bringt. Dazu kann eine Markierung an dem Sondenmolekül direkt gebunden sein oder aber das Sondenmolekül kann mit einem weiteren Molekül, insbesondere einem Sensorkomplex, verbunden sein, bevorzugt so, dass der Teil des Sensorkomplexes, der ggf. abgetrennt wird, markiert ist.

Unter einem "Sensorkomplex" soll eine Kombination aus mindestens zwei bindefähigen, bevorzugt spezifisch bindefähigen Molekülen verstanden werden, die bei Anwendung einer Zugkraft getrennt werden können, wobei die Kombination so ausgewählt wird, dass die Zugkraft, die zu ihrer Trennung notwendig ist, unter den gegebenen Versuchsbedingungen größer ist als die Zugkraft, die zur Trennung von unspezifisch gebundenen Molekülen notwendig ist, aber kleiner ist als die Zugkraft, die zur Trennung des spezifisch gebundenen Analyten von seinen Bindepartnern oder zur Abtrennung der immobilisierten, z.B. kovalent gebundenen Rezeptoren notwendig ist.

Das erfindungsgemäße Verfahren wird eingesetzt, um einen bzw. bevorzugt mehrere Analyten in einer Probe qualitativ oder quantitativ nachzuweisen. Die Probe oder Zusammensetzung, die möglicherweise den bzw. die zu bestimmenden Analyten enthält, auch Probesubstanz genannt, kann verschiedenster Art sein. In der Regel handelt es sich um Lösungen oder Suspensionen. Es können Zelllysate oder -homogenate sowie Gewebeextrakte oder -homogenate eingesetzt werden. Es kann sich auch um zu analysierende Körperflüssigkeiten wie Blut, Urin, Liquor, Lymphe usw. handeln. Die Zusammensetzung kann verdünnt worden sein, ebenso können geeignete Puffersubstanzen/-lösungen oder Salze zugesetzt worden sein. Die Ionenstärke und der pH-Wert der Zusammensetzung sind nicht besonders eingeschränkt, vorzugsweise liegen die Werte in einem Bereich, der die Bindung der Probe bzw. des Analyten an die Sonde nicht stark beeinträchtigt. Bevorzugte pH-Bereiche sind 1 bis 13, bevorzugter 3 bis 11, am bevorzugtesten 4 bis 10. Die Zusammensetzung kann einen einzelnen Analyten enthalten, in der Regel sind jedoch zwei oder mehrere verschiedene zu bestimmende Analyten in der Zusammensetzung bzw. Probe enthalten. Ein Vorteil des erfindungsgemäßen Verfahrens besteht darin, dass in einer Probe gleichzeitig mehrere verschiedene Analyten bestimmt werden können.

Die Art des zu bestimmenden Analyten ist nicht besonders eingeschränkt. Es können Moleküle, Molekülgruppen, Ionen oder Komplexe sein. Es können Monomere, Dimere oder Polymere sein. Üblicherweise handelt es sich um organische Verbindungen, vorzugsweise um Biomoleküle oder Biopolymere, wobei auch Kleinmoleküle denkbar sind. Bevorzugte Analyten sind Proteine, Peptide, Nucleinsäuren, Kohlenhydrate, Lipide oder auch metallhaltige Verbindungen und andere Biopolymere. Diese Analyten können Modifikationen aufweisen, insbesondere chemische Modifikationen. Es ist auch möglich, dass unterschiedliche Arten von Molekülen miteinander verbunden sind. Denkbar ist auch, dass natürlicherweise nicht vorkommende Verbindungen oder Fragmente davon mit einem der biologischen Moleküle verbunden sind. Insbesondere ist das erfindungsgemäße Verfahren geeignet für Proben, die mehrere Analyten enthalten, z.B. Körperflüssigkeiten, in denen verschiedene Arten von Molekülen und Molekülkomplexen nachgewiesen werden sollen.

Am bevorzugtesten handelt es sich bei dem bzw. den zu bestimmenden Analyten um Proteine, Polypeptide oder Peptide, sowie Nucleinsäuren. Diese können natürlicherweise vorkommende Moleküle sein, aber auch Fragmente, Abwandlungen, Konjugate und/oder Derivate davon. Die Proteine, Polypeptide oder Peptide können aus einer natürlichen Quelle stammen oder rekombinant hergestellt worden sein oder chemisch synthetisiert worden sein. Sie können in nativer oder in nicht-nativer Form, beispielsweise denaturiert, vorliegen. Die Proteine, Polypeptide oder Peptide können posttranslationale Modifikationen aufweisen, sie können glycosyliert, phosphoryliert, acyliert und/oder amidiert sein. Sie können ein "tag" enthalten, beispielsweise eine Peptidsequenz, die als Epitop von einem Antikörper erkannt wird. Bekannte tag-Sequenzen sind 6xHis-tag, FLAG-tag, myc-tag, HA-tag, usw. Weitere tags sind dem Fachmann bekannt. Auch nicht-peptidische tags können enthalten sein. Proteine als Analyten können mehrere Untereinheiten umfassen, es können Homo- oder Heterooligomere aus Polypeptiduntereinheiten sein.

Zur Durchführung des erfindungsgemäßen Verfahrens werden der bzw. die in einer Probe zu bestimmende(n) Analyt(en) mit zwei mit dem jeweiligen Analyten spezifisch bindefähigen Bindepartnern in Kontakt gebracht, wobei einer der Bindepartner auf einer ersten Oberfläche immobilisiert oder immobilisierbar ist, während der andere Bindepartner auf einer zweiten Oberfläche immobilisiert oder immobilisierbar ist. Die Auswahl der Bindepartner und ihre Immobilisierung sowie der Ort ihrer Immobilisierung sind in breitem Rahmen variabel. Wenn im folgenden die Ausdrücke "Analyt" bzw. "Analyten" verwendet werden, so soll dies jeweils, wenn nicht ausdrücklich anders definiert, sowohl die Einzahl als auch die Mehrzahl nachzuweisender Substanzen umfassen.

Ein Bindepartner (Rezeptor oder Sonde) wird auf einer ersten Oberfläche immobilisiert, die im Folgenden der Einfachheit halber als Unterlage bezeichnet wird, ohne dass dadurch festgelegt sein soll, dass diese Oberfläche die untere Lage bildet. Die als Unterlage bezeichnete Oberfläche kann ebenso die obere Lage bei Durchführung des Verfahrens bilden. Die zweite Oberfläche wird der Einfachheit halber als Stempel bezeichnet.

Das Material, aus dem die beiden Oberflächen bzw. Unterlage und Stempel bestehen, kann gleich oder verschieden sein und eine der beiden oder beide Oberflächen können in geeigneter Weise beschichtet sein. Bevorzugt ist entweder eine Oberfläche aus einem steifen Material und die andere aus einem elastischen Material gebildet oder beide Oberflächen sind aus elastischem Material, sodass sie sich beim Inkontaktbringen genau aneinander anpassen können und damit ein optimaler Kontakt der Bindepartner möglich ist. Die Unterlage und/oder der Stempel können beispielsweise aus Glas, Polydimethylsiloxan (PDMS), Nylon, Polystyrol oder anderen Kunststoffen gebildet werden. Bevorzugt wird mindestens eine der beiden Oberflächen aus einem elastischen Material hergestellt, bevorzugt einem elastischen Kunststoff. Besonders bevorzugt wird mindestens eine Oberfläche aus einem Siloxan, insbesondere Polydimethylsiloxan, gebildet. Es sind verschiedene andere flexible Materialien oder Mischungen davon möglich. Ein anderes mögliches Material ist Polyacrylamidgel, dessen elastische Eigenschaften durch das Molekulargewicht und den Vernetzungsgrad den experimentellen Bedürfnissen angepasst werden können. Die Oberfläche kann aus einem einzigen Material, einem Gemisch von Materialien oder auch einem System von Elementen aus einem oder verschiedenen Materialien aufgebaut sein.

Auf den Oberflächen können jeweils funktionelle Gruppen vorhanden sein oder nachträglich eingeführt werden. So kann unter anderem Glas verwendet werden, dessen Oberfläche derivatisiert ist, um Bindungsgruppen für die Immobilisierung eines Bindepartners bereitzustellen. Als Beispiel kann hier silanisiertes Glas genannt werden.

Die Oberflächen, auf denen die Bindepartner immobilisiert werden, können irgendeine Geometrie aufweisen, die es zulässt, sie miteinander in Kontakt zu bringen. In einer bevorzugten Ausführungsform bilden sowohl Unterlage als auch Stempel ebene Flächenbereiche, die in der Größe übereinstimmen. In einer anderen Ausführungsform ist die Oberfläche dreidimensional ausgebildet, z.B. als Zylinder, Walze etc. Zur Durchführung des erfindungsgemäßen Verfahrens ist es nur wichtig, das beide Oberflächen korrespondierende Flächenabschnitte aufweisen oder aber einander kongruent überdeckende Flächenabschnitte aufweisen, an denen jeweils Bindungspartner gebunden sind, und dass sich auf die Flächen eine Zugkraft ausüben lässt. Inbetracht kommen daher auch Teilchen im Nano- oder Mikromaßstab, z.B. Mikro- oder Nanopartikel.

Um unspezifische Wechselwirkungen der Analyten und Sonden mit der Unterlage zu minimieren, kann die Oberfläche von Unterlage und/oder Stempel bevorzugt durch die Anbindung von Proteinsubstanzen oder Polymeren, besonders bevorzugt durch die Beschichtung mit Polyethylenglycol, in an sich bekannter Weise passiviert werden.

Mindestens eine der beiden Oberflächen, in der Regel der Stempel, besitzt mechanische Eigenschaften, die einen guten Kontakt mit der anderen Oberfläche, in der Regel der Unterlage, über die gesamte Fläche ermöglichen. Der Begriff "guter Kontakt" beinhaltet den richtigen Abstand zwischen Unterlage und Stempel, der gewährleistet, dass die gebundenen Analyten und Sonden sich nah genug aneinander befinden, dass sie interagieren können, jedoch gewährleistet ist, dass sie nicht durch mechanische Druckbelastung geschädigt werden. Zugleich muss gewährleistet sein, dass der Kontakt mit der Unterlage über die gesamte Fläche des Stempels im Wesentlichen gleich ist.

Der Stempel kann aus einem einzigen Material, einem Gemisch von Materialien oder auch einem System von Elementen aus einem oder verschiedenen Materialien aufgebaut sein. Besonders gute Ergebnisse wurden erzielt, wenn die Oberfläche des Stempels und/oder der Unterlage rasterartige Vertiefungen aufweist, da dies die Verteilung der Flüssigkeit erleichtert. Außerdem können diese Vertiefungen dazu beitragen, Flächenabschnitte, die verschiedene Rezeptoren bzw. Sonden tragen, voneinander zu trennen.

Das erfindungsgemäße Verfahren kann durchgeführt werden, indem der bzw. die Analyt(en) direkt an der Unterlage immobilisiert bzw. gebunden werden. Eine Immobilisierung durch Adhäsion wird in den meisten Fällen eher ungünstig sein, da dann alle Moleküle einer Probe an der Oberfläche gebunden werden und dadurch die Empfindlichkeit beeinträchtigt wird. Für Proben, die außer dem Analyten keine weiteren immobilisierbaren Moleküle enthalten, kann jedoch auch eine unspezifische Adhäsion in betracht kommen. Weiterhin kann der Analyt direkt über funktionelle Gruppen an die Oberfläche gebunden werden. Diese Ausführungsform sollte jedoch bevorzugt nur dann eingesetzt werden, wenn die nachzuweisende Substanz eine funktionelle Gruppe aufweist, die sie von anderen in der Probe vorhandenen Substanzen unterscheidet. Eine weitere Möglichkeit besteht darin, die für die Bindung vorgesehene Oberfläche der Unterlage mit Spacer-Molekülen oder auch Abstandshaltergruppen zu versehen, die Funktionen zur Bindung des bzw. der Analyten bereitstellen. Derartige Spacer-Moleküle oder auch Brückenmoleküle sind dem Fachmann bekannt und bedürfen hier keiner näheren Erläuterung. Es sind alle Moleküle geeignet, die an die Unterlage binden können und eine funktionelle Gruppe zur Anbindung des Analyten bereitstellen können.

In einer bevorzugten Ausführungsform der vorliegenden Erfindung werden jedoch auf einer der beiden Oberflächen, bevorzugt der Unterlage. Rezeptoren immobilisiert. die Analyten spezifisch binden können. Die Immobilisierung der Rezeptoren kann sowohl über kovalente als auch nicht kovalente Bindungen, und Bindungen direkt an die Oberfläche oder Brückenmoleküle erfolgen. Eine Bindung über ein spezifisch bindendes Paar ist ebenfalls möglich.

Als Rezeptoren werden im Rahmen der vorliegenden Erfindung solche Substanzen bezeichnet, die mit einem Molekül eine spezifische Bindung eingehen können, d.h. zu einer molekularen Erkennung fähig sind bzw. eine von einem anderen Bindepartner erkennbare Gruppe aufweisen. Rezeptoren sind daher in der Regel Partner eines spezifisch bindenden Paares, wobei der andere Partner der nachzuweisende Analyt ist. Als spezifisch bindefähige Paare, von denen ein Partner als Rezeptor erfindungsgemäß eingesetzt wird, kommen die verschiedensten miteinander bindefähigen Partner in Betracht, wobei sich die Spezifität der Bindung sowohl auf einen speziellen Partner beziehen kann, wie z.B. Antigen-zugehöriger Antikörper oder Biotin-Avidin/Streptavidin, als auch auf eine Gruppe oder Klasse von Verbindungen, z.B. Antikörper-Protein A. Beispiele für spezifisch bindende Partner sind: Antikörper, Peptide, andere Scaffold-Proteine, DNA, RNA, RNA-Aptamere und somit die Paare Antigen-Antikörper, Hapten-Antikörper, Anti-Idiotyp-Antikörper-Antikörper, DNA-DNA, RNA-RNA, DNA-RNA, RNA-Aptamer-Peptid, Rezeptor-Ligand, Lectin-Zucker, Zinkfingerprotein-DNA, Enzym-Substrat, Biotin-Avidin/Streptavidin usw., sowie die jeweiligen Derivate oder Analoga. An Stelle von Antikörpern können auch deren bindefähige Fragmente und an Stelle von DNA oder RNA auch Nucleinsäurederivate etc. vennrendet werden.

In einer bevorzugten Ausführungsform werden als Bindungspaar Antikörper und eine Substanz mit einem von diesem erkannten Epitop, wie Antigene, Haptene oder Anti-Idiotyp-Antikörper, verwendet. Unter dem Begriff "Antikörper" werden in der vorliegenden Beschreibung auch Antikörperfragmente oder Antikörperderivate, sowie funktionelle Fragmente von Antikörpern oder Derivate davon, die das Epitop erkennen und binden können, verstanden. Die Antikörper können polyklonale oder monoklonale Antikörper sein, bevorzugt sind aber monoklonale Antikörper. Bekannte Fragmente und Derivate sind Fv-, Fab-, Fab'- oder F(ab')₂-Fragmente, "single-chain antibody fragments", bispezifische Antikörper, chimäre Antikörper, humanisierte Antikörper sowie Fragmente, die CDRs (complementarity determining regions) enthalten, die ein Epitop des Analyten erkennen. Der Rezeptor kann je nach Art des nachzuweisenden Analyten sowohl von Antigenen oder Haptenen als auch Antikörpern oder deren Derivaten oder Fragmenten gebildet werden.

In einer anderen bevorzugten Ausführungsform werden als Partner des spezifisch bindenden Paares Nucleinsäuren wie DNA (Desoxyribonucleinsäure) oder RNA (Ribonucleinsäure) und künstliche Nucleinsäuren wie LNA (Locked Nucleic Acid) und PNA (Peptide Nucleic Acid) und dreidimensionale Strukturen aus Nucleinsäuren, bevorzugt Aptamere, verwendet.

Gemäß der vorliegenden Erfindung ist es bevorzugt, daß die Probenmoleküle bzw. Analyten nicht direkt an die Unterlage binden, sondern über Moleküle, die auf der Unterlage immobilisiert sind. In einer Ausführungsform kann beispielsweise der Rezeptor biotinyliert sein und über ein Streptavidinmolkül mit der ebenfalls biotinylierten Unterlage verbunden werden. Ist der Rezeptor ein Antikörper, so kann dieser chemisch aktiviert werden, indem man bestimmte Gruppen der Glycosylierungen zu Aldehydgruppen oxidiert. Diese Aldehydgruppen können wiederum Aminogruppen oder Hydrazidgruppen einer modifizierten Oberfläche binden (siehe: Solomon et al. Journal of Chromatographie, 1990, Vol. 510, 321-329). Eine weitere Methode, die dem Fachmann geläufig ist, ist die Konjugation von Aminogruppen des Antikörpers mit Carboxygruppen einer Oberfläche durch den Einsatz von Ethyl-(Dimethylamino)-Carbodiimid /N-Hydroxy-Succinimid.

Das erfindungsgemäße Verfahren ist besonders vorteilhaft, wenn in einer Probe nicht nur ein Analyt, sondern gleichzeitig mehrere Analyten nebeneinander nachgewiesen werden sollen. Hierfür werden auf der Unterlage und/oder der zweiten Oberfläche, die erfindungsgemäß eingesetzt wird, mindestens zwei, bevorzugt mehrere Flächenabschnitte vorgesehen, die jeweils verschiedene Rezeptoren und/oder Sonden tragen. So werden z.B. Antikörper, Fragmente oder Derivate davon oder verschiedene Antigene oder auch Aptamere oder Nucleinsäureabschnitte oder Derivate davon als Rezeptoren und/oder Sonden eingesetzt, wobei ein Flächenabschnitt jeweils für eine Art von Rezeptor bzw. Sonde vorgesehen werden kann, d.h. dass pro Flächenabschnitt jeweils Rezeptoren und/oder Sonden, die eine Spezifität für jeweils einen Analyten haben, vorhanden sind, wobei verschiedene Flächenabschnitte Rezeptoren und/oder Sonden mit Spezifität für verschiedene Analyten tragen und zwar auf definierten Bereichen der Unterlage bzw. des Stempels, die jeweils getrennt voneinander sind. Die Flächenabschnitte können auch noch weiter unterteilt sein in kleinere Bereiche, von denen jeder Bereich wiederum eine andere Funktion haben kann oder unterschiedliche Rezeptoren bzw. Sonden tragen kann. So kann ein Abschnitt in mehrere Bereiche aufgeteilt werden, die alle für denselben Analyten bindefähig sind, wobei ein Bereich als Kontrollbereich, Leerwert oder zur Standardisierung ausgebildet ist, während die anderen Bereiche der Bestimmung eines Analyten dienen. Mehrere Bereiche können auch jeweils dieselbe Art von Rezeptor oder Sonde tragen. Solche Bereiche werden als "Spots" bezeichnet. Eine Unterlage kann bis zu 10.000 Spots/cm² umfassen. Man spricht auch von einem "microarray". Durch hochparallele Anordnung von Rezeptoren bzw. Sonden mit unterschiedlichen Spezifitäten, z.B. Antikörpern oder Antigenen, können viele verschiedene in einer Zusammensetzung enthaltene Analyten spezifisch an den jeweiligen Spots auf der Unterlage eingefangen und gebunden werden.

In einer bevorzugten Ausführungsform des erfindungsgemäßen Verfahrens ist mindestens eine der beiden Oberflächen, d.h. entweder Unterlage oder Stempel mit Flächenabschnitten versehen, die jeweils verschiedene mit verschiedenen Analyten bindefähige Bindepartner tragen. Besonders bevorzugt weisen sowohl Unterlage als auch Stempel jeweils Flächenabschnitte auf, die mit verschiedenen Analyten bindefähig sind, wobei jeweils korrespondierende Flächenabschnitte bzw. einander gegenüberliegende Flächenabschnitte von Unterlage und Stempel an verschiedene Bindungsstellen des gleichen Analyten binden können. Auf diese Weise ist es möglich, mit einer Vorrichtung und in einem Testdurchgang viele verschiedene Analyte nachzuweisen. Dadurch werden Zeit und Kosten gespart.

In einer weiteren Ausführungsform wird mindestens ein Flächenabschnitt von Unterlage oder Stempel in mehrere Spots unterteilt. Die Spots werden dann in einer Ausführungsform so mit Rezeptoren beladen, dass mindestens ein Spot als Kontrolle für den "richtigen" Kontakt von Stempel und Unterlage dienen kann, und die restlichen Spots jeweils die gleichen oder unterschiedliche mit dem nachzuweisenden Analyten bindefähige Rezeptoren tragen. Zur Kontrolle können auf einem Spot Rezeptoren immobilisiert werden, die direkt mit der Sonde bindefähig sind, sodass es in jedem Fall auch ohne Gegenwart von Analyt zu einer Kopplung kommt und die Markierung auf den Rezeptor übertragen wird, wenn der Kontakt ausreichend war. Diese Art von Kontrolle dient dazu, falsch-negative Ergebnisse durch unzureichenden Kontakt der Oberflächen zu erkennen und vermeiden zu können.

In einer anderen Variante kann mindestens ein Spot mit einer Kombination aus Rezeptor und nachzuweisendem Analyten als einer Art innerer Standard versehen werden, um sicherzustellen, dass die Testbedingungen eine Bindung von Analyt an Sonde zulassen und sich die Bindung von Analyt an Rezeptor nicht vorzeitig löst.

In einer weiteren Variante können auch bei einander gegenüberliegenden Spots die Anordnung von Rezeptoren und Sonden variiert werden, so dass es sowohl auf der Unterlage als auch auf dem Stempel Spots mit Rezeptoren und Spots mit Sonden gibt. Art und Anordnung der Rezeptoren und Sonden ist frei wählbar und kann für jeden Test und jeden Analyten optimiert werden.

In einer weiteren Variante der Erfindung werden auf einem Flächenabschnitt Spots mit verschiedenen Rezeptoren bzw. Sonden, die mit einem Analyt bindefähig sind, vorgesehen. Dies hat den Vorteil, dass auch dann, wenn Moleküle in unterschiedlichen Zuständen vorliegen können und dann mit unterschiedlichen Bindepartnern bzw. mit unterschiedlichen Bindestärken binden, immer noch ein aussagekräftiges Signal erhalten werden kann. Diese Ausführungsform ist auch dazu geeignet, gegebenenfalls unterschiedliche Zustände eines Analyten zu differenzieren. In einem solchen Fall kann es auch nützlich sein, verschiedene Sensorkomplexe mit verschiedener Bindekraft für die mit einem Analyt bindefähigen Sonden inbetracht zu ziehen, um weitere Differenzierungsmöglichkeiten zu haben.

An die zweite Oberfläche, in der Regel den Stempel, sind Sonden gebunden, die spezifisch an den Analyten binden können.

Wenn alle nachzuweisenden Analyten eine Bindestelle aufweisen, die allen gemeinsam ist, z.B. eine Gruppe oder der konstante Teil eines Antikörpers, so können bei einer Ausführungsform der vorliegenden Erfindung an den Stempeln nur eine Art von Sonden gebunden sein, die jeweils mit der allen gemeinsamen Bindestelle binden können. In einer anderen Ausführungsform weist der Stempel jeweils Flächenabschnitte auf, die verschiedene Sonden tragen, wobei die Sonden jeweils mit verschiedenen Analyten bindefähig sind.

Die Sonde ist wiederum ein Partner eines spezifisch bindenden Paares, dessen anderer Partner der nachzuweisende Analyt ist. Als Bindungspaare sind dieselben Paare, wie oben für Rezeptor-Analyt beschrieben, geeignet. Natürlich müssen Rezeptor und Sonde an verschiedenen Stellen des Analyten binden. Daher muss der Analyt mindestens zwei Bindungsplätze zur Verfügung stellen. Für den Fachmann ist klar ersichtlich, dass auch möglichst nicht eine Bindestelle durch die Bindung an der anderen Bindestelle sterisch behindert werden darf, d.h. dass beide Bindungsplätze sterisch so angeordnet sein sollten, dass sie für die jeweiligen Bindungspartner zur Verfügung stehen unabhängig davon, ob bereits ein Bindungsplatz besetzt ist.

Üblicherweise bindet die Sonde selektiv an den Analyten, sie ist spezifisch für einen bestimmten Analyten. Vorzugsweise ist die Sonde ein Antikörper, ein Fragment oder Derivat davon. Die obenstehend in Bezug auf die Rezeptoren für die Probe bzw. den Analyten angeführten Varianten von Antikörpern, Fragmenten und Derivaten davon sowie Nucleinsäuren sind ebenfalls als Sonden denkbar. Üblicherweise enthält die Sonde eine Markierung. Die Markierung kann von einer Detektionseinrichtung nachgewiesen werden. Vorzugsweise handelt es sich bei der Markierung um einen Fluoreszenzfarbstoff, beispielsweise Fluoresceinisothiocyanat (FITC), Fluorescein, Rhodamin, Tetramethyl-Rhodamin-5-(und-6)-lsothiocyanat (TRITC), Texas Red, Cyaninfarbstoffe (CY3 oder CY5) usw. Fluoreszenzfarbstoffe sind vorteilhaft, da sie in sehr geringer Menge nachgewiesen werden können. In der Regel ist die Markierung kovalent mit der Sonde verbunden. Als weitere Möglichkeiten einer Markierung sind Reporterenzyme (ELISA) oder der Einsatz radioaktiver Isotope zu nennen.

Wesentlich für die vorliegende Erfindung ist, dass die Bindung der Sonde an den Analyten und die Bindung des Analyten an die Unterlage bzw. an den Rezeptor unter einer von außen angelegten Zugkraft stabiler ist als die Bindung der Sonde an den Stempel bzw. die Bindung der beiden Bindepartner des Sensorkomplexes aneinander.

Die Unterlage oder der Stempel wird mit der Probe, die die nachzuweisenden Analyten enthält, solange in Kontakt gebracht, dass eine ausreichend hohe Effizienz der Bindung von Analyt an Rezeptoren bzw. Sonden bei möglichst geringer unspezifischer Adsorption erreicht wird. Dies wird üblicherweise durch Inkubation der Unterlage mit der Probe erfolgen. Anschließend wird die Unterlage (bzw. der Stempel) aus der Inkubationslösung entnommen, gegebenenfalls gewaschen, um überschüssige Substanzen zu entfernen, und gegebenenfalls getrocknet.

Der Stempel mit der Sonde wird an die Unterlage, die an den Rezeptoren gebunden die nachzuweisenden Analytmoleküle aufweist, angenähert in solcher Art und Weise, dass Sonde und Analyt interagieren und gegebenenfalls binden können. Anschließend werden Stempel und Unterlage voneinander getrennt und es wird bestimmt, ob die Sonde an die Unterlage gebunden ist, bzw. wie viel Sonde an die Unterlage gebunden ist. Durch die Anbindung der Sonden an eine zweite Oberfläche gewährleistet die vorliegende Erfindung, dass in angenähertem Zustand zur Markierung der Analyten die maximal mögliche Konzentration an Sonden vorhanden ist. Zudem wird durch den differentiellen Krafttest gewährleistet, dass die Sonde im wesentlichen nur übertragen wird, wenn der Analyt tatsächlich vorhanden ist.

Ein Vorteil des erfindungsgemäßen Verfahrens besteht darin, dass in der Nachweiskette an zwei Stellen eine Erhöhung der lokalen Konzentration von Reaktionspartnern stattfindet; die Anbindung des Analyten auf einem räumlich begrenzten Bereich ("Spot") führt zu einer Erhöhung der lokalen Konzentration des Analyten gegenüber der Konzentration in Lösung und die Anbindung der entsprechenden Sonden auf einem räumlich begrenzten Bereich der zweiten Oberfläche entspricht einer Erhöhung der lokalen Konzentration der Sonden gegenüber einer Zugabe der Sonden als Lösung bei gleichzeitig höherer Reinheit, da sich die Menge der Begleitstoffe nicht erhöht und die anderen Sonden aussortiert sind. Dies führt zu einem besseren Signal-Rausch-Verhältnis und damit zu einer verbesserten Empfindlichkeit und einer niedrigeren Nachweisgrenze. Dieser Vorteil wirkt sich vor allem bei starker Parallelisierung aus.

Um nachzuweisen, wie viel Sonde von dem Stempel auf die Unterlage durch Bindung an den Analyten übergegangen ist, trägt bevorzugt die Sonde eine Markierung oder ist mit einer Markierung verbunden. Als Markierung kann für das erfindungsgemäße Verfahren jede analytisch nachweisbare Gruppe oder Substanz eingesetzt werden. Unter Markierung wird im Rahmen der Erfindung eine Eigenschaft verstanden, durch die sich einige Bindungspartner von anderen unterscheiden und die nachweisbar ist. Bevorzugt werden physikalisch nachweisbare Parameter herangezogen. Insbesondere kommen für die Markierung Atome oder Gruppen, die radioaktiv sind oder die eine Änderung optischer oder elektrischer Eigenschaften bewirken, in Betracht. Alle dem Fachmann bekannten Reportergruppen sind hier geeignet. Beispiele sind radioaktive Marker wie 3H, 14C, 32P, 35S, fluoreszierende, lumineszierende, chromophore Gruppierungen oder Farbstoffe, Metalle oder leitfähige Gruppen aber auch Substrate von Enzymen oder Reporterenzyme. Bevorzugt werden fluoreszierende oder chromophore Gruppen als Markierung verwendet. Bei der Verwendung von Proteinen als Bindungspartner kommt auch eine Anfärbung der an der Oberfläche gebundenen Proteinsubstanz mit Coomassie-Blue oder Silverstaining in Betracht.

Vorzugsweise handelt es sich bei der Markierung um einen Fluoreszenzfarbstoff, beispielsweise Fluoresceinisothiocyanat (FITC), Fluorescein, Rhodamin, Tetramethylrhodamin-5-(und-6)-isothiocyanat (TRITC), Texas Red, Cyaninfarbstoffe (CY3 oder CY5) usw. Fluoreszenzfarbstoffe sind vorteilhaft, da sie in sehr geringer Menge nachgewiesen werden können. In der Regel ist die Markierung entweder direkt mit der Sonde oder mit dem Teil des Sensorkomplexes verbunden, der übertragen wird.

Es können pro spezifisch bindendem Paar auch mehrere Markierungen verwendet werden und es können auch für verschiedene Sonden oder für mit verschiedenen Analyten bindefähige Sonden unterschiedliche Markierungen verwendet werden

Eine bevorzugte technische Umsetzung besteht darin, dass die Sonde nicht unmittelbar an das Stempelmaterial gebunden ist, sondern über einen Sensorkomplex, der von zwei Bindungspartner gebildet wird. Dabei ist der Stempel fest mit einem ersten Bindungspartner verbunden und die Sonde fest mit einem zweiten Bindungspartner. Der erste Bindungspartner kann kovalent mit dem Stempel verbunden sein, der zweite Bindungspartner kann kovalent mit der Sonde verbunden sein. Der erste und der zweite Bindungspartner können spezifisch oder unspezifisch aneinander binden, wobei die Bindung üblicherweise nicht kovalent ist. Dabei ist die Bindung der Sonde an den Analyten und die Bindung des Analyten an die Unterlage unter einer von außen angelegten Zugkraft stabiler als die Bindung des ersten Bindungspartners an den zweiten Bindungspartner.

In diesem Fall trägt bevorzugt der Teil des Sensorkomplexes die Markierung, der mitübertragen wird, und nicht die Sonde selbst. Besonders bevorzugt werden zur Immobilisierung der Sonde zwei komplementäre Nucleinsäurestränge verwendet. Für das erfindungsgemäße Verfahren ist es nun vorteilhaft, wenn die Bindung so erfolgt, dass beim Ausüben einer Zugkraft auf die hybridisierten Stränge, die Bindungen reißverschlußartig aufgehen können. Bei dieser Ausführungsform wird der Bindepartner, bevorzugt die Sonde, an dem komplementären Strang so gebunden, dass es bei Anwendung einer Zugkraft zu einer reißverschlußartigen Trennung kommt. Dies wird wie folgt erreicht. Wenn der immobilisierte erste Strang mit seinem 3'-Ende an der Oberfläche fixiert ist, wobei das 5'-Ende unfixiert bleibt, wird der Bindepartner an dem komplementären zweiten Strang am 5'-Ende gebunden, so dass dann, wenn auf den Bindepartner Zug ausgeübt wird, die Kraft am 5'-Ende des komplementären zweiten Strangs angreift und damit jeweils nur auf eine Basenpaarung wirkt, die eine nach der anderen aufgehen können, so dass die Stränge reißverschlußartig getrennt werden. Diese Art wird als "Unzip-Paarung" bezeichnet. Wird der Bindepartner in diesem Fall am 3'-Ende gebunden, so ist die Kraft, die zur Trennung der Stränge erforderlich ist, viel größer, da sie auf alle Basenpaarungen gleichzeitig wirkt. Wenn der immobilisierte erste Strang mit dem 5'-Ende immobilisiert wird, muß der Bindepartner entsprechend am 3'-Ende des zweiten Strangs gebunden werden, um die reißverschlußartige Trennung zu bewirken.

In einer besonderen Ausführungsform ist der erste Bindungspartner eine natürliche oder künstliche Nucleinsäure, deren 5'-Ende mit dem Stempel verbunden ist. Der zweite Bindungspartner ist eine natürliche oder künstliche Nucleinsäure, die mit dem ersten Bindungspartner hybridisieren kann und einen Nucleinsäureduplex ausbilden kann. In dieser Ausführungsform ist das 3'-Ende des zweiten Bindungspartners mit der Sonde verbunden. Eine entsprechend umgekehrte Anordnung ist ebenfalls möglich, nämlich dass das 3'-Ende des ersten Bindungspartners mit dem Stempel verbunden ist und das 5'-Ende des zweiten Bindungspartners mit der Sonde verbunden ist.

In einer weiteren Ausführungsform wird die Sonde durch das 3'-Ende des zweiten Bindungspartners bzw. in der umgekehrten Anordnung das 5'-Ende des zweiten Bindungspartners dargestellt.

Die Sonde ist somit über die Wechselwirkung des Nucleinsäureduplexes an den Stempel gebunden. Wird nun der Stempel an die Unterlage angenähert, reagiert die Sonde mit dem Analyten in der Probe. Werden anschließend Stempel und Unterlage voneinander getrennt, so dissoziiert die Bindung zwischen erstem Bindungspartner und zweitem Bindungspartner. Die Kraft, die erforderlich ist, um diese Bindung zu trennen, ist verhältnismäßig gering, da der Nucleinsäureduplex gleichsam wie ein Reißverschluss auseinandergezogen wird. Während die Bindungsenergie und damit die Bindungsaffinität mit der Länge des Nucleinsäureduplexes wächst, bleibt die Kraft, die nötig ist um den Nucleinsäureduplex wie einen Reißverschluss zu trennen, konstant. Die große Bindungsaffinität zwischen den Nucleinsäuresträngen gewährleistet dabei, dass der Komplex nicht dissoziiert, ohne dass eine äußere Kraft an ihm anliegt bzw. dass die Sonde nicht frei in Lösung gelangen kann und an dem Stempel gebunden bleibt, solange sie nicht mit einem spezifisch an der Unterlage gebundenen Analyten interagiert hat.

Die Bindung des Bindepartners an den komplementären Nucleinsäurestrang sollte so erfolgen, dass weder die Hybridisierung der komplementären Nucleinsäurestränge noch die Bindung des Bindepartners mit dem anderen Partner sterisch behindert wird.

In einer weiteren Ausführungsform ist ein Bindepartner ein Polypeptid, an dessen C-terminalem Ende kovalent eine Nucleinsäure gekoppelt ist. Derartige Verbindungen können beispielsweise durch die in WO 01/04265 offenbarten Verfahren hergestellt werden. Das Verfahren arbeitet mit mRNA-Molekülen, die mit einem Puromycin-Tag modifiziert werden, und die an den C-Terminus ihres Polypeptids binden, nachdem sie in vitro exprimiert wurden.

Möglichkeiten zur Immobilisierung von Nucleinsäuresträngen sind dem Fachmann bekannt und bedürfen hier keiner näheren Erläuterung.

Ein Vorteil von Nucleinsäureduplexen als Sensorkomplex zur "Aufhängung" oder Immobilisierung der Sonde besteht darin, dass die grosse Bindungsaffinität zwischen den Nucleinsäuresträngen gewährleistet, dass der Komplex nicht frühzeitig von der Oberfläche abdissoziiert, ohne dass eine Interaktion zwischen den Partnern des spezifisch bindenden Paares möglich war und/oder ohne dass eine äußere Kraft angelegt wurde. Ein weiterer Vorteil ist es, dass durch Variationen der Basen, insbesondere des GC-Gehalts, ggf. auch, wenn es sich nicht um eine Unzip-Paarung handelt, der Länge des Duplexes, die Trennkraft des Duplexes genau eingestellt werden kann. Dem Fachmann ist bewusst, dass neben den Basen A, T, G, C und U auch andere Basen wie z.B. Inosin oder künstliche Nucleinsäuren wie PNA und/oder LNA verwendet werden können und so die Trennkraft weiter variiert werden kann. Ebenfalls kann die Trennkraft durch Modifikationen von Basen variiert werden. Dadurch kann der Fachmann ein "Feintuning" der Trennkraft vornehmen.

Es ist in der Regel nicht notwendig, die exakten Trennkräfte des Sensorkomplexes und der Bindung der spezifisch bindenden Partner zu kennen. Es ist in der Regel ausreichend, die Größenordnung zu kennen, wenn sich die Trennkräfte der zwei Komplexe stark unterscheiden. So ist beispielsweise die Trennkraft zwischen Antigen und Antikörper in der Regel deutlich höher als die Kraft, die erforderlich ist, um einen Nucleinsäureduplex in der oben beschriebenen Weise "reißverschlussartig" zu trennen.

Für den Fall, dass die Trennkräfte sehr genau eingestellt werden sollen, besteht die Möglichkeit, die Trennkraft zwischen zwei Molekülen mit einem Kraftmikroskop direkt zu messen. In verschiedenen wissenschaftlichen Veröffentlichungen wurde diese Technik im Detail beschrieben. Antikörper-Antigen-Trennkräfte liegen im Bereich von 50 pN bis 150 pN (Schwesinger et al., PNAS, 2000, Bd. 97, 18, 9972-9977); um einen Nucleinsäureduplex "reißverschlussartig" zu trennen, müssen bei einer reinen A/T-Sequenz 9 ± 3 pN und bei einer reinen C/G-Sequenz 20 ± 3 pN aufgewendet werden (Rief et al., Nature structural biology, 1999, Bd. 6, 4, 346-349). Um dagegen einen Nucleinsäureduplex zu trennen, indem man eine Kraft auf das 5'-Ende des ersten und das 5'-Ende des zweiten Stranges bzw. auf die 3'-Enden ausübt, müssen je nach Anzahl der beteiligten Basenpaare 30 bis 150 pN aufgebracht werden. Eine unter äußerer Kraft besonders stabile Bindung bildet Biotin zu Avidin aus. Um diese Bindung zu trennen, müssen 160 ± 20 pN aufgebracht werden (Florin et al., Science, 1994, Bd. 264, 415-417).

Eine andere mögliche Umsetzung besteht darin, dass die Sonde über nur einen Binder an die Stempeloberfläche gebunden ist. Dieser Binder kann kovalent mit der Sonde verbunden sein. Der Binder bindet unspezifisch direkt an die Stempeloberfläche. Dabei ist die Bindung der Sonde an den Analyten und die Bindung des Analyten an die Unterlage unter einer von außen angelegten Zugkraft stabiler als die Bindung des Binders an den Stempel.

Vorzugsweise hat der erste Bindungspartner eine hohe Affinität bzw. eine geringe Dissoziationsrate zum zweiten Bindungspartner, bzw. der Binder eine hohe Affinität bzw. eine geringe Dissoziationsrate zur Stempeloberfläche. Dadurch wird verhindert, dass die Sonde frei in Lösung gelangt und vom Stempel abdissoziiert. Eine hohe Affinität bzw. eine geringe Dissoziationsrate bei gleichzeitig geringer Stabilität unter einer von außen angelegten Zugkraft kann durch die Variation der Bindungsweite der Bindung des ersten Bindungspartners an den zweiten Bindungspartner bzw. des Binders an die Oberfläche erreicht werden. Wird die Bindungsweite bei gleicher Aktivierungsenergie der Trennung größer, vermindert sich die Kraft, die ausreicht, um die Bindung zu trennen. In der Regel ist unter einer von außen angelegten Zugkraft die Bindung einer unspezifisch an einen Rezeptor gebundenen oder an die Unterlage adsorbierten Probensubstanz schwächer als die spezifische Bindung an einen Rezeptor. Damit lässt sich die Stabilität der Bindung der Sonde an den Stempel unter einer von außen angelegten Zugkraft so einstellen, dass sie stärker ist als die Bindung einer unspezifisch an einen Rezeptor gebundenen oder an die Unterlage adsorbierten Probensubstanz, aber schwächer als die spezifische Bindung der Sonde an die Probensubstanz und schwächer als die Bindung der Probensubstanz an den Rezeptor. Das hat den Vorteil, dass bei einer Interaktion der Sonde mit einer unspezifisch gebundenen oder adsorbierten Probensubstanz die Sonde nicht an die Unterlage gebunden wird, sondern an den Stempel gebunden bleibt. Bei herkömmlichen Verfahren, in denen eine die markierte Sonde enthaltende Lösung mit der Unterlage inkubiert wird, bindet die Sonde auch an die Unterlage, wenn sie an unspezifisch gebundene Probensubstanzen bindet, was zu einem erhöhten Hintergrund führt. Das kann durch die vorliegende Erfindung vermieden werden.

Erfindungsgemäß ist es bevorzugt, dass der erste Bindungspartner und der zweite Bindungspartner natürliche oder künstliche Nucleinsäuren sind, bevorzugter handelt es sich um einzelsträngige DNA und/oder einzelsträngige RNA und/oder einzelsträngige LNA und/oder einzelsträngige PNA.

In einer weiteren Ausführungsform, in der die Sonde nur über einen Binder an die Stempeloberfläche gebunden ist, besteht dieser bevorzugt aus einem Polymer, das mit der Stempeloberfläche wechselwirkt. Das Polymer ist bevorzugt ein Biopolymer, eine Polyaminosäure oder ein Polyzucker, das z.B. über geladene, hydrophobe oder polare Seitengruppen die Wechselwirkung mit der Unterlage vermittelt.

Erfindungsgemäß ist es bevorzugt, dass der Stempel mehrere voneinander getrennte Bereiche aufweist, in denen Sonden mit unterschiedlicher Spezifität für unterschiedliche Probemoleküle gebunden sind. Die Unterlage ist dabei in Form eines Microarrays ausgebildet, vorzugsweise in Form eines Proteinmicroarrays. Der Stempel wird mit dem Microarray bzw. Analytarray in Kontakt gebracht, wobei die Sonden so angebracht sind, daß sie die Anbindungsfläche des korrespondierenden Analyten bzw. des korrespondierenden Rezeptors überdecken. Gebundene Probemoleküle werden so mit der Sonde interagieren. Bei herkömmlichen Techniken, bei denen die Sonden als Gemisch in einer Lösung auf die Unterlage gegeben werden, kann jede Sonde auf jeder Anbindungsfläche mit den gebundenen Probensubstanzen wechselwirken. Es wird dabei die Probensubstanz markiert, unabhängig davon, ob sie spezifisch oder unspezifisch gebunden ist oder ob sie nur an der Oberfläche adsorbiert ist. Dies kann durch die vorliegende Erfindung vermieden werden. Da die Sonden so angebracht sind, dass sie nur die Anbindungsfläche des korrespondierenden Analyten bzw. des korrespondierenden Rezeptors überdecken, wird dort und nur dort der korrespondierende Analyt nachgewiesen. Alle anderen dort unspezifisch gebundenen oder an die Oberfläche adsorbierten Probesubstanzen werden nicht markiert. Diese Art der direkten Adressierung einer Sonde an die Probemoleküle, die ihr zugehören, verhindert außerdem die eventuelle Kreuzreaktion der Sonde mit anderen Probemolekülen oder Rezeptoren. Es wird also jeweils an einem vorbestimmten Ort nur eine Art von Analyt gebunden und markiert. Vorteile der direkten Adressierung sind, dass die Moleküle angereichert vorliegen, in großer Reinheit ohne störende Begleitstoffe, die sonst den Test stören können.

Microarrays können nach an sich bekannten Methoden hergestellt werden. Vergleiche hierzu McBeath et al., Science, 2000, Vol. 289, 1760-1763 oder Lueking et al. Analytical Biochemistry, 1999, Vol. 270, 103-111. Zur Herstellung von Microarrys geeignet sind auch die in WO 00/27521 und EP 0865323 beschriebenen Verfahren. Der Nachweis der an die Unterlage gebundenen Sonde erfolgt ebenfalls durch Nachweis der Markierung mit an sich im Stand der Technik bekannten Methoden. Beispielsweise werden Fluoreszenzmarker durch Scannen mit einem Standard-Fluoreszenzscanner nachgewiesen.

Die Sonde dient insbesondere der Markierung des auf der Unterlage immobilisierten Analyten. Eine Besonderheit der Erfindung liegt darin, dass die Sonde lokal der Stelle zugeführt wird, an der der zu ihr gehörende Analyt gebunden ist. Der Vorteil ist, dass die Sonde dabei nicht frei in Lösung gelangt. Dies geschieht vorzugsweise dadurch, dass die Sonde auf einem Stempel mit geringer Kraft aber hoher Affinität angebunden wird. Der Stempel wird mit dem Analyt-Array in Kontakt gebracht, wobei die Sonde so angebracht wird, dass sie die Anbindungsfläche des korrespondierenden Analyten überdeckt. Probensubstanzen, die an einem Rezeptor gebunden sind, werden so auch mit der zugehörigen Sonde interagieren.

Trennt man den Stempel nun von der Unterlage, so kommt es zur Trennung der Bindung der Sonde zum Stempel, die Sonde verbleibt auf dem Analyt-Array und markiert somit den zu ihr spezifischen Analyten. Trifft die Sonde beim Kontakt des Stempels mit der Unterlage auf kein Probemolekül, zu der sie spezifisch ist, so verbleibt sie am Stempel. Durch den beschriebenen Ablauf wird gewährleistet, dass nur diejenigen Probenmoleküle mit einer Sonde markiert werden, die spezifisch an eine Sonde gebunden haben. Alle Probenmoleküle, die unspezifisch an die Unterlage gebunden haben, bleiben unmarkiert und tragen so nicht mit zum Hintergrund bei.

Wesentlich für die bevorzugte Ausführungsform des erfindungsgemäßen Verfahrens ist es, dass auf der Unterlage Rezeptoren immobilisiert sind, die mit dem nachzuweisenden Analyten spezifisch bindefähig sind und am Stempel Sonden immobilisiert sind, die ebenfalls mit dem Analyten spezifisch bindefähig sind, wobei die Immobilisierung der Sonden über einen Sensorkomplex erfolgt. Wird eine Bestimmung des Analyten durchgeführt, so wird z.B. zuerst die Unterlage mit der Probe in Kontakt gebracht, so dass der Analyt jeweils an seinen Rezeptor binden kann. Anschließend wird der Stempel mit der Unterlage in Kontakt gebracht so, dass die Sonde ebenfalls an den Analyten binden kann. Die Sonde wiederum ist über einen Sensorkomplex mit dem Stempel verbunden. Wenn daher der nachzuweisende Analyt in der Probe vorhanden ist und durch In-Kontakt-Bringen von Unterlage und Stempel an dem dafür vorgesehenen Rezeptor und die entsprechende Sonde gebunden hat, liegt eine Verkettung vor von Rezeptor-Analyt-Sonde-Sensorkomplex, wobei Sensorkomplex und Rezeptor jeweils fest an ihre Oberflächen gebunden sind. Wenn nun die beiden Oberflächen voneinander getrennt werden, wird auf diese Verkettung eine Zugkraft ausgeübt, die dazu führt, dass die schwächste dieser Bindungen sich löst. Wenn daher die Bindung der beiden Bindepartner im Sensorkomplex so ausgewählt wird, dass sie schwächer ist als die Bindung des Analyten an Rezeptor und Sonde, aber stärker ist als eine unspezifische Bindung von Analyt oder Sonde an einer Oberfläche oder einen nicht spezifischen Bindepartner, so geht dann, wenn der Analyt an seinen richtigen Rezeptor und an die richtige Sonde gebunden hat, bei Ausüben der Zugkraft der Sensorkomplex auf, während dann, wenn es zu einer unspezifischen Bindung zwischen Sonde und Unterlage gekommen ist oder aber ein nicht spezifischer Analyt von der Sonde gebunden wurde, der Sensorkomplex bestehen bleibt. Da der Bindepartner des Sensorkomplexes, der nicht an dem Stempel gebunden ist, die Markierung trägt, geht im ersten Fall die Markierung auf die Unterlage über, während im zweiten Fall die Markierung am Stempel gebunden bleibt. Man kann daher nach Trennen der Oberflächen feststellen, ob Markierung auf die Unterlage übergegangen ist bzw. wie viel Markierung übergegangen ist. Der Anteil von auf die Unterlage übergegangener Markierung ist ein Maß für die Art und Menge von in der Probe befindlichem Analyten, da nur bei Bindung eines Analyten die Lösung des Sensorkomplexes und damit das Übergehen der Markierung auf die Unterlage möglich ist.

Diese Ausführungsform der Erfindung kann auch so durchgeführt werden, dass die Sonde an der Unterlage gebunden ist und der Rezeptor am Stempel, d.h. dass die Sonde über den Sensorkomplex an der Unterlage immobilisiert wird, die Unterlage mit über den Sensorkomplex immobilisierter Sonde mit der Probe, die den nachzuweisenden Analyten enthält, in Kontakt gebracht wird, wobei der Analyt, falls er in der Lösung vorhanden ist, an die Sonde bindet. In einer weiteren Stufe wird dann der Stempel mit Rezeptor an die Unterlage so herangeführt, dass eine Interaktion zwischen Sonde, gegebenenfalls gebundenem Analyten und Rezeptor möglich ist und anschließend werden die beiden Oberflächen wieder getrennt. In diesem Fall wird dann, wenn der Analyt an der richtigen Stelle gebunden wurde, bei Anlegen der Zugkraft der Sensorkomplex getrennt und in diesem Fall die Markierung auf den Stempel übertragen. Welche Ausführungsform für den jeweiligen Einsatzzweck am geeignetsten ist, kann von dem Fachmann leicht durch Routineversuche festgestellt werden. Es kommt für die Durchführung der Erfindung nicht darauf an, wo Rezeptor und Sonde immobilisiert sind, sondern wichtig ist nur, dass die Bindungskräfte so ausgewählt werden, dass die Bindung der Bindepartner der Verkettung die oben aufgeführte Bedingung erfüllt.

Wird gemäß der besonders bevorzugten Ausführungsform das erfindungsgemäße Verfahren so durchgeführt, dass mehrere Analyten in einer Probe gleichzeitig nachgewiesen werden, werden Unterlage und bevorzugt auch Stempel so ausgebildet, dass eine entsprechende Anzahl an Flächenabschnitten vorgesehen werden, die jeweils mit den spezifischen Rezeptoren oder Sonden bestückt werden. Um die Empfindlichkeit des Tests und die Sicherheit zu erhöhen, kann es bevorzugt sein, mehrere Abschnitte jeweils mit dem gleichen Rezeptor zu versehen und diese Abschnitte auszuwerten und dann einen Durchschnitt der Messergebnisse zu bilden. Ebenso ist es aber auch möglich, jeweils nur einen Flächenabschnitt für einen Analyten vorzusehen.

Bei einer alternativen Ausführungsform des erfindungsgemäßen Verfahrens können auch auf Unterlage und Stempel Flächenabschnitte vorgesehen werden, die sich in ihrer Anzahl gleichen und einander gegenüberliegen, wobei z.B. auf einer der beiden Oberflächen vier Flächenabschnitte vorgesehen werden, die jeweils für den gleichen Analyten den entsprechenden Bindepartner bereitstellen, während auf den entsprechenden gegenüberliegenden Flächenabschnitten der anderen Oberfläche zwei oder vier verschiedene Bindepartner für denselben Analyten bereitgestellt werden, um die Sicherheit noch weiter zu erhöhen. Es können auch z.B. mehrere Flächenabschnitte mit Sonden für denselben Analyten vorgesehen werden, wobei sich die Sonden der einzelnen Flächenabschnitte in der Art ihrer Markierung unterscheiden, so dass z.B. verschiedene Markierungsarten oder von einer Markierungsart verschiedene Moleküle eingesetzt werden.

In einer weiteren Variante können auch mindestens zwei Flächenabschnitte jeweils auf Unterlage und Stempel vorgesehen werden, die jeweils den gleichen Analyten binden, wobei jeweils ein Flächenabschnitt Rezeptoren trägt und der andere Sonden trägt, so dass für denselben Analyten einmal die Rezeptoren auf der Unterlage angeordnet sind und einmal die Sonden. Auch dies dient der Erhöhung der Sicherheit und der Vermeidung von falsch-positiven oder falsch-negativen Ergebnissen.

Die vorliegende Erfindung betrifft ebenfalls eine Vorrichtung zur Bestimmung eines Analyten in einer Probe. Die erfindungsgemäße Vorrichtung umfasst eine Oberfläche (Stempel), an die eine Sonde gebunden ist, die an den zu bestimmenden Analyten binden kann, wenn sie damit in Kontakt kommt, wobei die Bindung der Sonde an den Analyten unter einer von außen angelegten Zugkraft stabiler ist als die Bindung der Sonde an den Stempel. Die bevorzugten Ausführungsformen der erfindungsgemäßen Vorrichtung entsprechen den bevorzugten Ausführungsformen des erfindungsgemäßen Verfahrens und wurden obenstehend erläutert.

Vorzugsweise ist ein erster Bindungspartner fest mit dem Stempel verbunden und ein zweiter Bindungspartner fest mit der Sonde verbunden, wobei der erste Bindungspartner und der zweite Bindungspartner nicht-kovalent aneinander binden können. Vorzugsweise sind der erste Bindungspartner und der zweite Bindungspartner Nucleinsäuren wie DNA und/oder RNA.

In einer bevorzugten Ausführungsform ist der erste Bindungspartner eine Nucleinsäure, dessen 5'-Ende mit dem Stempel verbunden ist, der zweite Bindungspartner ist ebenfalls eine Nucleinsäure, wobei das 3'-Ende des zweiten Bindungspartners mit der Sonde verbunden ist. Ebenso kann das 3'-Ende des ersten Bindungspartners mit dem Stempel verbunden sein und das 5'-Ende des zweiten Bindungspartners mit der Sonde verbunden sein.

Vorzugsweise weist der Stempel mehrere abgegrenzte Bereiche auf, in denen verschiedene Sonden mit unterschiedlicher Spezifität für verschiedene Analyten gebunden sind. Der Stempel dieser Vorrichtung kann dann an eine entsprechende Unterlage angenähert werden, auf der verschiedene Analyten in Form eines Arrays gebunden sind.

Die Vorrichtung kann weiterhin eine weitere Oberfläche (Unterlage) umfassen, auf der ein oder mehrere Analyten immobilisiert werden können. Ebenfalls kann sie eine Einrichtung zum Annähern und Trennen von Unterlage und Stempel und/oder eine Einrichtung zur Bestimmung, ob die Sonde an die Unterlage gebunden ist, umfassen.

In einer bevorzugten Ausführungsform wird erfindungsgemäß eine Vorrichtung zur Verfügung gestellt zum qualitativen und/oder quantitativen Nachweis eines Analyten in einer Probe, wobei die Vorrichtung eine Unterlage und einen Stempel umfasst, die so ausgebildet sind, dass sie miteinander in Kontakt gebracht werden können, wobei auf mindestens einer der beiden Oberflächen mindestens zwei Flächenabschnitte vorgesehen sind und wobei die Unterlage mit Rezeptoren versehen ist, während der Stempel mit über einen Sensorkomplex immobilisierten Sonden versehen ist und wobei verschiedene Flächenabschnitte mit verschiedenen Analyten bindefähige Rezeptoren bzw. Sonden tragen.

Ein Flächenabschnitt kann dabei aus mehreren Spots aufgebaut sein, wobei die Spots jeweils gleiche oder verschiedene Rezeptoren und/oder Sonden tragen können.

Die vorliegende Erfindung stellt ein Verfahren und eine Vorrichtung zur Bestimmung eines oder mehrerer Analyten in einer Probe zur Verfügung und macht es möglich, viele Analyten gleichzeitig mit hoher Spezifität und Empfindlichkeit nachzuweisen. Durch die Verwendung von zwei verschiedenen mit dem oder den nachzuweisenden Analyten spezifisch bindefähigen Bindepartnern, von denen einer über einen Sensorkomplex an eine Oberfläche gebunden ist, ist es möglich, die Nachteile des Standes der Technik zu überwinden und unspezifische Signale weitgehend zu vermeiden. Dadurch wird eine Reduktion des Signal/Hintergrund-Verhältnisses erzielt, was zu einer höheren Spezifität und Empfindlichkeit führt.

Mit der vorliegenden Erfindung wird ein Verfahren zur Verfügung gestellt, das einfacher, mit weniger aufwändiger Ausstattung und kostengünstiger durchgeführt werden kann, als die Verfahren des Standes der Technik.

Die Erfindung kann vielfältig eingesetzt werden: Es können Expressionsprofile von Zellen oder Geweben verglichen werden. Beispielsweise ist es möglich, das Expressionsprofil von Krebszellen und "normalen" Zellen zu vergleichen. Solche Vergleiche können auch diagnostisch eingesetzt werden. Es ist auch möglich, den Einfluss bestimmter Stoffe auf die Expression von Zellen zu untersuchen. Eine Einsatzmöglichkeit ist z.B.das mRNA-Profiling.

Eine wichtige Anwendung ist auch der Nachweis von Erkrankungen. Bestimmte Körperflüssigkeiten können durch ein Array verschiedener Antikörper untersucht werden, die Anwesenheit bzw. Konzentration eines spezifischen Antigens in der Probenzusammensetzung kann dann auf eine bestimmte Erkrankung hindeuten. So können in einem Experiment zahlreiche Aussagen gewonnen werden. Für den Fachmann sind weitere mögliche Anwendungen denkbar.

Gegenstand der Erfindung ist daher auch die Verwendung der Vorrichtung, wie oben definiert, zum parallelen Nachweis von mehreren Parametern, die für eine Krankheit charakteristisch sind, oder von mehreren Antigenen oder Antikörpern.

Weiterhin ist die Verwendung des erfindungsgemäßen Verfahrens zum parallelen Nachweis mehrerer für eine Krankheit spezifischer Parameter oder von mehreren Antigenen oder Antikörpern in einem Assay Gegenstand der Erfindung.

Zur Verdeutlichung der mit dem Gegenstand der Erfindung erzielbaren Vorteile werden im folgenden noch die nacheinander ablaufenden Schritten eines Nachweisverfahrens erläutert und die Möglichkeiten verschiedener Bindeereignisse. Den Bindeereignissen können verschiedene Bindungsarten zugrunde liegen; wobei insbesondere unspezifische Bindungen, z.B. durch Adsorption, und auf Kreuzspezifität oder Kreuzreaktivität beruhende spezifische Bindungen unerwünscht sind, da sie zu falsch-positiven, falsch-negativen Ergebnissen oder durch Erhöhung des Hintergrundsignals zu unempfindlichen Nachweissignalen führen.

Je nach Art des Nachweissystems ist für die Identifikation des Analyten der Ort der Nachweisreaktion (etwa Position auf einem Chip) und die Art der Bindung von entscheidender Bedeutung.

Bei den Verfahren des Standes der Technik können unerwünschte Bindungen bei der ersten Stufe, der Bindung des Analyten, durch spezifische Bindung am nicht dafür vorgesehenen Rezeptor und in der zweiten Stufe, der Bindung der Sonde, durch Bindung am nicht dafür vorgesehenen Analyten, am zur Bindung des Analyten vorgesehenen Rezeptor, durch unspezifische Adsorption der Sonde oder kreuzspezifische Bindung erfolgen. Erfindungsgemäß sollen diese unerwünschten Bindungen vermieden werden, unter anderem indem die Adressierung gezielter erfolgt.

Betrachtet man ein übliches mehrstufiges Nachweisverfahren, so lassen sich bei den einzelnen Schritten die folgenden Fälle unterscheiden:

| 1. Schritt: Bindung des Analyten A am Rezeptor R: | |
|---|---|
| spezifisch, am vorgesehenen Ort: | A-sp |
| spezifisch, am dafür nicht vorgesehenen Ort (Kreuzspezifität): | A-ksp |
| unspezifisch, am dafür nicht vorgesehenen Ort: | A-unsp |

| 2. Schritt: Bindung der Sonde S an die Unterlage/den gebundenen Analyten: | |
|---|---|
| spezifisch, am vorgesehenen Ort: | S-sp |
| spezifisch, am dafür nicht vorgesehenen Ort (Kreuzspezifität): | S-ksp |
| unspezifisch, am dafür nicht vorgesehenen Ort: | S-unsp |

Es ergeben sich also folgende Kombinationsmöglichkeiten:

| | |
|---|---|
| **a) Asp-S sp** | **"richtig-positiv", erwünscht** |
| b) A sp - S ksp | falsch-positiv |
| c) A sp - S unsp | falsch-positiv, falsch-negativ, Hintergrundsignalerhöhung |
| | |
| d) A ksp - S sp | falsch-positiv, falsch-negativ |
| e) A ksp - S ksp | falsch-positiv, falsch-negativ |
| f) A ksp - S unsp | falsch-positiv, falsch-negativ; Hintergrundsignalerhöhung |
| | |
| g) A unsp - S sp | Hintergrundsignalerhöhung |
| h) A unsp - S ksp | Hintergrundsignalerhöhung |
| i) A unsp - S unsp | Hintergrundsignalerhöhung |

Bei spezifischen und empfindlichen Nachweisverfahren sollte es im Idealfall nur die Kombination "A sp - S sp" geben, alle anderen Fälle sind weniger günstig, da sie zu
- falsch-positiven (z.B. falsch markierten Analyten),
- falsch-negativen (z.B. durch Belegung der Rezeptoren, Verdrängung des eigentlich vorgesehenen Analyten) oder
- unempfindlichen Nachweissignalen (durch Erhöhung des Hintergrundsignals) führen:

Mit dem Verfahren der vorliegenden Erfindung wird es ermöglicht, den Anteil der Kombinationen gemäß a) zu erhöhen und den Anteil der Kombinationen b) bis i) zu vermindern oder in bevorzugten Fällen weitgehend zu vermeiden. Dies erhöht die Zuverlässigkeit, Genauigkeit und Empfindlichkeit der Testverfahren im Vergleich zu den Verfahren des Standes der Technik.

Die Identifikation des Analyten basiert auf dem Ort der Sonde nach der Nachweisreaktion, also z.B. der Position auf dem Chip, verbunden mit der Annahme, dass die erwünschte Art von Bindung zugrunde liegt. So sind alle Fälle, in denen mindestens ein Schritt enthalten ist, der zu unerwünschter Bindung (ob nun bezüglich Ort und/oder Art der Bindung) führt, von Nachteil (Fälle b bis i).

Wenn der am vorgesehenen Ort spezifisch gebundene Analyt durch eine kreuzspezifisch oder unspezifisch bindende, nicht adressierte Sonden markiert wird, so kann es zum Absinken der Sondenkonzentration kommen, was die Nachweisgrenze für den eigentlich nachzuweisenden Analyten beeinträchtigen kann. Zudem kommt es zu falsch-positiven Signalen, wenn die nicht adressierte Sonde kreuzspezifisch oder unspezifisch z.B. an andere Rezeptoren bindet. Diese Fälle b), e) h) werden erfindungsgemäß dadurch vermieden, dass auf entsprechend präparierten Oberflächen die unspezifische Bindung gegenüber einer von außen angelegten Kraft instabiler ist als eine spezifische Bindung. Bei dem erfindungsgemäßen Verfahren hat eine mögliche Kreuzspezifität von Sonden gegenüber anderen Analyten keine negativen Auswirkungen, da die Sonden durch die erfindungsgemäß vorgesehene Adressierung nur an den vorgesehenen Ort der Bindung gelangen können:

Bei den bisher bekannten Verfahren kann durch unspezifische Bindung der Sonde am "freien" Rezeptor oder an dort falsch gebundenem Analyten mit nicht adressierten Sonden eine falsch-positive Nachweisreaktion entstehen, wenn dies nicht in einem Reinigungsschritt behoben werden kann. Die Sonden können je nach Ausmaß der unspezifischen Bindung - und nur bei unadressierten Sonden - auch zu falsch-negativen Signalen führen, wenn sie zunächst Bindungsstellen für die eigentlich vorgesehenen Sonden blockieren und dann im darauf folgenden Reinigungsschritt entfernt werden. Erfindungsgemäß werden diese Fälle c), f) und i) vermieden durch die Kombination aus direkter Adressierung der Sonde und der Anwendung des differentiellen Krafttests. Bei Anwendung des erfindungsgemäßen Verfahrens gelangt die Sonde nur dorthin, wo sie spezifisch binden soll, und sie bleibt nur gebunden, wenn die Bindung spezifisch an "ihren" Analyt erfolgt ist. Bei unspezifischer Bindung wird die Sonde durch Anwendung des differentiellen Krafttests wieder entfernt. Falsch-positive Signale, falsch-negative Signale oder Erhöhung des Hintergrundsignals durch Sonden können also vermieden werden. Auch wird die Nachweisgrenze nicht beeinträchtigt, da es nicht zu Verlusten von Sonden und damit verbundener Konzentrationsverringerung kommt.

Weiterhin können bei den Verfahren des Standes der Technik nicht adressierte Sonden am falschen Ort binden, was zu einer Erhöhung des Hintergrundsignals und damit zu einer Verminderung der Empfindlichkeit bzw. Verschlechterung der Nachweisgrenze führt. Die Erhöhung des Hintergrundsignals ist somit für die Fälle g), h) und i) ein entscheidendes Problem. Das Problem von falsch-positiven Signalen und einer Erhöhung des Hintergrundsignals durch unspezifische Bindung eines Analyten wird erfindungsgemäß durch direkte Adressierung der Sonde, verbunden mit dem differentiellen Krafttest, gelöst.

Wird der Analyt durch Kreuzspezifität am falschen Ort gebunden, so führt dies zu falsch-positiven Nachweissignalen (bei Identifikation über die unadressierte Sonde) oder auch zu falsch-negativen Signalen (bei Identifikation über den Ort des Rezeptors, wenn der eigentlich vorgesehene Analyt verdrängt wird bzw. seine Bindestellen blockiert werden).

Wird bereits der Analyt unspezifisch gebunden, so steigt das Hintergrundsignal, die Empfindlichkeit und Nachweisgrenze verschlechtern sich. Ein Nachweis des Analyten wird auch dadurch erschwert, dass bei geringen Analytkonzentrationen die Verluste an Analyt relativ groß sein können. Besonders nachteilig ist dies bei Verwendung nicht adressierter Sonden.

### Beschreibung der Figuren:

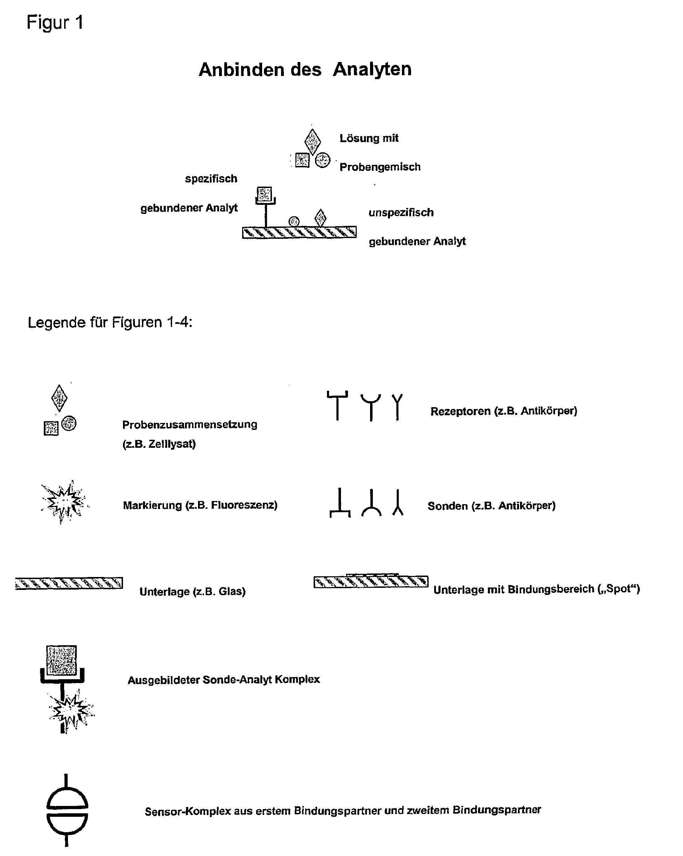

Figur 1 zeigt das Prinzip eines Ligandenassays, bei dem eine Probenzusammensetzung mit einer Unterlage in Kontakt gebracht wird. Neben spezifischer Bindung an einen Rezeptor kommt es zu unspezifischer Bindung von Analyten an die Unterlage. Unterhalb ist eine Erklärung der verwendeten Symbole angegeben, die für alle Figuren gilt.

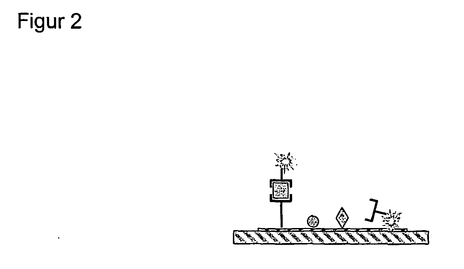

Figur 2 zeigt einen "Spot" auf einer Unterlage mit spezifisch an den Analyten gebundener markierter Sonde, unspezifisch gebundenen Analyten und unspezifisch gebundener Sonde.

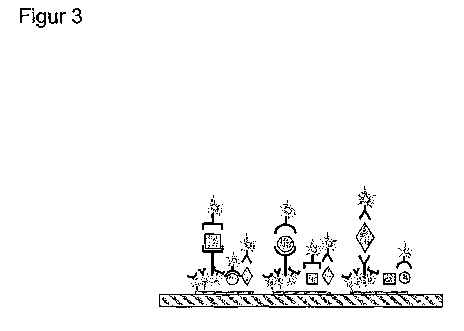

Figur 3 zeigt eine Unterlage mit drei "Spots" auf einer Unterlage. Jeder "Spot" trägt einen Antikörper, der spezifisch für ein Probenmolekül ist. Durch viele "Spots", zahlreiche verschiedene Analyten und zahlreiche verschiedene spezifische markierte Sonden steigt der Hintergrund stark an. Der Hintergrund wächst proportional zur Anzahl der "Spots" bzw. Sonden.

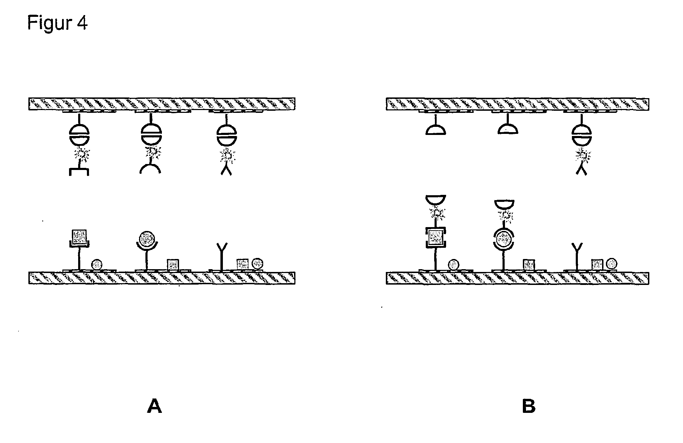

Figur 4 zeigt schematisch eine Ausführungsform des erfindungsgemäßen Verfahrens.
   (A) Auf einer Unterlage befinden sich drei "Spots", auf denen jeweils Antikörper mit einer bestimmten Spezifität immobilisiert sind. Nach Inkubation mit einer Probenzusammensetzung sind die Antikörpermoleküle von zwei "Spots" mit Probenmolekülen dekoriert, das Antikörpermolekül des dritten "Spots" weist keine Bindung auf, da die Probenzusammensetzung keinen entsprechenden Analyten enthielt. Auf den "Spots" befinden sich ebenfalls unspezifisch gebundene Probenmoleküle. Oberhalb ist ein Stempel gezeigt, der drei komplementäre "Spots" aufweist, die mit einem ersten Bindungspartner verbunden sind. Daran gebunden ist ein zweiter Bindungspartner, der fest mit einer markierten Sonde verbunden ist. Die markierten Sonden eines jeden "Spots" weisen ebenfalls eine bestimmte Spezifität für das Probenmolekül auf. Die Stempeloberfläche wird nun so angeordnet und an die Unterlage angenähert, daß die markierten Sonden mit den immobilisierten Probenmolekülen interagieren können.
   (B) Anschließend werden Stempel und Unterlage voneinander getrennt. Die beiden linken "Spots" der Unterlage enthalten nun Probenmoleküle, an die markierte Sonden gebunden sind, da die Kraft, die erforderlich ist, um die Sonde vom Analyten zu trennen, höher ist, als die Kraft, die erforderlich war, um den ersten Bindungspartner vom zweiten Bindungspartner zu trennen. Bei dem dritten "Spot" (rechts) konnte die Sonde nicht mit einem spezifisch gebundenen Analyten interagieren. Deshalb wurde der Komplex aus erstem und zweitem Bindungspartner nicht getrennt. In einem weiteren Schritt kann nun bestimmt werden, ob die Sonden bzw. wieviel der jeweiligen Sonden an die entsprechenden "Spots" der Unterlage gebunden ist.
   Die Darstellungen der Figuren 1 bis 4 sowie 5, 6, 8, 12,13, 17 sind nicht maßstabsgetreu, um die Klarheit zu erhöhen. Der Übersichtlichkeit halber ist nur ein Antikörpermolekül pro "Spot" gezeigt.

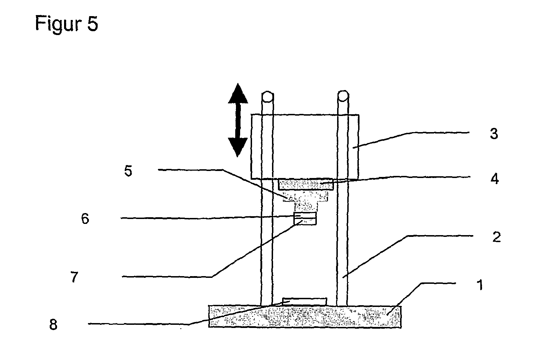

Figur 5 zeigt eine mögliche Ausführungsform eines Stempelapparates, der zur Durchführung des erfindungsgemäßen Verfahrens geeignet ist. Eine genauere Beschreibung einer möglichen Ausführungsform findet sich im Abschnitt "Durchführungsbeispiele".

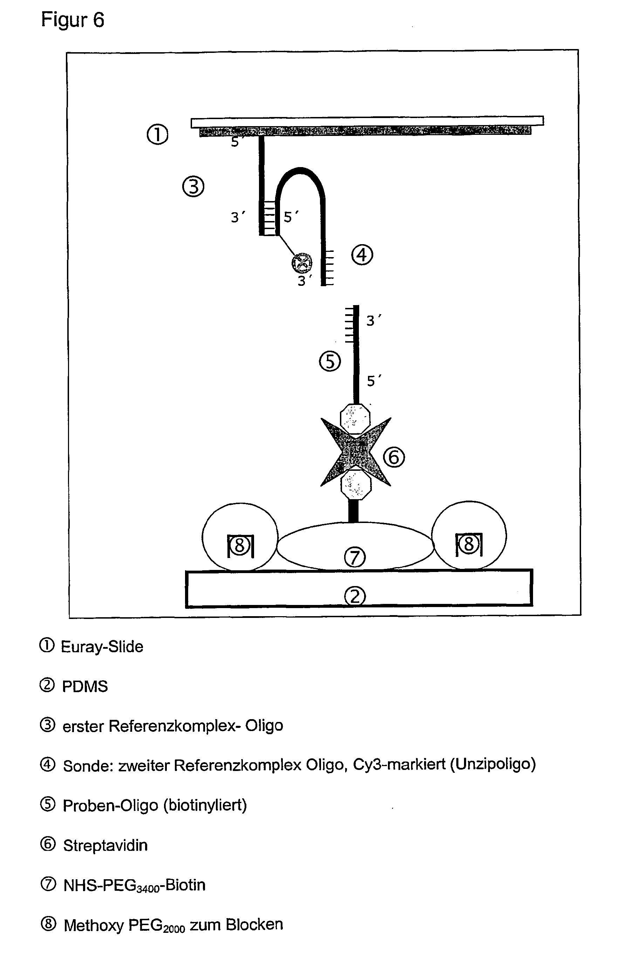

Figur 6 zeigt schematisch den Aufbau des experimentellen Beispiels 3.

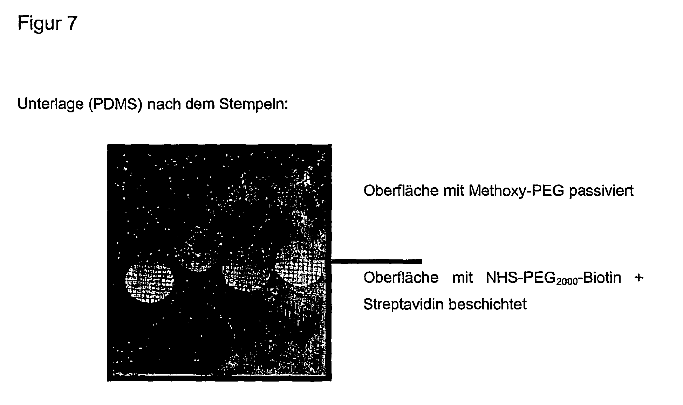

Figur 7 zeigt ein Beispiel eines Fluoreszenz-Scans einer Unterlage nach dem Stempeln (vgl. experimentelles Beispiel 3)

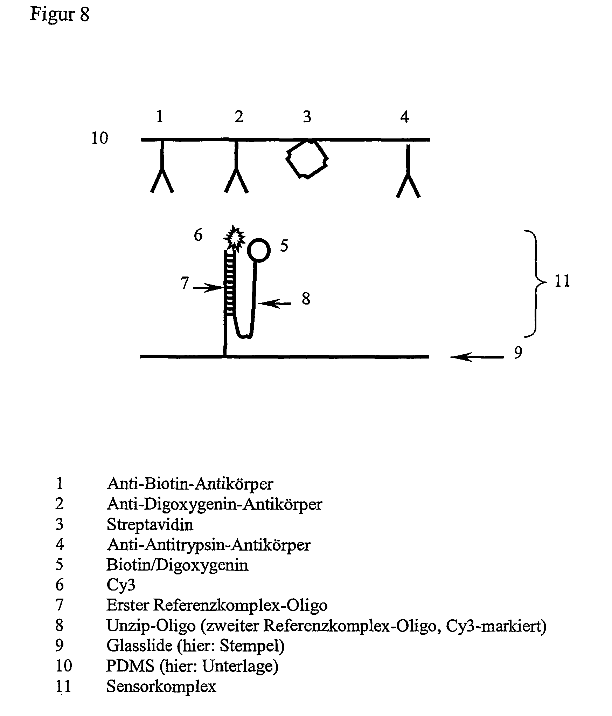

Figur 8 zeigt schematisch den Aufbau des experimentellen Beispiels 4.

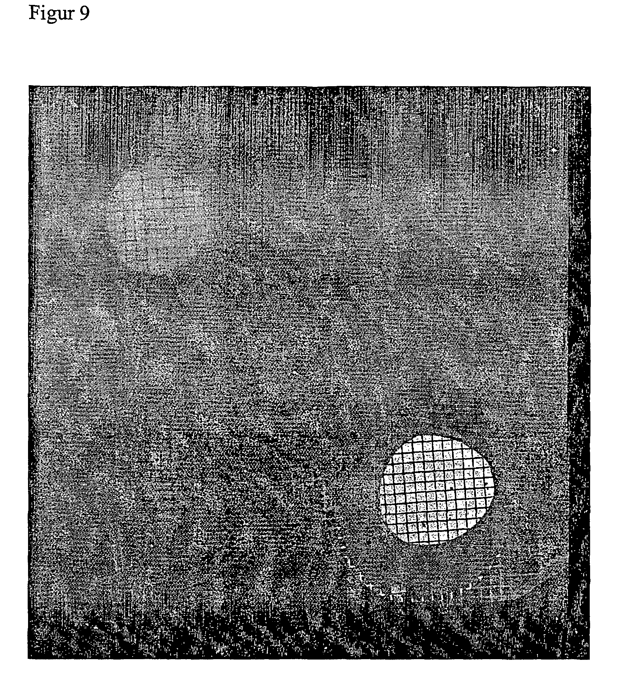

Figur 9 zeigt ein Beispiel für einen Fluoreszenz-Scan eines PDMS-Abschnitts (hier: Unterlage) nach dem Stempeln des Unzip-Oligos mit Biotin. Links oben war Streptavidin angebunden, rechts oben Anti-Digoxygenin, links unten Anti-Antitrypsin, rechts unten Anti-Biotin. Die hellen Flecken sind die mit Unzip-Oligo markierten Antibiotin-Antikörper bzw. Streptavidin. Die dunkleren Bereiche eines unspezifischen Transfers von Anti-Digoxygenin- und Anti-Antitrypsin-Antikörpern sind auch zu sehen.

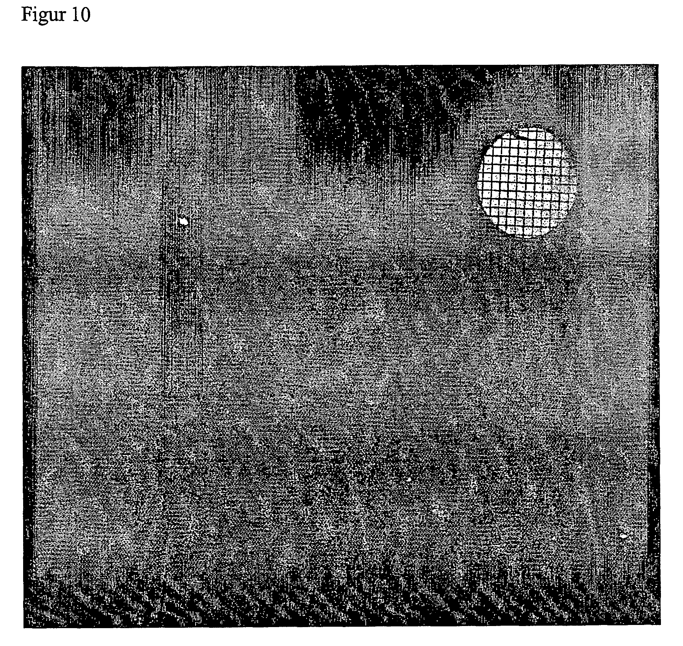

Figur 10 zeigt ein Beispiel für einen Fluoreszenz-Scan eines PDMS-Abschnitts (hier: Unterlage) nach dem Stempeln des Unzip-Oligos mit Digoxygenin. Links oben war Streptavidin angebunden, rechts oben Anti-Digoxygenin, links unten Anti-Antitrypsin, rechts unten Anti-Biotin. Der einzige helle Bereich ist dort, wo Antidigoxygenin-Antikörper immobilisiert war, alle anderen Spots sind sehr schwach.

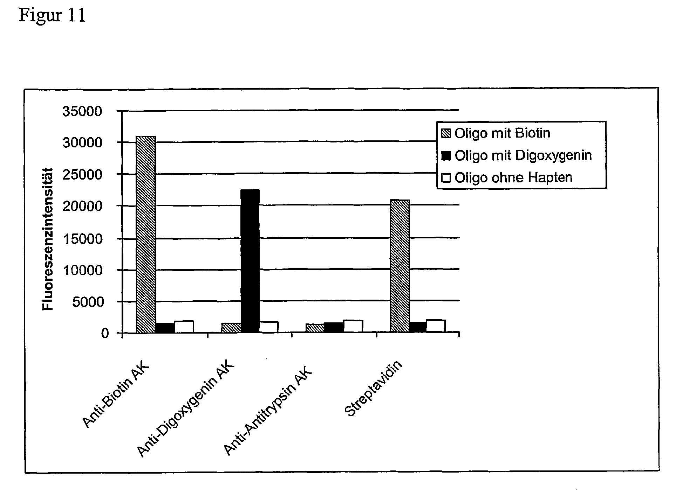

Figur 11 zeigt ein Diagramm, in dem die Ergebnisse des experimentellen Beispiels 4 zusammengefaßt sind: Stempeln eines Unzip-Oligos mit und ohne Hapten auf Oberflächen, die mit vier verschiedenen Proteinen beschichtet waren (Anti-Biotin, Anti-Digoxygenin, Anti-Antitrypsin-Antikörper, Streptavidin)

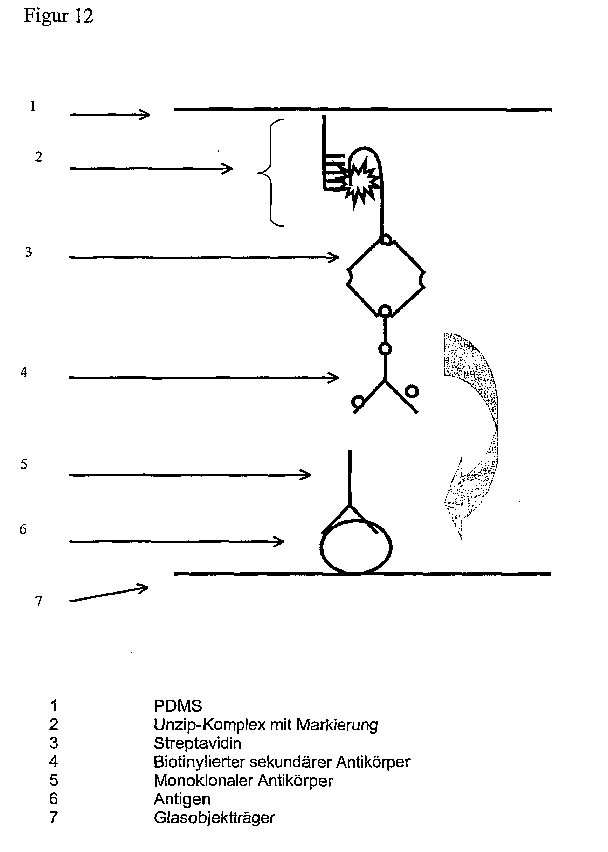

Figur 12 zeigt den allgemeinen schematischen Aufbau des experimentellen Beispiels 6.

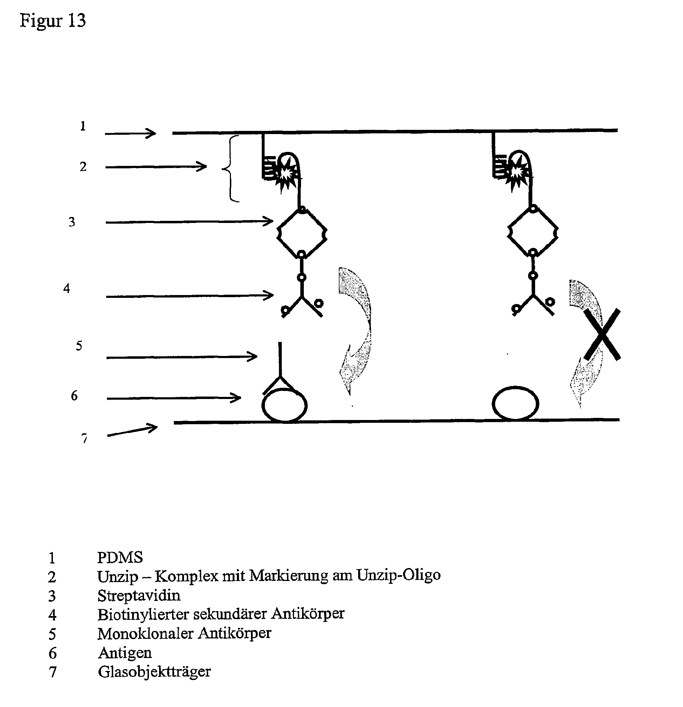

Figur 13 zeigt den schematischen Aufbau des experimentellen Beispiels 6, Ansatz 1.

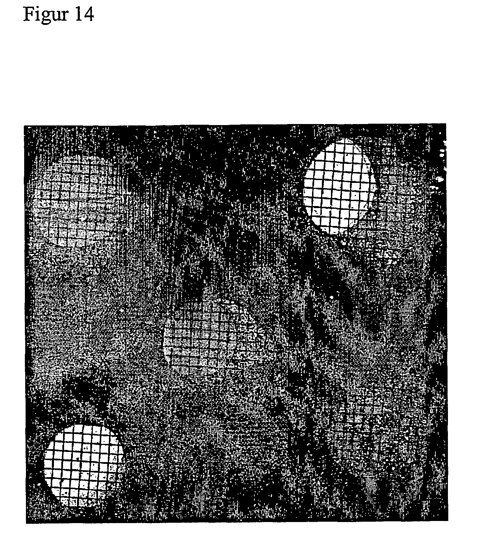

Figur 14 zeigt Überträge an Fluoreszenzmarkierung auf die Unterlage, wenn die Probenlösung den Anti-β-Galaktosidase und den Anti-Interferonγ-Antikörper enthielt. An der Unterlage war links oben Troponin T, rechts oben β-Galaktosidase, links unten Interferonγ, rechts unten α1-Antitrypsin angebunden. Die hellen Bereiche sind dort zu finden, wo der Unzip-Oligo die gebundenen Anti-β-Galaktosidase- bzw. Anti-Interferonγ-Antikörper markiert hat. Die dunkleren Bereiche des unspezifischen Übertrags auf die Unterlage dort, wo der Sensorkomplex direkt auf die keinen Analyten tragenden Rezeptoren oder auf die Passivierung (mittig) gestempelt wurde, sind ebenfalls zu erkennen.

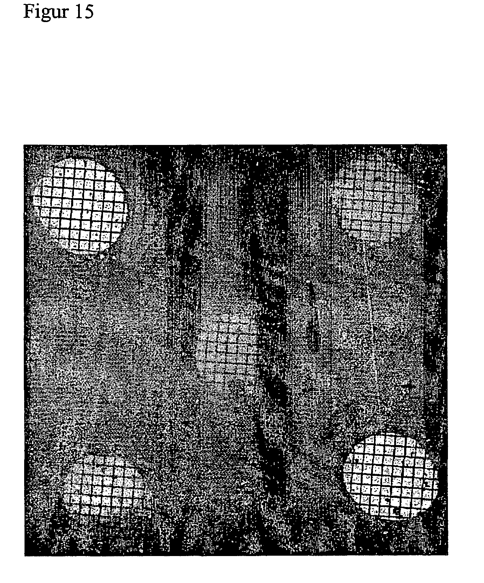

Figur 15 zeigt Überträge an Fluoreszenzmarkierung auf die Unterlage, wenn die Probenlösung den Anti-Troponin-Antikörper und den Anti-Antitrypsin-Antikörper enthielt. An der Unterlage war links oben Troponin T, rechts oben β-Galaktosidase, links unten Interferonγ, rechts unten α1-Antitrypsin angebunden. Die helleren Bereiche sind dort zu finden, wo der Unzip-Oligo die gebundenen Anti-Troponin- bzw. Anti-Antitrypsin-Antikörper markiert hat. Die dunkleren Bereiche des unspezifischen Übertrags auf die Unterlage dort, wo der Sensorkomplex direkt auf die keinen Analyten tragenden Rezeptoren oder auf die Passivierung (mittig) gestempelt wurde, sind ebenfalls zu erkennen.

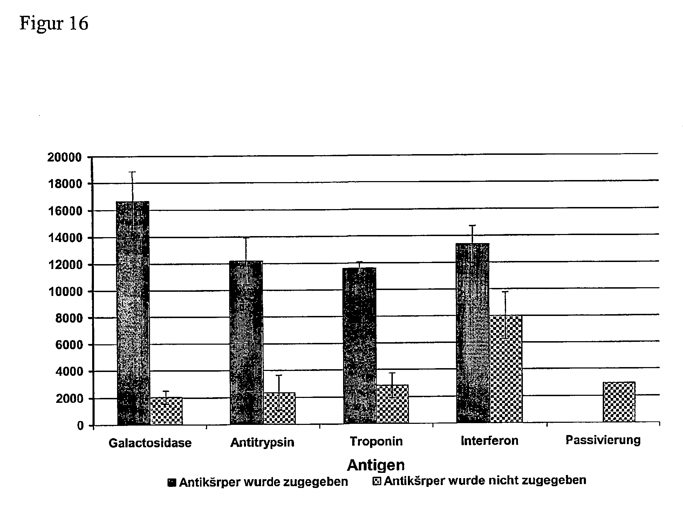

Figur 16 zeigt ein Diagramm, das die Ergebnisse des experimentellen Beispiels 6, Ansatz 1 zusammenfaßt.

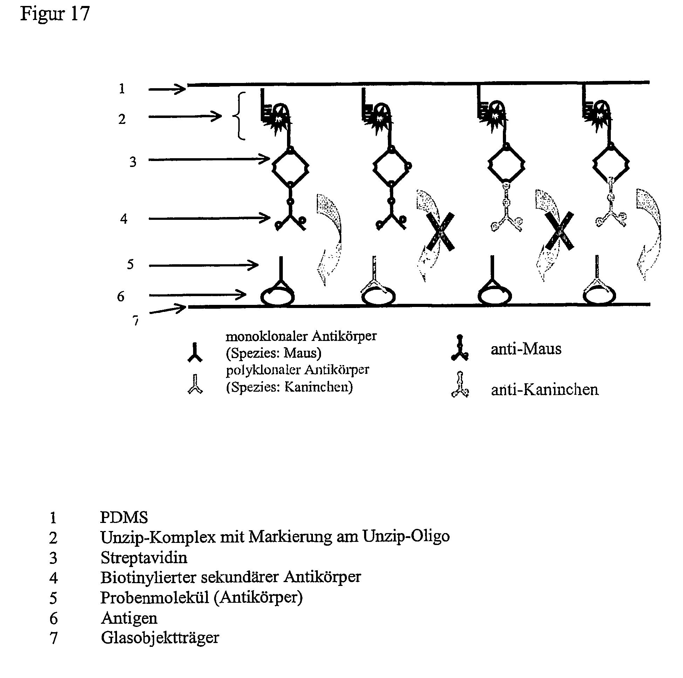

Figur 17 zeigt den schematischen Aufbau des experimentellen Beispiels 6, Ansatz 2.

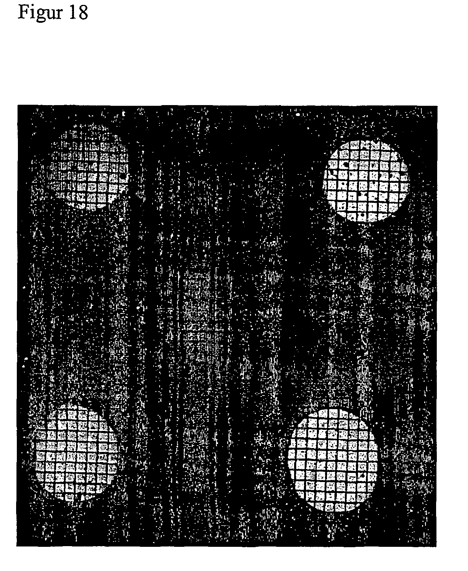

Figur 18 zeigt Überträge an Fluoreszenzmarkierung auf die Unterlage, wenn die Probenlösung den Anti-Troponin-, Anti-β-Galaktosidase-, und den Anti-Interferonγ-, Anti-Antitrypsin- (monoklonale Antikörper, Spezies Maus) und Anti-Aflatoxin-Antikörper (polyklonaler Antikörper, Spezies Kaninchen) enthielt. An der Unterlage war links oben Troponin T, rechts oben β-Galaktosidase, links unten Interferonγ, rechts unten α1-Antitrypsin angebunden, mittig Aflatoxin B1-BSA-Konjugat. Die hellen Bereiche sind dort zu finden, wo der Unzip-Oligo mit dem Anti-Maus-Antikörper als Sonde die gebundenen monoklonalen Antikörper (Spezies Maus) markiert hat. Der dunklere Bereich des unspezifischen Übertrags auf die Unterlage dort, wo der Sensorkomplex auf den polyklonalen Antikörper (Spezies Kaninchen) gestempelt wurde, ist ebenfalls zu erkennen.

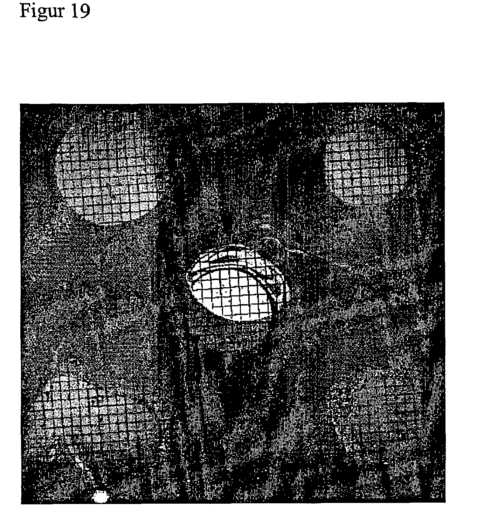

Figur 19 zeigt Überträge an Fluoreszenzmarkierung auf die Unterlage, wenn die Probenlösung den Anti-Troponin-, Anti-β-Galaktosidase-, und den Anti-Interferonγ-, Anti-Antitrypsin- (monoklonale Antikörper, Spezies Maus) und Anti-Aflatoxin-Antikörper (polyklonaler Antikörper, Spezies Kaninchen) enthielt. An der Unterlage war links oben Troponin T, rechts oben β-Galaktosidase, links unten Interferonγ, rechts unten α1-Antitrypsin angebunden, mittig Aflatoxin B1-BSA-Konjugat. Der hellere Bereich ist dort zu finden, wo der Unzip-Oligo mit dem Anti-Kaninchen-Antikörper als Sonde den gebundenen Anti-Aflatoxin-Antikörper markiert hat. Die dunkleren Bereiche des unspezifischen Übertrags auf die Unterlage dort, wo der Sensorkomplex auf die Antikörper der Spezies Maus oder auf die Passivierung gestempelt wurde, sind ebenfalls zu erkennen.

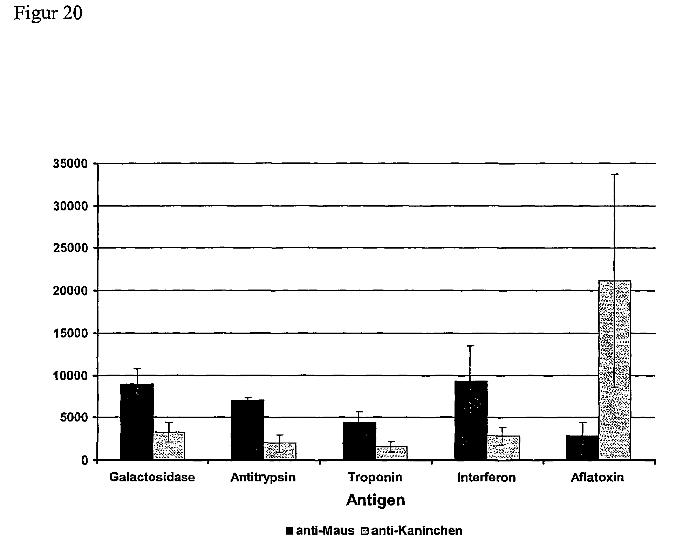

Figur 20 zeigt ein Diagramm, das die Ergebnisse des experimentellen Beispiels 6, Ansatz 2 zusammenfaßt.

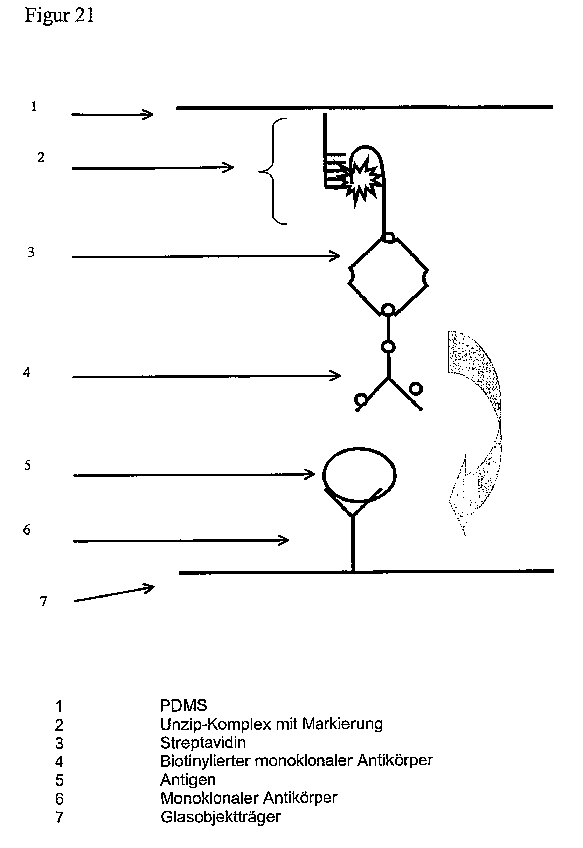

Figur 21 zeigt den schematischen Aufbau des in Beispiel 7 beschriebenen Tests.

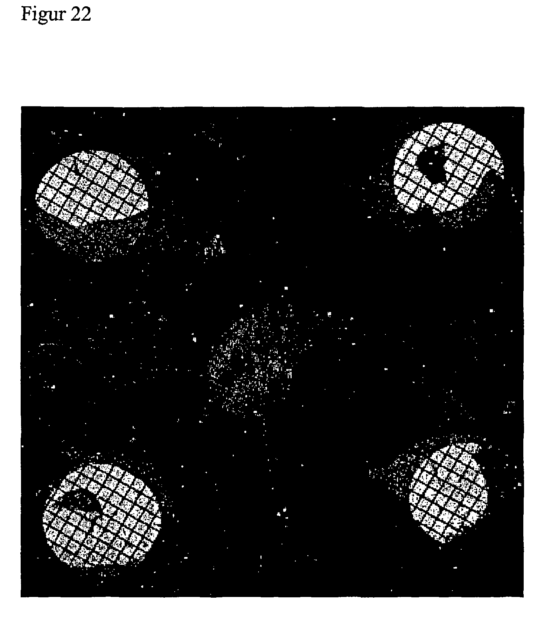

Figur 22 zeigt Überträge an Fluoreszenzmarkierung auf die Unterlage für den in Beispiel 7 beschriebenen Test.

Die zur Beschreibung der Erfindung verwendeten Begriffe werden folgendermaßen definiert:
Adsorption: Bindung eines Liganden oder einer Sonde an die Chip-Oberfläche, wobei die Bindung nicht über einen Rezeptor vermittelt wird.
Rezeptor: Ein Molekül, das einen Liganden spezifisch bindet, insbesondere ein Molekül, das einen Analyten spezifisch bindet; im Rahmen der vorliegenden Erfindung jede Substanz oder jedes Molekül, das für einen Analyten eine molekulare Erkennungsstelle aufweist und den Analyten in spezifischer Weise binden kann. Analyt: bezieht sich auf alle Arten von Molekülen, Substanzen oder Materialien, die in einer Probe nachgewiesen werden sollen. In Betracht kommen hier alle Moleküle oder Komplexe, ob organisch, anorganisch oder mit organischen und anorganischen Anteilen, die mindestens zwei spezifisch bindefähige Stellen aufweisen. Analyten werden in der vorliegenden Beschreibung auch als Probensubstanzen oder Probemoleküle bezeichnet.
Ligand : Ein Molekül, das einen Rezeptor spezifisch bindet.
Spezifisch: Eine Wechselwirkung zwischen zwei Molekülen, die auf einem hohen Maß an molekularer Erkennung beruht, ist spezifisch. Eine molekulare Erkennung, wie sie etwa für die Wechselwirkung eines Enzyms mit einem Substrat in der Substratbindungstasche, eines Antikörpers mit einem Antigen über seine "complementarity determining regions" (CDR) oder eines DNA-Einzelstrangs mit einem weiteren komplementären Einzelstrang über Watson-Crick-Basenpaarung typisch ist. Ein Rezeptor mit hoher Spezifität bindet innerhalb einer Population verschiedener Liganden nur einen oder wenige, wobei ein Rezeptor geringer Spezifität innerhalb der gleichen Population mehrere oder viele Liganden bindet.
Unspezifisch: Eine Wechselwirkung eines Moleküls mit einem anderen Molekül oder einem Körper, die nicht auf einem hohen Maß molekularer Erkennung beruht, ist unspezifisch.
Bestimmung: wird hier sowohl für den qualitativen als auch den quantitativen Nachweis eines Analyten verwendet und kann die Bestimmung der Menge und/oder Konzentration des Analyten umfassen. Umfasst von diesem Begriff sind auch die Identifizierung und/oder jegliche Charakterisierung eines Analyten anhand physikalischer Parameter.
Selektive Sonde: Eine Sonde, die nur einen oder sehr wenige Liganden eines Probegemischs bindet.
Direkte Markierung: Eine Markierung, die direkt und nicht mittels einer Sonde an einen Analyten angebracht wird.
Indirekte Markierung: Eine Markierung, die mittels einer Sonde an einen Analyten angebracht wird.
Kreuzreaktion: Unerwünschte, spezifische Wechselwirkung einer Sonde oder eines Rezeptors mit einem Analyten oder einer Sonde mit einem Rezeptor.
Sonde: Ein Molekül, das einen Liganden, der einen Analyten darstellt, bindet und zur Markierung dieses Liganden verwendet wird; im Rahmen der vorliegenden Erfindung jede Substanz oder jedes Molekül, das für einen Analyten eine molekulare Erkennungsstelle aufweist und den Analyten in spezifischer Weise binden kann und darüber hinaus eine Markierung mit dem Analyten in Verbindung bringt. Dazu kann eine Markierung an dem Sondenmolekül direkt gebunden sein oder aber das Sondenmolekül kann mit einem weiteren Molekül, insbesondere einem Sensorkomplexbestandteil, verbunden sein, an dem wiederum eine Markierung gebunden ist.
Bindungsweite: Die Wegstrecke, über welche die Bindungsenergie aufgebracht werden muß, wenn man den Komplex trennt.
Unterlage: Oberfläche, auf der die Rezeptoren gebunden sind; (unter anderem denkbar in Form einer ebenen Fläche, einer Folie oder zum Beispiel auch in Form eines Zylinders)
Stempel: Oberfläche, auf der die Sonden gebunden sind; (unter anderem denkbar in Form einer ebenen Fläche, einer Folie oder zum Beispiel auch in Form eines Zylinders)
Sensorkomplex: eine Kombination aus mindestens zwei spezifisch bindefähigen Molekülen, die bei Anwendung einer Zugkraft getrennt werden können, wobei die Kombination so ausgewählt wird, dass die Zugkraft, die zu ihrer Trennung notwendig ist, kleiner ist als die Zugkraft, die zur Trennung des spezifisch gebundenen Analyten von seinen Bindepartnern oder zur Abtrennung der immobilisierten, z.B. kovalent gebundenen Rezeptoren notwendig ist.
Probe, Probezusammensetzung: Zusammensetzung, die möglicherweise den/die zu bestimmenden Analyten enthält.

### Durchführungsbeispiele:

### Übertragung der Sonden

Die Übertragung der Sonden kann, wie beschrieben, so durchgeführt werden, dass zwei entsprechend ausgestaltete Oberflächen während eines Stempelvorgangs in innigen Kontakt gebracht werden.

Die Stempelprozedur kann mit einem einfachen Apparat erfolgen, wie es beispielsweise in Figur 5 dargestellt ist. Die hier abgebildete Apparatur besteht aus einer Bodenplatte (1), zwei Führungsstangen (2), einem Schlitten (3), einer Stempelkopfpolsterung (4), dem Stempelkopf (5) und der Stempelpolsterung (6) (siehe Figur 5). Die Bodenplatte, die Führungsstangen und der Schlitten können aus Metall gefertigt sein. Das Stempelkopfpolster kann aus einem Schaumgummi und der Stempelkopf aus Plexiglas bestehen. Die oben befestigte Oberfläche (Stempel oder Unterlage) kann quadratisch sein und hat in dieser Ausführungsform die Fläche von einem cm2 sowie eine Dicke von 1 mm. Das Stempelpolster hat die gleichen Abmessungen, ist jedoch auf beiden Seiten glatt und besteht z.B. aus besonders weichem PDMS.

Das Stempelkopfpolster hat die Funktion, den Stempelkopf beim Aufsetzen des Stempels in eine exakt parallele Position zur Unterlage zu bringen. Das Stempelpolster dient dazu, geringe Unebenheiten zwischen Stempel und Unterlage auszugleichen.

Zum Stempeln wird die Unterlage auf die Bodenplatte und der Stempel auf das Stempelpolster gelegt. (Alternativ kann auch der Stempel auf die Bodenplatte gelegt werden und die Unterlage auf das Stempelpolster, vgl. Bsp. 3 oder 4) Beide werden mit Puffer bedeckt. Der Schlitten wird in die Führungsstangen eingeführt und solange per Hand abgesenkt, bis der Stempel mit der Unterlage in Kontakt kommt. Das Trennen erfolgt ebenfalls per Hand.

### Beispiel 1

### Probekomplex: Biotin-Streptavidin, Sensorkomplex : Iminobiotin-Streptavidin

Zur Demonstration des Aufbringens der fluoreszenzmarkierten Sonden wurden die Probenmoleküle über Polymerspacer kovalent an der Unterlage angebunden. Als Analyt wurde Biotin und als Sonde fluoreszenzmarkiertes Streptavidin verwendet. Die Bindung des Analyten an die Unterlage über Rezeptoren wurde hier durch eine kovalente Bindung ersetzt. Der Sensorkomplex wird durch eine Streptavidin-Iminobiotin Bindung dargestellt. Iminobiotin wird auf dem Stempel spiegelbildlich zu den Biotin-Spots der Unterlage so an den Stempel gekoppelt, dass die Iminobiotin-Streptavidin-Spots auf dem Stempel während des Kontakts der Oberflächen auf den Biotin-Spots zu liegen kommen.

Da die Streptavidin-Iminobiotin Bindung schwächer ist als die Streptavidin-Biotin Bindung und diese schwächer als die kovalente Bindung des Biotins an die Unterlage, lässt sich das auf der Unterlage gebundene Biotin durch einen gezielten Übertrag des Streptavidins von den Iminobiotinspots auf den Analyten nachweisen.

### Herstellung des Stempels:

Es wurde ein mikrostrukturierter Stempel aus PDMS (Polydimethylsiloxan) gefertigt. Die Strukturen bestanden dabei aus Stempelfüßchen von ca. 100 x 100 µm, die durch Vertiefungen von ca. 25µm Breite und 1µm Tiefe getrennt wurden. Hierzu wurde ein Ansatz aus einer 1:10 Mischung von Vernetzungsreagenz und Silikonelastomer (Sylgard 184, Dow Corning) nach mehrfachem Entgasen auf einen entsprechend strukturierten Siliziumwafer gegossen und für 24h bei RT inkubiert. Nach der Polymerisierung wurde die strukturierte Oberfläche des Stempels bei 1 mbar in einem Plasmaofen für 60s einem H₂O-Plasma ausgesetzt.

### Beschichtung des Stempels:

Die oxidierte Stempeloberfläche wurde mit 2% Triethoxyaldehydsilan (Amchro, Hattersheim, Deutschland) in 10% H₂O und 88% Ethanol für 30min inkubiert. Die silanisierte Oberfläche wurde mit Ethanol und mit Reinstwasser gewaschen und mit Stickstoff trocken geblasen. An die Aldehydgruppen des Silans wurde ein bifunktionales PEG angebunden, dessen eines Ende über eine Aminogruppe, das andere über eine Carboxygruppe verfügte. 20µl einer Lösung mit 20mg/ml NH2-PEG-COOH (Shearwater, Huntsville, USA) wurden unter einem Deckglas für 1h auf einem Stempel mit einer Fläche von 1 cm2 inkubiert. Es wurde mit Reinstwasser gewaschen und mit Stickstoff trocken geblasen. Anschließend wurden die Schiffschen Basen mit 1%gew Natriumborhydrid in H₂O, 30 min reduziert.

Iminobiotinhydrazid (Sigma, St. Louis) wurde aus einer PBS Lösung (Phosphate buffered Saline; Sigma) mit 5mg/ml EDC (1-Ethyl-3-(3-Dimethylamino-propyl)carbiimide; Sigma) und 5mg/ml NHS (N-hydroxy-succinimid; Sigma, St. Louis) an die Carboxygruppen des PEGs angebunden. Die Unterlage wurde mit Reinstwasser gespült und mit Stickstoff trocken geblasen.

Der Stempel mit dem immobilisierten Iminobiotin wurde mit 0,1 mg/ml Alexa-Fluor®-546-Streptavidin-Konjugat in PBS mit 0.02%Vol Tween20 inkubiert, mit Reinstwasser gespült und mit Stickstoff trocken geblasen.

### Beschichtung der Unterlage:

Als Unterlage wurden Aldehydsilan beschichtete Glasobjektträger (Typ CSS, Genpak, Brighton, UK) verwendet. An die Aldehydgruppen des Silans wurde ein bifunktionales PEG angebunden, dessen eines Ende über eine Aminogruppe, das andere über eine Carboxygruppe verfügte. 150µl einer Lösung mit 20mg/ml NH2-PEG-COOH (Shearwater, Huntsville) wurden unter einem 24x60 mm2 Deckglas für 2h bei 40 °C inkubiert. Es wurde mit Reinstwasser gewaschen und mit Stickstoff trocken geblasen. Anschließend wurden die Schiffschen Basen mit 1%gew Natriumborhydrid in H₂O, 30 min reduziert.

Biotinhydrazid (Sigma, St. Louis) wurde aus PBS (Phosphate buffered Saline ; Sigma) mit 5mg/ml EDC (1-Ethyl-3-(3-Dimethylamino-propyl)carbümide; Sigma) und 5mg/ml NHS (N-hydroxy-succinimid; Sigma,, Taufkirchen) an die Carboxygruppen des PEGs angebunden. Die Unterlage wurde mit Reinstwasser gespült und mit Stickstoff trocken geblasen.

### Übertragen der Sonde:

Die Oberflächen wurden unter PBS Lösung so einander angenähert, dass das auf dem Stempel gebundene Streptavidin mit dem an der Unterlage gebundenen Analyten, dem kovalent gebundenen Biotin, interagieren kann. Nach einer Inkubationszeit von 30min wurden die Oberflächen voneinander getrennt.

### Messung:

Die Unterlage wurde mit einem Laser-Scanner (Perkin Elmer GeneTac LS IV) nach der übertragenen Sonde, dem Alexa-Fluor®-546 markierten Streptavidin abgescannt.

### Beispiel 2

### Probekomplex : DNA-DNA ; Sensorkomplex : DNA-DNA:

Es sollen verschiedene nicht markierte DNA Moleküle in einem Probengemisch nachgewiesen werden. Die Rezeptoren werden durch DNA Moleküle dargestellt, die mit dem 3' Ende an die Unterlage gebunden sind. Diese hybridisieren jeweils spezifisch mit einem Bereich am 5' Ende des DNA Probenmoleküls. Die zugehörigen Sonden werden durch markierte DNA Moleküle dargestellt, die mit einem am 3' Ende des Probenmoleküls liegenden Bereich hybridisieren können. Der erste Bindungspartner des Sensorkomplexes ist ein DNA Molekül, dessen 5'-Ende mit dem Stempel verbunden ist. Der zweite Bindungspartner des Sensorkomplexes wird durch einen Bereich am 5' Ende des DNA Sondenmoleküls dargestellt. Durch diese Anordnung greift die Zugkraft beim Auseinanderziehen der Oberflächen an den beiden 3'-Enden des Rezeptor/Analyt-Duplexes und an den beiden 5'-Enden des Sonde/Analyt-Komplexes an. Beim Sensorkomplex greift die Kraft hingegen an dem 3'-Ende des zweiten Bindepartners des Sensorkomplexes und dem 5'-Ende des ersten Bindepartners des Sensorkomplexes an, was eine reißverschlußartige Auftrennung des Sensorkomplexes ermöglicht.

Die Sonden werden auf dem Stempel spiegelbildlich zu den Rezeptor-Spots der Unterlage gekoppelt, so dass die Spots mit den Sonden während des Kontakts der Oberflächen stets auf den Spots mit den zugehörigen Rezeptoren zu liegen kommen. Ist ein Analyt an den Rezeptoren gebunden, kann in der Kontaktphase die zugehörige Sonde an das 3' Ende des Analyten binden. Da der reißverschlußartig hybridisierte Sensorkomplex eine geringere Stabilität unter einer an das gesamte Konjugat angelegten Zugkraft besitzt, öffnet sich beim Trennen der Oberflächen der Sensorkomplex, während die fluoreszenzmarkierte Sonde am Analyten gebunden bleibt.

### Herstellung des Stempels:

Es wurde ein mikrostrukturierter Stempel aus PDMS (Polydimethylsiloxan) gefertigt. Die Strukturen bestanden dabei aus Stempelfüßchen von ca. 100 x 100 µm, die durch Vertiefungen von ca. 25µm Breite und 1µm Tiefe getrennt wurden. Hierzu wurde ein Ansatz aus einer 1:10 Mischung von Vernetzungsreagenz und Silikonelastomer (Sylgard 184, Dow Corning) nach mehrfachem Entgasen auf einen entsprechend strukturierten Siliziumwafer gegossen und für 24h bei RT inkubiert. Nach der Polymerisierung wurde die strukturierte Oberfläche des Stempel bei 1 mbar in einem Plasmaofen für 60s einem H₂O-Plasma ausgesetzt.

### Beschichtung des Stempels:

Die oxidierte Stempeloberfläche wurde mit 3% Aminopropyldimethylethoxysilan (ABCR, Karlsruhe, Deutschland) in 10% H₂O und 87% Ethanol für 30min inkubiert. Die silanisierte Oberfläche wurde mit Ethanol und mit Reinstwasser gewaschen und mit Stickstoff trocken geblasen. An die Aminogruppen des Silans wurde ein bifunktionales NHS-PEG-Biotin (Shearwater, Huntsville, USA) angebunden. 20µl einer Lösung mit 20mg/ml des PEG wurden unter einem Deckglas für 1 h auf einem Stempel mit einer Fläche von 1 cm2 inkubiert. Es wurde mit Reinstwasser gewaschen und mit Stickstoff trocken geblasen.

An das Biotin wurde Streptavidin (Sigma, Taufkirchen, Deutschland) 0,1 mg/ml in PBS angebunden. Die Anbindung des Biotin-Oligos erfolgte aus 25 µM in PBS, 20µl unter einem Deckglas in 30 min. Die fluoreszenzmarkierten (Cy3) Sonden wurden aus 25 µM in 5xSSC (saline sodium citric buffer) bei RT in 3µl Tröpfchen je Spot angebunden.

### Beschichtung der Unterlage:

Als Unterlage wurden Aldehydsilan beschichtete Glasobjektträger (Typ CSS. Genpak, Brighton, UK) verwendet. An die Aldehydgruppen des Silans wurde ein bifunktionales PEG angebunden, dessen eines Ende über eine Aminogruppe, das andere über eine Carboxygruppe verfügte. 150 µl einer Lösung mit 20mg/ml NH2-PEG-COOH (Shearwater, Huntsville) wurden unter einem 24x60 mm2 Deckglas für 2h bei 40 °C inkubiert. Es wurde mit Reinstwasser gewaschen und mit Stickstoff trocken geblasen. Anschließend wurden die Schiffschen Basen mit 1%gew Natriumborhydrid in H₂O, 30 min reduziert.

Die Carboxygruppen des angebundenen PEG wurden mit 5mg/ml EDC (1-Ethyl-3-(3-Dimethylamino-propyl)carbiimide; Sigma) und 5mg/ml NHS (N-hydroxy-succinimid; Sigma, Taufkirchen) in PBS (Phosphate buffered Saline; Sigma) 30 min aktiviert. Das Anbinden des Oligos erfolgte aus 25 µM in PBS, 1µl Tröpfchen je Spot innerhalb von 30 min unter gesättigter Wasseratmosphäre bei RT.

Die Analyten wurden aus 20µl eines Probengemischs in 5xSSC unter Deckglas bei 70C° anhybridisiert. Während des Anbindens konnte die Probe innerhalb von 30 min auf RT abkühlen.

### Übertragen der Sonde:

Die Oberflächen wurden in 5xSSC so einander angenähert, dass die auf dem Stempel gebundenen DNA Sonden mit den an der Unterlage gebundenen Analyten, hybridisieren konnten. Nach einer Inkubationszeit von 45min wurden die Oberflächen voneinander getrennt.

### Messung:

Die Unterlage wurde mit einem Laser-Scanner (Perkin Elmer GeneTac LS IV) nach der fluoreszenzmarkierten übertragenen Sonde abgescannt.

### Beispiel 3

### Probekomplex: Biotin-Streptavidin, Sensorkomplex: DNA - DNA:

Es sollen biotinylierte, unmarkierte DNA-Moleküle nachgewiesen werden.

Der Referenzkomplex wird folgendermaßen realisiert (vgl. Figur 6): DNA-Moleküle werden mit dem 5'-Ende an die Unterlage gebunden (3); diese hybridisieren jeweils spezifisch mit einem Bereich am 5'-Ende von weiteren DNA-Molekülen (4), den Sonden. Die Sonden weisen am 3'-Ende einen Bereich auf, der seinerseits mit Bereichen am 3'-Ende der Probemoleküle (5) hybridisieren kann. Die Sonden tragen zudem eine Fluoreszenzmarkierung.

Es resultiert folglich eine Anordnung, bei der, sobald eine ausreichende Kraft am 3'-Ende der Sonden (4) angreift, die beiden den Referenzkomplex bildenden Oligos (3+4) reißverschlußartig getrennt werden.

Bei dieser Durchführung wurden die Referenzkomplexe in Form von vier identischen Spots angebunden.Der Rezeptor wurde durch Streptavidin (6) dargestellt, das über NHS-PEG3400-Biotin auf der Oberfläche immobilisiert war.

Bei dieser speziellen Durchführung war der Rezeptor nicht in Form von Spots, sondern flächig auf einer Hälfte der Oberfläche angebunden; die zweite Hälfte der Oberfläche wurde zur Bestimmung des unspezifischen Übertrags mit Methoxy-PEG2000 geblockt (vgl. Figur 7).

Die Oberfläche wurde flächig mit Streptavidin inkubiert, wobei dieses nur auf der Seite binden konnte, auf der Biotin immobilisiert war. Bei einer weiteren flächigen Inkubation der Oberfläche mit den biotinylierten Probemolekülen (biotinyliertes Oligo) wurden diese nur dort gebunden, wo im vorigen Schritt Streptavidin immobilisiert wurde.

Dort wo der Analyt an den Rezeptoren (Streptavidin) gebunden hat, kann in der Kontaktphase die zugehörige Sonde an das 3'-Ende des Analyten hybridisieren. Da der reißverschlußartig hybridisierte Sensorkomplex eine geringere Kraftbeständigkeit aufweist als die Hybridisierung zwischen Sonde und Analyt, bei der die Kraft jeweils an den 5'-Enden der DNA-Moleküle und damit an allen Basenpaarungen gleichzeitig angreift, öffnet sich beim Trennen der Oberflächen der Sensorkomplex, während die fluoreszenzmarkierte Sonde am Analyten gebunden bleibt.

### Herstellung der Unterlage:

Es wurde eine mikrostrukturierte Unterlage aus PDMS (Polydimethylsiloxan) gefertigt. Die Strukturen bestanden dabei aus Füßchen von ca. 100 x 100 µm, die durch Vertiefungen von ca. 25µm Breite und 1µm Tiefe getrennt wurden. Hierzu wurde ein Ansatz aus einer 1:10 Mischung von Vernetzungsreagenz und Silikonelastomer (Sylgard 184, Dow Coming) nach mehrfachem Entgasen zwischen einen entsprechend strukturierten Siliziumwafer und eine glattte Plexiglasplatte gegossen und senkrecht für 24h bei RT inkubiert. Nach der Polymerisierung wurde die strukturierte Oberfläche bei 1 mbar in einem Plasmaofen für 60s einem H₂O-Plasma ausgesetzt.

### Beschichtung der Unterlage:

Die oxidierte Oberfläche wurde mit 3% Aminopropyldimethylethoxysilan (ABCR, Karlsruhe, Deutschland) in 10% H₂O und 87% Ethanol für 30 min inkubiert. Die silanisierte Oberfläche wurde mit Ethanol und mit Reinstwasser gewaschen und mit Stickstoff trocken geblasen. An die Aminogruppen des Silans wurde ein bifunktionales NHS-PEG-Biotin (Shearwater, Huntsville) angebunden. 10µl einer Lösung mit 20mg/ml des PEG wurden unter einem Deckglas für 1h auf der unteren Hälfte der Unterlage (mit einer Fläche von 1 cm2) inkubiert. Es wurde mit Reinstwasser gewaschen und mit Stickstoff trocken geblasen.

Die gesamte Oberfläche wurde anschließend mit einer 6 mM Lösung von NHS-PEG2000-methoxy (Rapp Polymere, Tübingen, Deutschland) geblockt.25µl der Lösung wurden dazu 1h unter einem runden Deckglas (12 mm Durchmesser) in Wasserdampfatmosphäre bei RT inkubiert Anschließend wurde kurz mit Reinstwasser abgespült.

An das Biotin wurde flächig Streptavidin (Sigma, Taufkirchen, Deutschland) aus 5 ml einer 1 µg/ml Lösung in PBS angebunden. Die Inkubation des biotinylierten Proben-Oligos erfolgte aus 25 µM in PBS, 20µl unter einem Deckglas in 60 min bei RT, über der gesamten Fläche. Anschließend wurde 2 x 10 min und 2 x 1 min mit 1 x PBS/0,01%Vol Tween 20 gewaschen und mit Stickstoff trockengeblasen.

### Beschichtung des Stempels:

Als Stempel wurden Euray-Slides von Exiqon (Biozym-Art.-Nr.: 590.202, product no.: 10212-6225-01, Lot: 40900-14) verwendet.

Das Anbinden des Referenzkomplex-Oligos A erfolgte nach Anleitung des Slideherstellers aus 2 µM in 1 x Spottingsolution, 1µl Tröpfchen je Spot innerhalb von 12 h in gesättigter Kochsalzlösung bei RT.

Die fluoreszenzmarkierten (Cy3) Sonden wurden aus 25 µM in 5xSSC bei RT in 1µl Tröpfchen je Spot unter Wasserdampfatmosphäre angebunden. Danach wurde 2x10 min mit 1xSSC/0,05%Vol Tween 20 und 2 x 1 min mit 0,2xSSC/0,05%Vol Tween gewaschen.

### Übertragen der Sonde:

Die Oberflächen wurden in 5xSSC so einander angenähert, dass die auf dem Stempel gebundenen DNA-Sonden mit den an der Unterlage gebundenen Probemolekülen hybridisieren konnten. Nach einer Inkubationszeit von 15min wurden die Oberflächen voneinander getrennt.

Da wie beschrieben nur auf einer Hälfte der Unterlage Biotin und Streptavidin immobilisiert wurde, konnte auch nur auf dieser Hälfte Analyt (also biotinyliertes Oligo) gebunden werden.Auf der mit Methoxy-PEG2000 geblockten Hälfte konnte kein biotinyliertes Oligo binden.

Demzufolge kann dort, wo das Probenoligo gebunden ist, beim Stempeln das Sondenoligo auf dem Stempel mit dem Probenoligo auf der PDMS-Unterlage hybridisieren. Beim darauf folgenden Trennen von Stempel und Unterlage bleibt die markierte Sonde an dem Probenmolekül gebunden, da die Bindungstrennkraft der Bindung zwischen Probenmolekül und Sonde größer ist als die der reißverschlussartigen Unzip-Bindung des Referenzkomplexes.

Gleichzeitig dient der innige Kontakt des Teils der Unterlage, an dem kein Analyt gebunden hat (obere Hälfte der Fläche, vgl. Figur 7) mit dem Stempel der Verifizierung, dass die Sonde nur bei spezifischer Bindung an das immobilisierte Probemolekül übertragen wird, dagegen nicht bei Kontakt mit der passivierten Oberfläche, die keinen Analyten trägt.

Folgende Ansätze wurden durchgeführt:

**Tabelle 1**

| | | |
|---|---|---|
| Ansatz | obere Hälfte der Unterlage (vgl. Figur 7) | untere Hälfte der Unterlage (vgl. Figur 7) |
| Unterlage | Kontrolle: | NHS-PEG₃₄₀₀-Biotin + Streptavidin + biotinyliertes Probenoligo - dann gewaschen |
| | Methoxy-PEG₂₀₀₀ + biotinyliertes Probenoligo - dann gewaschen | |
| Stempel | Referenzkomplex-Oligos | Referenzkomplex-Oligos |

### Messung:

Stempel und Unterlage wurden mit einem Laserscanner (GenePix 4000B, Axon Instruments Inc., USA) bei 532 nm nach der mit Cy3 fluoreszenzmarkierten Sonde abgescannt.

### Auswertung und Ergebnis:

### Prinzip:

Die Spezifität des Übertrages der fluoreszenzmarkierten Sonde ist nachgewiesen, wenn auf die Hälfte der Oberfläche, an die der Analyt über Streptavidin binden konnte, ein deutlich höherer Übertrag der Fluoreszenz statttfindet als auf die mit Methoxy-PEG2000 geblockte Oberfläche (Figur 7, sowie Tabelle 2).

Die auf der Unterlage gemessene Fluoreszenz entspricht der übertragenen Sonde. Figur 7 zeigt die am Scanner gemessenen Überträge an Fluoreszenzmarkierung auf die Unterlage.

Zur Auswertung der Scans wurde das Programm NIH Image verwendet. Dieses Programm ermöglicht es, über ausgewählte Bereiche Histogramme zu erstellen. Die Histogramme zeigen die Häufigkeiten der gemessenen Intensitäten im ausgewählten Bereich. Aus den Histogrammen wurde die mittlere Intensität der Kontaktflächen mit den Stempelfüßchen ermittelt, sowie und die mittlere Intensität an den Stellen, wo sich die Vertiefungen des PDMS befanden. Diese Werte wurden als Hintergrundfluoreszenz von den Intensitäten in den Kontaktflächen subtrahiert, um die Intensität des Übertrages zu erhalten.

Übertrag des Sondenoligos vom Stempel auf die Unterlage:

**Tabelle 2**

| | Übertrag auf den Analyten Spezifisch (untere Hälfte der Fläche, vgl. Figur 7) | Übertrag auf die Passivierung unspezifisch (obere Hälfte der Fläche, vgl Figur 7) | Verhältnis spezifisch/unspezifisch |
|---|---|---|---|
| PDMS-Unterlage nach dem Stempeln | 1314+/-453 | 153+/-39 | 11,4:1 |

Der Quotient aus dem spezifischen und dem unspezifischen Übertrag beträgt im Mittel 11,4:1. Spezifischer und unspezifischer Übertrag können folglich deutlich unterschieden werden. Der Analyt kann identifiziert werden.

### Beispiel 4

### Probekomplex: Protein-Hapten: Sensorkomplex: DNA - DNA (Nachweis des Proteins)

Hier wurde die Bindung durch den Rezeptor durch eine kovalente Bindung an die Oberfläche ersetzt.

Es wurden Versuche durchgeführt, bei denen auf einem Glasträger fluoreszenzmarkierte Bindepartner über einen Sensorkomplex immobilisiert waren und auf einer zweiten Oberfläche die komplementären Bindungspartner bzw. nicht spezifisch bindefähige Bindepartner immobilisiert waren.

Dieses Beispiel dient dazu, zu zeigen, dass dann, wenn zwei Oberflächen in Kontakt gebracht werden, an denen jeweils spezifisch bindefähige Bindepartner immobilisiert sind, die Sonde nur dann übertragen wird, wenn sie auf den spezifischen Bindepartner trifft, dass jedoch ein Übertrag nicht stattfindet, wenn auf der anderen Oberfläche ein nicht spezifisch bindefähiger Partner ist (z.B. ein an diesem Ort unerwünscht gebundenes, etwa schlicht adsorbiertes, Probemolekül). Außerdem wird gezeigt, dass bei der Ausübung von Kraft auf eine Kette aus Probekomplex und Referenzkomplex die als Referenzkomplex verwendete reißverschlussartige Bindung zwischen zwei DNA-Ketten aufgeht und nicht die Bindung zwischen zwei spezifischen Bindepartnern oder die kovalente Bindung der Immobilisierung, die jeweils eine stärkere Bindungskraft haben.

Für alle Versuche wurde ultrareines Wasser für das Waschen und die Herstellung der Puffer verwendet. Ölfreies N₂-Gas wurde zum Trocknen verwendet. Alle Chemikalien hatten Analysequalität.

### a) Vorbereitung der Glasträger

Es wurden Glasträger mit Aldehydgruppen an der Oberfläche (Typ CSS, Genpak, Brighton, GB) verwendet. Diese Träger wurden mit 20 mg/ml HCl-NH₂-PEG-COOH (Shearwater, Huntsville, USA) in PBS pH 7,4 beschichtet. 150 µl Lösung wurden pro Träger verwendet; die Träger wurden über Nacht in feuchter Atmosphäre bei Raumtemperatur inkubiert, wobei sie mit einem Deckglas mit 24 x 60 mm² bedeckt waren. Anschließend wurden die Träger mit Wasser gespült und mit N₂ getrocknet.

Die gebildeten Schiffschen Basen wurden dann mit 1%gew NaBH₄ in Wasser 30 Minuten bei Raumtemperatur reduziert. Anschließend wurde wiederum mit Wasser gespült und mit N₂ getrocknet.

In einer weiteren Stufe wurde an dem mit PEG beschichteten Träger Oligonucleotide gebunden, die die Verbindung zwischen Bindepartner und Glasträger herstellen sollten. Hierzu wurde ein als Oligo 61 bezeichneter erster Referenzkomplex-Oligo mit einer Aminogruppe am 5'-Ende verwendet. Eine 25 µmolare Verdünnung von Oligo 61 in PBS plus 7,5 mg/ml EDC wurde eingesetzt. 0,6 µl dieser Lösung wurden aufgetupft und in feuchter Atmosphäre 2 Stunden bei 40°C inkubiert. Anschließend wurde mit Wasser gespült und mit N₂ getrocknet.

Die Sequenzen der verwendeten Oligos sind wie folgt:
Oligo 61 (Referenzkomplex-Oligo A):
   5'-NH₂-AAA AAA AAA ATC TCC GGC TTT ACG GCG TAT-3'
Oligo 78 (Unzip, ohne Hapten):
   5'-Cy3-ATA CGC CGT AAA GCC GGA GAC AGA TAA GAC GCT ACA TGA AAA AAA AAA AA-3'
Oligo 79 (Unzip, mit Biotin):
   5'-Cy3-ATA CGC CGT AAA GCC GGA GAC AGA TAA GAC GCT ACA TGA AAA AAA AAA AA-biotin-3'
Oligo 82 (Unzip, mit Digoxygenin):
   5'-Cy3-ATA CGC CGT AAA GCC GGA GAC AGA TAA GAC GCT ACA TGA AAA AAA AAA AA-digoxygenin-3`

### b) Vorbereitung und Herstellung der PDMS-Stempel

Ein Stempel mit Mikrostruktur wurde hergestellt, der aus 1 mm dickem PDMS (Polydimethylsiloxan) bestand, das ein Raster mit Quadraten von 100 x 100 µm², die durch Vertiefungen mit 25 µm Breite und 1 µm Tiefe getrennt wurden, aufwies.

Zur Herstellung der PDMS-Unterlage wurde eine Mischung von Siliconelastomer (Sylgard 184, Dow Corning) und Vernetzungsreagenz (10:1) nach intensivem Entgasen zwischen einen strukturierten Siliciumwafer und eine ebene PMMA-(Polymethylacrylat)-Platte gegossen. Diese Anordnung wurde zur Polymerisierung 24 Stunden lang senkrecht bei Raumtemperatur aufbewahrt. Das polymerisierte PDMS wurde zu Stempeln in eine Größe von 1 cm^2² geschnitten.

Das strukturierte PDMS wurde in einem Plasma-Cleaner 60 Sekunden lang in Gegenwart von Eis funktionalisiert. Der Druck wurde dabei auf etwa 2 mbar reduziert.

Die mit Plasma behandelten PDMS-Stücke wurden dann silanisiert. Dazu wurden 2% Aldehyd-Ethoxysilan, 88% Ethanol und 10% Wasser vermischt und es wurden 50 µl/cm² dieser Mischung mit dem PDMS in feuchter Atmosphäre 30 Minuten lang bei Raumtemperatur inkubiert. Die Stücke wurden dann zweimal mit Ethanol, dreimal mit Wasser gewaschen und mit N₂ getrocknet.

Die so vorbehandelten silanisierten Stücke wurden dann mit PEG beschichtet. 20 mg/ml HCl-NH₂-PEG-COOH (Shearwater, Huntsville, USA) in PBS pH 7,4 wurden verwendet. Dazu wurden jeweils 150 bis 200 µl Lösung pro PDMS-Stück (1 cm^2²) in feuchter Atmosphäre bei Raumtemperatur über Nacht inkubiert. Es wurde mit Wasser gespült und mit N₂ getrocknet.

Die gebildeten Schiffschen Basen wurden dann mit 1 %gew NaBH₄ in Wasser 30 Minuten bei Raumtemperatur reduziert. Danach wurde mit Wasser gespült und mit N₂ getrocknet.

Die funktionellen Gruppen wurden dann mit EDC/NHS aktiviert. Dazu wurden 10 mg/ml EDC und 10 mg/ml NHS in PBS pH 7,4 hergestellt. Die PDMS-Stücke wurden mit jeweils 30 µl 30 Minuten lang bei Raumtemperatur inkubiert unter einem Deckglas mit 12 mm Durchmesser. Danach wurde wieder mit Wasser gespült und mit N₂ getrocknet.

An die so aktivierten Oberflächen wurden dann die spezifisch bindefähigen Bindepartner gebunden. Als Bindepartner wurden Antibiotin-Antikörper, Antidigoxygenin-Antikörper, Antitrypsin-Antikörper und Streptavidin verwendet. Es wurden jeweils Lösungen von 5 mg/ml polyklonalem Antibiotin-Antikörper, 5 mg/ml polyklonalem Antidigoxygenin-Antikörper, 50 µg/ml monoklonalem Anti-Antitrypsin-Antikörper bzw. 100 µg/ml Streptavidin in PBS mit 40% Glycerin hergestellt. Jeweils 4 µl wurden auf ein PDMS-Stück aufgetupft und 1 Stunde bei Raumtemperatur in feuchter Atmosphäre inkubiert. Dann wurde zuerst mit PBS mit 0,05% Tween 20 und 1 % BSA 1 Minute lang und dann mit PBS mit 0,05% Tween 20 2 bis 3 Minuten lang gewaschen.

Die noch freien funktionellen Gruppen auf dem PDMS wurden dann mit 2% BSA mindestens 2 Stunden lang bei Raumtemperatur blockiert. In dieser Blockierungslösung konnten die Stücke auch aufbewahrt werden. Vor der Inkubation mit dem Sensorkomplex auf dem Glasträger wurde das PDMS mit PBS 2 bis 3 Minuten lang gewaschen, mit Wasser gespült und mit N₂ getrocknet.

Um den Transfer der über die Detektorbindung gebundenen Bindepartner auf die Bindepartner, die an das PDMS gebunden waren, durchzuführen, wurde das PDMS auf den Glasträger in 1 x SSC 30 Minuten bei Raumtemperatur aufgedrückt. Danach wurden die zwei Oberflächen vorsichtig getrennt, mit Wasser gespült und mit N₂ getrocknet.

Die bei diesem Transfer übertragenen Fluorophore wurden mit einem Fluoreszenz-Mikroarray-Scanner GenePix 4000 B (Axon Instruments Inc., USA) gemessen. Beide Oberflächen wurden mit dem 532-nm-Laser gemessen (PDMS: PMT 600 V, 100% Energie, Fokus 100; Glasträger: PMT 550 V, 33% Energie, Fokus 0). Die Hintergrundfluoreszenz des PDMS (vor dem Stempeln außerhalb der Spots) ist etwa 200 und die Hintergrundfluoreszenz des Glasträgers (außerhalb der gebundenen Oligos) ist etwa 100. Die Intensitäten der Spots wurden mit der Software gemessen, die mit dem Instrument zusammen geliefert wird, indem ein Mittelwert in einem kreisförmigen Bereich berechnet wurde. Durch diese Messung wird die gitterartige Struktur in den Flecken vernachlässigt.

Als Kontrolle wurde ein Unzip-Oligo aus der Lösung auf die blockierten Antikörperflecken gebunden. Jeweils eine Art von Oligo (mit Biotin, Digoxygenin oder ohne Hapten) wurde in PBS mit 10% Glycerin und 0,05% Tween 20 für einige Versuche (siehe Tabelle 8) verdünnt. Die endgültige Konzentration des Oligos war 2 µM oder 8 µM. 10 µl dieser Lösung wurden auf den vier Antikörperspots 30 Minuten bei Raumtemperatur inkubiert unter einem Deckglas mit 12 mm Durchmesser. In dem Fall, in dem der Oligo in PBS ohne Tween 20 gelöst wurde, wurde der Glasträger nur mit Wasser gespült und getrocknet. Ansonsten wurde der Glasträger 2 bis 3 Minuten lang mit PBS mit 0,05% Tween 20 gewaschen, mit Wasser gespült und mit N₂ getrocknet.

### Ergebnisse

Ein PDMS-Stück mit vier Spots von verschiedenen Rezeptoren (Antikörpern bzw. Streptavidin) wurde auf einen Glasträger aufgedrückt, auf dem Unzip-Oligo mit Biotin an den ersten Referenzkomplex-Oligo hybridisiert war. Danach wurde ein PDMS-Abschnitt mit dem Fluoreszenz-Scanner auf übertragene Oligos gescannt. Der Versuch wurde mehrere Male wiederholt und die Ergebnisse sind in Tabelle 3 zusammengefasst. Die jeweiligen Glasträger wurden vor und nach dem Stempeln gescannt und die Ergebnisse in Tabelle 4 zusammengefasst. Fig. 9 zeigt ein Beispiel für einen Fluoreszenz-Scan eines PDMS-Abschnitts nach dem Stempeln des Unzip-Oligos mit Biotin. Links oben war Streptavidin angebunden, rechts oben Anti-Digoxygenin, links unten Anti-Antitrypsin, rechts unten Anti-Biotin. Die hellen Flecken sind die mit Unzip-Oligo markierten Antibiotin-Antikörper bzw. Streptavidin. Die dunkleren Bereiche eines unspezifischen Transfers von Anti-Digoxygenin- und Anti-Antitrypsin-Antikörpern sind auch zu sehen.

**Tabelle 3**

| Fluoreszenzintensität auf dem PDMS nach Stempeln mit Unzip-Oligo mit Biotin | | | | | |
|---|---|---|---|---|---|
| Spot | Antibiotin-Antikörper | Antidigoxygenin -Antikörper | Anti-Antitrypsin-Antikörper | Streptavidin | Hintergrund zwischen den Flecken |
| 1 | 34918 | 2072 | 1912 | 28084 | 324 |
| 2 | 10294 | 427 | 369 | 6291 | 104 |
| 3 | 39841 | 1831 | 1278 | 24327 | 254 |
| 4 | 38897 | 1738 | 1296 | 24439 | 252 |
| Mittelwert | 30988 | 1517 | 1214 | 20785 | 238 |
| Standardabw. | 13960 | 740 | 636 | 9819 | 99 |

**Tabelle 4**

| Fluoreszenzintensität auf dem Glasträger vor und nach dem Stempeln mit Unzip-Oligo mit Biotin | | | | | | | | | | |
|---|---|---|---|---|---|---|---|---|---|---|
| Aufgestem pelt | Antibiotin-Antikörper | | Antidigoxygenin-Antikörper | | Anti-Antitrypsin-Antikörper | | Streptavidin | | Hintergrund zwischen den Flecken | |
| | vor | nach | vor | nach | vor | nach | vor | nach | vor | nach |
| 1 | 20723 | 8131 | 19015 | 8103 | 24581 | 10771 | 23498 | 9306 | 4337 | 2194 |
| 2 | 30712 | 22592 | 34469 | 26696 | 40265 | 29454 | 38061 | 27448 | 8138 | 6628 |
| 3 | 45484 | 28470 | 46352 | 33597 | 45343 | 30158 | 47983 | 30202 | 10648 | 8232 |
| 4 | 25108 | 21975 | 26660 | 21127 | 32111 | 28045 | 32506 | 23107 | 4856 | 5242 |
| Mittelwert | 30507 | 20292 | 31624 | 22381 | 35575 | 24607 | 35512 | 22516 | 6995 | 5574 |
| Std. abw. | 10789 | 8620 | 11671 | 10799 | 9134 | 9266 | 10253 | 9278 | 2960 | 2563 |

Ein PDMS-Stück mit vier Spots von verschiedenen Rezeptoren (Antikörpern bzw. Streptavidin) wurde auf einen Glasträger aufgedrückt, auf dem Unzip-Oligo mit Digoxygenin an den ersten Referenzkomplex-Oligo hybridisiert war. Danach wurde ein PDMS-Abschnitt mit dem Fluoreszenz-Scanner auf übertragene Oligos gescannt. Der Versuch wurde mehrere Male wiederholt und die Ergebnisse sind in Tabelle 5 zusammengefasst. Die jeweiligen Glasträger wurden vor und nach dem Stempeln gescannt. Die Ergebnisse sind in Tabelle 6 zusammengefasst. Ein Beispiel für einen Fluoreszenz-Scan eines PDMS, das mit einem Unzip-Oligo mit Digoxygenin gestempelt wurde, ist in Fig. 10 gezeigt. Links oben war Streptavidin angebunden, rechts oben Anti-Digoxygenin, links unten Anti-Antitrypsin, rechts unten Anti-Biotin. Der einzige helle Bereich ist dort, wo Antidigoxygenin-Antikörper immobilisiert war, alle anderen Spots sind sehr schwach.

**Tabelle 5**

| Fluoreszenzintensität auf dem PDMS nach dem Aufstempeln von Unzip-Oligo mit Digoxygenin | | | | | |
|---|---|---|---|---|---|
| Spot | Antibiotin-Antikörper | Antidigoxygenin -Antikörper | Anti-Antitrypsin-Antikörper | Streptavidin | Hintergrund zwischen den Spots |
| 1 | 2031 | 16810 | 1770 | 1900 | 752 |
| 2 | 1107 | 25362 | 1284 | 1209 | 349 |
| 3 | 1091 | 25047 | 1282 | 1213 | 351 |
| Mittelwert | 1410 | 22406 | 1445 | 1441 | 484 |
| Standardabw. | 538 | 4849 | 281 | 398 | 232 |

**Tabelle 6**

| Fluoreszenzintensität auf dem Glasträger vor und nach dem Aufstempeln mit Unzip-Oligo mit Digoxygenin | | | | | | | | | | |
|---|---|---|---|---|---|---|---|---|---|---|
| Aufges tempelt | Antibiotin-Antikörper | | Antidigoxygenin-Antikörper | | Anti-Antitrypsin-Antikörper | | Streptavidin | | Hintergrund zwischen den Spots | |
| | vor | nach | vor | nach | vor | nach | vor | nach | vor | nach |
| 1 | 30857 | 17340 | 29939 | 15270 | 34023 | 17562 | 32886 | 16116 | 15167 | 8802 |
| 2 | 54238 | 28446 | 51522 | 34243 | 53214 | 34678 | 51641 | 37493 | 19637 | 13269 |
| 3 | 42921 | 47280 | 42044 | 41268 | 44449 | 46901 | 47121 | 41584 | 20181 | 25678 |
| Mittelwert | 42672 | 31022 | 41168 | 30260 | 43895 | 33047 | 43883 | 31731 | 18328 | 15916 |
| Std. abw. | 11692 | 15135 | 10818 | 13449 | 9607 | 14737 | 9788 | 13677 | 2751 | 8744 |

Ein PDMS-Stück mit vier Spots von verschiedenen Rezeptoren (Antikörpern bzw. Streptavidin) wurde auf einen Glasträger aufgedrückt, auf dem Unzip-Oligo ohne Hapten an den ersten Referenzkomplex-Oligo hybridisiert war. Danach wurde ein PDMS-Abschnitt mit dem Fluoreszenz-Scanner auf übertragene Oligos gescannt. Das Ergebnis ist in Tabelle 7 zusammengefasst. Der jeweilige Glasträger wurde vor und nach dem Aufstempeln gescannt und das Ergebnis ist in Tabelle 8 zusammengefasst.

**Tabelle 7**

| Fluoreszenzintensität auf dem PDMS nach dem Aufstempeln von Unzip-Oligo ohne Hapten | | | | | |
|---|---|---|---|---|---|
| Spot | Antibiotin-Antikörper | Antidigoxygenin -Antikörper | Anti-Antitrypsin-Antikörper | Streptavidin | Hintergrund zwischen den Spots |
| | 1876 | 1685 | 1934 | 1823 | 429 |

**Tabelle 8**

| Fluoreszenzintensität auf dem Glasträger vor und nach dem Aufstempeln von Unzip-Oligo ohne Hapten | | | | | | | | | | |
|---|---|---|---|---|---|---|---|---|---|---|
| Aufges tempelt | Antibiotin-Antikörper | | Antidigoxygenin-Antikörper | | Anti-Antitrypsin-Antikörper | | Streptavidin | | Hintergrund zwischen den Spots | |
| | vor | nach | vor | nach | vor | nach | vor | nach | vor | nach |
| | 54316 | 39966 | 61090 | 40687 | 50410 | 39049 | 61371 | 44179 | 8604 | 8048 |

Als Kontrolle wurden die Oligos mit und ohne Hapten auch aus der Lösung auf Proteinspots gebunden. Die entstehenden Fluoreszenzintensitäten sind in Tabelle 9 zusammengefasst.

**Tabelle 9**

| Fluoreszenzintensität auf den Spots nach Bindung der Oligos aus der Lösung (Oligo 78 = Unzip-Oligo ohne Hapten, Oligo 79 = Unzip-Oligo mit Biotin, Oligo 82 = Unzip-Oligo mit Digoxygenin, PBST = PBS mit 0,05% Tween 20) | | | | | | |
|---|---|---|---|---|---|---|
| | Bindepuffer | Antibiotin-Antikörper | Antidigoxygen in-Antikörper | Anti-Antitrypsin-Antikörper | Streptavidin | Hintergrund zwischen den Spots |
| Oligo 79 (2 µM) | PBST | 2560 | 281 | 58 | 7624 | 54 |
| Oligo 79 (8 µM) | PBST | 14569 | 493 | 100 | 1490 | 105 |
| Oligo 79 (8 µM) | PBST | 18441 | 72 | 93 | 4237 | 93 |
| Oligo 79 (8 µM) | PBS | 13330 | 97 | 90 | 3692 | 74 |
| Oligo 82 (2 µM) | PBST | 373 | 10472 | 549 | 659 | 522 |
| Oligo 82 (8 µM) | PBST | 550 | 5468 | 336 | 628 | 494 |
| Oligo 82 (8 µM) | PBST | 234 | 2600 | 167 | 243 | 156 |
| Oligo 82 (8 µM) | PBS | 490 | 3083 | 319 | 313 | 271 |
| Oligo 78 (2 µM) | PBST | 225 | 196 | 135 | 175 | 118 |
| Oligo 78 (8 µM) | PBST | 187 | 434 | 249 | 791 | 251 |

Die Ergebnisse, die beim Stempeln erhalten wurden, die in Fig. 11 zusammengefasst sind, zeigen einen höchst spezifischen Transfer des Unzip-Oligos auf den entsprechenden Proteinspot (Fluoreszenzintensitäten zwischen 20 000 und 30 000), während der unspezifische Transfer auf die anderen Proteinspots ziemlich niedrig ist (etwa 1600) und unabhängig von dem verwendeten Oligo ist. Das Verhältnis von spezifischem zu unspezifischem Transfer ist größer als 10:1.

Fig. 11 zeigt eine Zusammenfassung der beim Stempeln des Unzip-Oligos mit und ohne Hapten auf vier verschiedene Proteinspots erhaltenen Ergebnisse. Die Bindung des Oligos aus der Lösung liefert weniger intensive Spots im Fall einer spezifischen Bindung (etwa 5800), aber auch eine geringere unspezifische Bindung (etwa 300).

Dies führt zu einem ähnlichen Verhältnis von spezifischer zu unspezifischer Bindung. Die höheren Intensitäten der Spots, die durch das Aufstempeln erreicht werden, beruhen auf einer höheren lokalen Konzentration. Weder die Gegenwart von Tween 20 im Bindepuffer noch unterschiedliche Waschstufen beeinflussen die Antigen-Bindung sonderlich.

### Beispiel 5

### Probekomplex : Antikörper-Antigen ; Sensorkomplex : DNA - DNA (Nachweis des Antigens)

Es sollen verschiedene nicht markierte Proteine in einem Probengemisch nachgewiesen werden. Die Rezeptoren werden durch biotinylierte Antikörper dargestellt, die über Streptavidin an die Unterlage gebunden sind. Die zugehörigen Sonden werden durch DNA-Antikörperfragment Konjugate dargestellt, die eine Fluoreszenzmarkierung tragen. Der Sensorkomplex wird durch zwei reißverschlußartig hybridisierte DNA Moleküle dargestellt. Der erste Bindungspartner des Sensorkomplexes ist ein DNA Molekül, dessen 5'-Ende über eine Aminogruppe mit dem Stempel verbunden ist. Der zweite Bindungspartner des Sensorkomplexes wird durch ein fluoreszenzmarkiertes DNA Molekül dargestellt, das eine Thiolgruppe am 3' Ende besitzt und darüber an ein freies C-terminales Cystein des Antikörper Fragments gekoppelt wird.

Die Sonden werden auf dem Stempel spiegelbildlich zu den Rezeptor-Spots der Unterlage lokalisiert, so dass die Spots mit den Sonden während des Kontakts der Oberflächen stets auf den Spots mit den zugehörigen Rezeptoren zu liegen kommen. Ist ein Analyt an den Rezeptoren gebunden, kann in der Kontaktphase die zugehörige Sonde an den Analyten binden. Da der reißverschlußartig hybridisierte Sensorkomplex eine geringere Stabilität unter einer an das gesamte Konjugat angelegten Zugkraft besitzt als die Antikörper(Fragment)-Antigen Bindung, öffnet sich beim Trennen der Oberflächen der Sensorkomplex, während die fluoreszenzmarkierte Sonde am Analyten gebunden bleibt.

### Herstellung des Stempels:

Es wurde ein mikrostrukturierter Stempel aus PDMS (Polydimethylsiloxan) gefertigt. Die Strukturen bestanden dabei aus Stempelfüßchen von ca. 100 x 100 µm, die durch Vertiefungen von ca. 25µm Breite und 1 µm Tiefe getrennt wurden. Hierzu wurde ein Ansatz aus einer 1:10 Mischung von Vemetzungsreagenz und Silikonelastomer (Sylgard 184, Dow Corning) nach mehrfachem Entgasen auf einen entsprechend strukturierten Siliziumwafer gegossen und für 24h bei RT inkubiert. Nach der Polymerisierung wurde die strukturierte Oberfläche des Stempel bei 1 mbar in einem Plasmaofen für 60s einem H₂O-Plasma ausgesetzt.

### Beschichtung des Stempels:

Die oxidierte Stempeloberfläche wurde mit 2% Aminopropyldimethylethoxysilan (ABCR, Karlsruhe, Deutschland) in 10% H₂O und 88% Ethanol für 30min inkubiert. Die silanisierte Oberfläche wurde mit Ethanol und mit Reinstwasser gewaschen und mit Stickstoff trocken geblasen. An die Aldehydgruppen des Silans wurde ein bifunktionales PEG angebunden, dessen eines Ende über eine NHS-Gruppe, das andere über eine Carboxygruppe verfügte. 25µl einer Lösung mit 20mM NHS-PEG-COOH (Shearwater, Huntsville) wurden unter einem Deckglas für 2h auf einem Stempel mit einer Fläche von 1 cm2 inkubiert. Es wurde mit Reinstwasser gewaschen und mit Stickstoff trocken geblasen.

Der erste Bindungspartner des Kraftsensors wurde über eine Aminogruppe aus 25 µM Lösung in PBS(Phosphate buffered Saline; Sigma) mit 5mg/ml EDC (1-Ethyl-3-(3-Dimethylamino-propyl)carbiimide; Sigma) und 5mg/ml NHS (N-hydroxy-succinimid; Sigma, St. Louis) aus 20µl pro cm2 unter einem Deckglas für 30 min an die Carboxygruppen des PEGs angebunden.

Die Sonde wurde durch Koppeln des zweiten Bindungspartners des Kraftsensors, einem mit Cy3 fluoreszenzmarkierten Oligo über eine Thiolgruppe an das C-terminale Cystein des Antikörperfragments hergestellt.

Die Sonden wurden aus 3µl Tröpfchen je Spot aus einer 25 µM Lösung in 5xSSC (saline sodium citric buffer; Sigma, Taufkirchen) bei RT anhybridisiert.

### Beschichtung der Unterlage:

Die Oberfläche wurde mit NaOH, 10g in 40ml H₂O und 60 ml Ethanol, für 2 Stunden behandelt. Die Stempeloberfläche wurde mit 3% Aminopropyldimethylethoxysilan (ABCR, Karlsruhe, Deutschland) in 10% H₂O und 87% Ethanol für 30min inkubiert. Die silanisierte Oberfläche wurde mit Ethanol und mit Reinstwasser gewaschen und mit Stickstoff trocken geblasen. An die Aminogruppen des Silans wurde ein bifunktionales NHS-PEG-Biotin (Shearwater, Huntsville) angebunden. 150µl einer Lösung mit 20mg/ml des PEG wurden unter einem 24x60mm^2 Deckglas für 1 h inkubiert. Es wurde mit Reinstwasser gewaschen und mit Stickstoff trocken geblasen.

An das Biotin wurde Streptavidin (Sigma, Taufkirchen, Deutschland) aus 0,1 mg/ml in PBS mit 0.05%Vol Tween angebunden. Dazu wurden 150 µl der Streptavidinlösung 30 min unter einem 24x60 mm2 Deckglas inkubiert. An das Streptavidin wurden die biotinylierten Rezeptor- Antikörper als 1µl Spots aus 0.5mg/ml in PBS mit 0.05%Vol Tween und 40% Glycerin angebunden. Das Anbinden der Analyten erfolgte aus einem Probengemisch in PBS mit 0.05%Vol Tween.

### Übertragen der Sonde:

Die Oberflächen wurden unter PBS mit 0.05%Vol Tween so einander angenähert, dass die auf dem Stempel gebundenen Sonden mit den auf der Unterlage gespotteten Analyten binden können. Nach einer Inkubationszeit von 30min wurden die Oberflächen voneinander getrennt.

### Messung:

Die Unterlage wurde mit einem Laser-Scanner nach der fluoreszenzmarkierten übertragenen Sonde abgescannt.

### Beispiel 6

### Probekomplex: Antikörper-Antigen; Sensorkomplex: DNA - DNA (Nachweis des Antikörpers)

Es sollen verschiedene nicht markierte Antikörper aus einer Mischung von Antikörpern nachgewiesen werden. Die Rezeptoren werden durch das jeweilige Antigen, das der Antikörper erkennen soll, dargestellt. Die Antigene werden kovalent an die Unterlage gebunden. Die zugehörigen Sonden werden durch Komplexe aus DNA und sekundären speziesspezifischen Antikörpern dargestellt, die die jeweiligen Proben-Antikörper spezifisch erkennen. Die Verbindung zwischen sekundärem Antikörper und DNA wird durch biotinylierte DNA und biotinylierte Antikörper realisiert, die durch Streptavidin verbunden werden. Der Sensorkomplex wird durch zwei reißverschlußartig hybridisierte DNA Moleküle dargestellt. Der erste Bindungspartner des Sensorkomplexes ist ein DNA Molekül, dessen 5'-Ende über eine Aminogruppe mit dem Stempel verbunden ist. Der zweite Bindungspartner des Sensorkomplexes wird durch ein fluoreszenzmarkiertes DNA Molekül dargestellt, das Biotin am 3'-Ende besitzt. Dieses Biotin ermöglicht die Anbindung von Streptavidin und damit die Verbindung mit dem biotinylierten sekundären Antikörper (vgl. Figur 12).

Die Sonden werden auf dem Stempel spiegelbildlich zu den Rezeptor-Spots der Unterlage lokalisiert, so dass die Spots mit den Sonden während des Kontaktes der Oberflächen stets auf den Spots mit den zugehörigen Rezeptoren zu liegen kommen. Ist ein Analyt an den Rezeptoren gebunden, kann in der Kontaktphase die zugehörige Sonde an den Analyten binden. Da der reißverschlußartig hybridisierte Sensorkomplex eine geringere Stabilität unter einer an das gesamte Konjugat angelegten Zugkraft besitzt als alle anderen Bindungen im Konjugat (Streptavidin-Biotin-Bindungen, Antigen-Antikörper-Wechselwirkung sowie die kovalente Bindung des Antigens an die Unterlage), öffnet sich beim Trennen der Oberflächen der Sensorkomplex, und die fluoreszenzmarkierte Sonde bleibt am Analyten gebunden.

### Herstellung des Stempels:

Es wurde ein mikrostrukturierter Stempel aus PDMS (Polydimethylsiloxan) gefertigt. Die Strukturen bestanden dabei aus Stempelfüßchen von ca. 100 x 100 µm, die durch Vertiefungen von ca. 25 µm Breite und 1 µm Tiefe getrennt wurden. Hierzu wurde ein Ansatz aus einer 1:10 Mischung von Vernetzungsreagenz und Silikonelastomer (Sylgard 184, Dow Corning) nach mehrfachem Entgasen zwischen einen entsprechend strukturierten Siliciumwafer und eins glatte Plexiglasplatte gegossen und senkrecht für 24h bei Raumtemperatur inkubiert. Nach der Polymerisierung wurde die strukturierte Oberfläche des Stempels bei 2 mbar in einem Plasmaofen für 60s einem H₂O-Plasma ausgesetzt.

### Beschichtung des Stempels:

Die oxidierte Oberfläche wurde mit einer Lösung aus 2% Aminopropyldimethylethoxysilan (ABCR, Karlsruhe, Deutschland) in 10% H₂O und 88% Ethanol für 30 min inkubiert. Die silanisierte Oberfläche wurde mit Ethanol und Reinstwasser gewaschen und mit Stickstoff trocken geblasen. An die Aminogruppen des Silans wurde ein bifunktionales PEG angebunden, dessen eines Ende über eine Amino-reaktive NHS-Gruppe (N-hydroxy-succinimid), das andere über eine Carboxygruppe verfügte. 25 µl einer 20 mM NHS-PEG-COOH (Shearwater, Huntsville, USA) Lösung (in H₂O) wurden unter einem runden Deckglas mit 12 mm Durchmesser für 2 h in feuchter Atmosphäre bei RT inkubiert. Es wurde mit Reinstwasser gewaschen und mit Stickstoff trocken geblasen. Noch nicht abreagierte Aminogruppen der Oberfläche wurden mit einer 6 mM Lösung (in H₂O von NHS-PEG-methoxy (Rapp Polymere, Tübingen, Deutschland) geblockt. 25 µl der Lösung wurden dazu 1h unter einem runden Deckglas inkubiert.

Nach diesem Schritt steht eine Oberfläche mit Carboxygruppen für die Kopplung des ersten Referenzkomplex-Oligos zur Verfügung. Dazu wurde der erste Referenzkomplex-Oligo (Sequenz: siehe Anhang, vgl. Beispiel4) mit einer wässrigen EDC Lösung (1-Ethyl-3-(3-Dimethylamino-propyl)carbümide; Sigma, Taufkirchen, Deutschland) gemischt. Die Endkonzentration an Oligo war 25 µM, die EDC Konzentration war 50 mM. Die frisch angesetzte Lösung wurde spotförmig ( 5 x 1 µl Spots) auf die Oberfläche aufgetragen und 2 h bei RT inkubiert. Anschließend wurde mit 1x SSC + 0,5 % SDS 2x 30 min gewaschen und über Nacht bei 4 °C mit 1 %gew Rinderserumalbumin (BSA) geblockt. Schließlich wurde mit Wasser nachgespült und mit Stickstoff trocken geblasen.

### Aufbau der Sonde:

Zur Hybridisierung des Unzip-Oligos mit Biotin (Sequenz: siehe Anhang; vgl Beispiel4) wurde eine 2 µM Lösung in 5x SSC hergestellt. 10 µl dieser Hybridisierungslösung wurden unter einem runden Deckglas (12 mm Durchmesser) 1h bei RT in feuchter Atmosphäre inkubiert. Es wurde 3 x 5 min mit 1x SSC + 0,1 % SDS gewaschen, mit Reinstwasser gespült und mit Stickstoff trocken geblasen.

Die Anbindung des Streptavidins (Sigma, Taufkirchen, Deutschland) erfolgte durch Schütteln der PDMS-Stücke in einer mit Streptavidin-Lösung gefüllten Schale. Die Konzentration an Streptavidin betrug 1 µg/ ml in PBS + 0,2 % BSA. Die PDMS-Stücke wurden in 5 ml Lösung 45 min bei RT geschüttelt und anschließend mit PBS + 0,05% Tween 20 gewaschen.

Ebenso wurde der biotinylierte sekundäre Anti-Maus Antikörper (Molecular Probes, Leiden, Niederlande) aus einem großen Lösungsvolumen (5 ml) an das Streptavidin gebunden. Die Antikörper-Stammlösung wurde ebenfalls auf 1 µg/ml in PBS + 0,2% BSA verdünnt. Die PDMS-Stücke wurden 1 h in dieser Lösung geschüttelt, mit PBS + 0,05% Tween gewaschen, mit Wasser gespült und mit Stickstoff trocken geblasen.

Bei den Ansätzen, in denen die Sonde mit dem Anti-Kaninchen Antikörper (Molecular Probes, Leiden, Niederlande) aufgebaut wurde, wurde mit diesem so verfahren, wie für den Anti-Maus Antikörper beschrieben.

### Beschichtung der Unterlage:

Als Unterlage wurden Aldehydsilan beschichtete Glasobjektträger (Typ CSS, Genpak, Brighton, UK) verwendet. An die Aldehydgruppen des Silans wurde ein bifunktionales PEG angebunden, dessen eines Ende über eine Aminogruppe, das andere über eine Carboxygruppe verfügte. 200 µl einer Lösung aus 6 mM HCl-NH2-PEG-COOH (Shearwater, Huntsville, USA) in H₂O wurde unter einem 24 x 60 mm2 Deckglas für 2 h in feuchter Atmosphäre bei 40 °C inkubiert. Es wurde mit Reinstwasser gewaschen und mit Stickstoff trocken geblasen. Anschließend wurden die Schiffschen Basen mit 1Gew.-% Natriumborhydrid in H₂O 30 min reduziert.

Nach diesem Schritt steht eine Oberfläche mit Carboxygruppen für die Kopplung der Antigene zur Verfügung. Dazu wurden die Carboxygruppen mit EDC und NHS (N-hydroxy-succinimid; Sigma, Taufkirchen, Deutschland) flächig aktiviert. Die Oberfläche wurde mit einer frisch angesetzten Lösung (200 µl) aus 50 mM EDC und 50 mM NHS unter einem 24 x 60 mm² Deckglas für 30 min in feuchter Atmosphäre bei RT inkubiert. Anschließend wurde mit Wasser gewaschen und mit Stickstoff trocken geblasen.

### Immobilisierung der Antigene:

Alle Antigene wurden ausgehend von ihrer Stammlösung in 2%vol Glycerin verdünnt (Endkonzentration: 20 µg/ml-100 µg/ml). Die eingesetzten Konzentrationen waren in Versuchen ermittelt worden, in denen die Antigene auf der Unterlage immobilisiert worden waren und im folgenden durch ihre monoklonalen und fluoreszenzmarkierte sekundäre Antikörper nachgewiesen worden waren. Diejenige Konzentration, bei der intensive und homogene Spots gemessen wurden, wurde für die weiteren Versuche verwendet. Die verwendeten Antigene und ihre optimalen Konzentrationen sind in folgender Tabelle zusammen gefasst:

| **Antigen** | **Molekulargewicht** | **Konzentration** | **Hersteller** |
|---|---|---|---|
| β-Galactosidase | 450 kDa | 50 µg/ml | Roche, Mannheim |
| α₁-Antitrypsin | 52 kDa | 100 µg/ml | Calbiochem, Darmstadt |
| Interferon γ | 16,7 kDa | 20 µg/ml | Biotrend, Köln |
| Troponin T | nicht bekannt | 25 µg/ml | Biotrend, Köln |

Die Spot-Lösungen der verschiedenen Antigene wurden als 1 µl Spots von Hand auf die Unterlage pipettiert. Die Kombination und Anordnung der verschiedenen Antigene war je nach Versuchsansatz unterschiedlich. Die Spots wurden 1 h bei RT in feuchter Atmosphäre auf der Oberfläche inkubiert und mit PBS abgewaschen. Die restlichen aktivierten Carboxygruppen neben den Spots wurden über Nacht mit 1%gew BSA in PBS geblockt. Anschließend wurde mit PBS + 0,05% Tween 20 gewaschen.

Für eine Negativkontrolle wurde ein Antigen benötigt, das von einem polyklonalen Antikörper, z.B. aus Kaninchen, erkannt wird. Dazu wurde ein BSA Konjugat von Aflatoxin B1 verwendet (Sigma, Taufkirchen, Deutschland). Das Konjugat wurde in einer Konzentration von 10 µg/ml nach obiger Vorschrift gespottet.

### Anbindung der Analyten (monoklonale Antikörper) aus einer Mischung:

Die monoklonalen Antikörper gegen obige Antigene sind in der folgenden Tabelle zusammengefasst:

| **Antikörper gegen** | **Spezies** | **Konzentration** | **Hersteller** |
|---|---|---|---|
| β-Galactosidase | Maus monoklonal | 0,5 µg/ml | Roche, Mannheim |
| α₁-Antitrypsin | Maus monoklonal | 0,5 µg/ml | Calbiochem, Darmstadt |
| Interferon γ | Maus monoklonal | 0,5 µg/ml | Biotrend, Köln |
| Troponin T | Maus monoklonal | 0,5 µg/ml | Biotrend, Köln |

Die Antikörper wurden in PBS + 0,2 % BSA auf die angegebene Konzentration verdünnt. Die Inkubation erfolgte in 4 ml Lösung 1 h bei RT auf einem Schüttler. Welche Antikörper der Inkubationslösung zugesetzt wurden, war abhängig vom jeweiligen Versuchsansatz. Nach der Inkubation wurden die Objektträger in PBS + 0,05% Tween gewaschen, mit Wasser abgespült und mit Stickstoff trocken geblasen.

Als Negativkontrolle wurde an das Aflatoxin B1 BSA Konjugat ein polyklonaler Antikörper aus Kaninchen angebunden. Auch dieser wurde je nach Ansatz der Probenlösung zugegeben. Dabei wurde der polyklonale Antikörper 1:8000 in PBS + 0,2% BSA verdünnt. Dieser Kaninchen Antikörper darf beim Stempeln nur durch eine Sonde mit einem Anti-Kaninchen Antikörper markiert werden, und nicht durch den Anti-Maus Antikörper, der zum Nachweis der monoklonalen Antikörper verwendet wird.

### Stempeln:

Die Oberflächen wurden unter PBS so einander angenähert, dass die auf dem Stempel spotförmig gebundenen Sonden mit den auf der Unterlage befindlichen Analyten in Kontakt kommen und wechselwirken können. Nach einer Inkubationszeit von 30min wurden die Oberflächen voneinander getrennt.

### Messung:

Die Unterlage wurde mit einem Laser-Scanner nach der fluoreszenzmarkierten übertragenen Sonde abgescannt.

### Auswertung und Ergebnis:

Die Spezifität des Übertrages der fluoreszenzmarkierten Sonde wurde in 2 verschiedenen Ansätzen nachgewiesen. Im ersten Ansatz sollte nachgewiesen werden, dass ein signifikant höherer Übertrag der Fluoreszenz stattfindet, wenn der Analyt an sein Antigen gebunden hat, als wenn ein direkter Kontakt zwischen Rezeptor und Sonde vorliegt (vgl. Figur 13). Der zweite Ansatz zeigt, dass gebundene Analyten nur dann durch die Sonde markiert werden, wenn die Sonde spezifisch für den jeweiligen Analyten ist (vgl. Figur 17).

### 1. Ansatz: Übertrag der Sonde nur bei gebundenem Analyten

Dieser Versuchsansatz war so aufgebaut, dass ein Vergleich der Überträge erfolgen kann, wenn zum einen der Analyt aus Lösung an den Rezeptor gebunden hat und zum anderen keine Analyt an den Rezeptor gebunden hat, so dass ein direkter Kontakt zwischen Sonde und Rezeptor stattfinden kann.

Dazu wurde die Sonde aus Unzip-Komplex, Streptavidin und dem sekundären Antikörper (Anti-Maus) aufgebaut. Dabei wurden 4 Spots so plaziert, dass jeder auf einen der 4 Antigen-Spots auf der Unterlage treffen. Ein weiterer Spot wurde so angeordnet, dass er beim Stempeln keinen Spot auf der Unterlage trifft. Dieser Spot stellt eine Kontrolle für einen unspezifischen Übertrag auf die Passivierung dar. Auf der Unterlage wurden in 2 festgelegten Bereichen jeweils die 4 verschiedenen Antigene gespottet. Dazu wurde jeweils 1 µl jedes Antigens auf die Unterlage pipettiert. Die Anordnung der Spots auf beiden Oberflächen erfolgte nach folgendem

Schema:    Unterlage:    Stempel:

Troponin T β-Galactosidase    Anti-Maus Anti-Maus

Anti-Maus

Interferon γ α1-Antitrypsin    Anti-Maus Anti-Maus

Ein spezifischer Übertrag der Fluoreszenzmarkierung des Kraftsensors während des Stempelns ist nachgewiesen, wenn die Rezeptor-Spots (Antigen), deren monoklonaler Antikörper der Probenlösung zugegeben wurde, eine deutlich höhere Fluoreszenz aufweisen, als die Antigen-Spots deren Antikörper in diesem Ansatz nicht vorhanden ware. Es wurden 2 Ansätze durchgeführt, wobei die Probenlösung in einem Ansatz den Anti-β-Galactosidase- und den Anti-Interferon γ- Antikörper enthielt und im anderen Ansatz die Antikörper gegen Troponin T und α1-Antitrypsin. Alle Ansätze wurden mehrfach wiederholt und Mittelwerte ermittelt.

Figur 14 zeigt die am Scanner gemessenen Überträge an Fluoreszenzmarkierung auf die Unterlage, wenn die Probenlösung den Anti-β-Galactosidase und den Anti-Interferon γ Antikörper enthielt. Figur 15 zeigt den Ansatz mit den Antikörpern gegen Troponin T und α1-Antitrypsin.

Zur Auswertung der Scans wurde das Programm NIH Image verwendet. Dieses Programm ermöglicht es, über ausgewählte Bereiche Histogramme zu erstellen. Die Histogramme zeigen die Häufigkeiten der gemessenen Intensitäten im ausgewählten Bereich. Aus den Histogrammen wurde die mittlere Intensität der Kontaktflächen mit den Stempelfüßchen ermittelt, sowie die mittlere Intensität an den Stellen, wo sich die Vertiefungen des PDMS befanden. Diese Werte wurden als Hintergrundfluoreszenz von den Intensitäten in den Kontaktflächen subtrahiert, um die Intensität des Übertrages zu erhalten. Die Mittelwerte und Standardabweichungen der Messungen sind in folgender Tabelle sowie Figur 16 zusammengefasst:

| | Intensitäten des Fluoreszenzübertrages bei Zugabe von Anti- | |
|---|---|---|
| - Antigen | β-Galactosidase & Interferon γ | α₁-Antitrypsin & Troponin T |
| β-Galactosidase | 16643 ± 2227 | 2018 ± 488 |
| α₁-Antitrypsin | 2335 ± 1285 | 12176 ± 1744 |
| Interferon γ | 13366 ± 1362 | 8022 ± 1751 |
| Troponin T | 2820 ± 907 | 11613 ± 457 |
| Passivierung | 2681 ± 1247 | 3216 ± 224 |

Der Vergleich der Überträge auf ein Antigen, wenn Antikörper gebunden war bzw. wenn ein direkter Kontakt zwischen Rezeptor und Sonde vorlag, zeigt für β-Galactosidase, α₁-Antitrypsin und Troponin T, dass der Übertrag mit Antikörper signifikant höher ist, als der Übertrag auf die Passivierung oder direkt aufs Antigen. Das Verhältnis von spezifischem zu unspezifischem Übertrag erlaubt daher die Schlußfolgerung, dass es sich beim Übertrag der Fluoreszenzmarkierung auf den Antikörper um einen spezifischen Übertrag handelt. Für diese Proteine liegt der unspezifische Übertrag in der gleichen Größenordnung wie der Übertrag auf die Passivierung. Für Interferon γ ist der Unterschied zwischen spezifischem und unspezifischem Übertrag deutlich geringer. Dennoch überwiegt der spezifische Anteil.

### 2. Ansatz: Übertrag der Sonde nur bei spezifischer Interaktion zwischen Analyt und Sonde

Dieser Versuchsansatz war so aufgebaut, dass ein Vergleich der Überträge erfolgen kann, wenn zum einen die Sonde spezifisch für den Analyten ist und zum anderen der Analyt mit einer nicht spezifischen Sonde in Kontakt gebracht wird.

Dazu wurde die Sonde auf dem Stempel entweder aus Unzip-Komplex, Streptavidin und sekundären Anti-Maus Antikörper oder aus Unzip-Komplex, Streptavidin und sekundären Anti-Kaninchen Antikörper aufgebaut. 5 Spots wurden so plaziert, dass jeder einen der 5 Antigen-Spots auf der Unterlage trifft. Auf der Unterlage wurden in 2 definierten Bereichen jeweils die 5 verschiedenen Antigene gespottet. Dazu wurde jeweils 1 µl jedes Antigens auf die Unterlage pipettiert. Die Anordnung der Spots auf beiden Oberflächen erfolgte nach folgendem Schema:

Unterlage:                                    Stempel:

Troponin  T β-Galactosidase          sek. Antikörper          sek. Antikörper

AflatoxinB1-BSA                                sek. Antikörper

Interferon γ          α₁-Antitrypsin          sek. Antikörper          sek. Antikörper

Ein spezifischer Übertrag der Fluoreszenzmarkierung ist nachgewiesen, wenn nach dem Stempeln die Antigenspots, die ihren monoklonalen Antikörper (aus Maus) gebunden haben, eine deutlich höhere Fluoreszenz aufweisen, wenn die Sonde den Anti-Maus Antikörper enthält, als das AflatoxinB1-BSA, das einen polyklonalen Antikörper aus Kaninchen bindet. Andererseits muss der Spot aus AflatoxinB1-BSA eine höhere Intensität als die anderen Antigenspots aufweisen, wenn die Sonde den Anti-Kaninchen Antikörper enthält.

Es wurden 2 Ansätze durchgeführt, wobei die Sonde im einen Ansatz den Anti-Maus Antikörper enthielt und im anderen Ansatz den Anti-Kaninchen Antikörper. Alle Ansätze wurden mehrfach wiederholt und Mittelwerte aus 3 Messungen ermittelt.

Figur 18 zeigt die am Scanner gemessenen Überträge an Fluoreszenzmarkierung auf die Unterlage, wenn der Anti-Maus Antikörper für die Sonde verwendet wurde. Figur 19 zeigt den Ansatz mit dem Anti-Kaninchen Antikörper.

Die Auswertung der Scans erfolgte wie für Ansatz 1 beschrieben. Die Mittelwerte und Standardabweichungen der Messungen sind in folgender Tabelle sowie Figur 20 zusammengefasst:

| | **Intensitäten der Fluoreszenzübertrage für eine Sonde mit Anti-** | |
|---|---|---|
| Antigen | **Maus** | **Kaninchen** |
| β-Galactosidase | 8955 ± 1861 | 3279 ± 1138 |
| α₁-Antitrypsin | 7035 ± 300 | 1947 ± 977 |
| Interferon γ | 9358 ± 4131 | 2788 ± 1053 |
| Troponin T | 4394 ± 1250 | 1582 ± 659 |
| AflatoxinB1-BSA | 2822 ±1577 | 21168 ± 12524 |

Der Vergleich der Überträge mit dem Anti-Maus Antikörper und dem Anti-Kaninchen Antikörper zeigt deutlich, dass β-Galactosidase, α1-Antitrypsin, Troponin T und Interferon γ vom Anti-Maus Antikörper signifikant stärker fluoreszenzmarkiert werden als vom Anti-Kaninchen Antikörper. Da diese Proteine die monoklonalen Antikörper (aus Maus) aus der Probenlösung binden, findet im Fall einer spezifischen Interaktion ein etwa 3fach höherer Übertrag mit dem Anti-Maus Antikörper statt. Der Übertrag auf den AflatoxinB1-BSA Spot stellt in diesem Ansatz den unspezifischen Übertrag dar (vgl. Figur 18). Andererseits muss beim Stempeln mit dem Anti-Kaninchen Antikörper bei einer spezifischen Wechselwirkung ein signifikant höherer Übertrag auf das AflatoxinB1-BSA erfolgen. Auch dies ist hier der Fall. Hier zeigen die anderen 4 Antigenspots den unspezifischen Übertrag (vgl. Figur 19). Der Vergleich der unspezifischen Überträge zeigt, dass diese unabhängig von der verwendeten Sonde etwa gleich groß sind. Dies deutet darauf hin, dass die Größenordnung des unspezifischen Übertrages durch die Versuchsanordnung selbst bedingt ist und die spezifischen Überträge je nach Protein schwanken. So kann zum Beispiel mit dem hier verwendeten polyklonalen Antikörper als Analyt ein deutlich höherer Übertrag erreicht werden, als mit den monoklonalen Antikörpern.

### Beispiel 7

### Probekomplex: Antikörper-Antigen; Sensorkomplex: DNA - DNA (Nachweis des Antigens)

Ein Antigen soll parallel auf 4 Spots spezifisch nachgewiesen werden. Die Rezeptormoleküle werden durch einen Antikörper, der das Antigen erkennen soll, dargestellt. Die Antikörper werden kovalent an die Unterlage gebunden. Die zugehörige Sonde wird durch Komplexe aus DNA und einem zweiten biotinylierten Antikörper dargestellt, der gegen das Antigen gerichtet ist. Die Verbindung zwischen diesem biotinylierten Antikörper und der DNA wird durch biotinylierte DNA und Streptavidin realisiert. Als Sensorkomplex werden zwei reißverschlußartig hybridisierte DNA-Moleküle verwendet. Der erste Bindungspartner des Sensorkomplexes ist ein DNA Molekül, dessen 5'-Ende über eine Aminogruppe mit dem Stempel verbunden ist. Der zweite Bindungspartner des Sensorkomplexes wird durch ein fluoreszenzmarkiertes DNA Molekül dargestellt, an dessen 3'-Ende Biotin gebunden ist. Dieses Biotin ermöglicht die Anbindung von Streptavidin und damit die Verbindung mit dem biotinylierten Antikörper (vgl. Figur 21).

Die Sonden werden auf dem Stempel spiegelbildlich zu den Rezeptor-Spots der Unterlage so lokalisiert, dass die Spots mit den Sonden während des Kontaktes der Oberflächen stets den Spots mit den zugehörigen Rezeptoren gegenüberliegen. Hat ein Analyt an ein Rezeptormolekül gebunden, kann in der Kontaktphase die zugehörige Sonde an den Analyten binden. Wenn dann beim Trennen von Stempel und Unterlage auf die gebildeten Bindungen eine Zugkraft ausgeübt wird, muss die schwächste Bindung aufgehen. Da der reißverschlußartig hybridisierte Sensorkomplex eine geringere Stabilität unter einer an das gesamte Konjugat angelegten Zugkraft besitzt als die anderen Bindungen im Konjugat, nämlich Streptavidin-Biotin-Bindungen, Antigen-Antikörper-Wechselwirkung sowie die kovalente Bindung des Antikörpers an die Unterlage, öffnet sich beim Trennen der Oberflächen der Sensorkomplex, und die fluoreszenzmarkierte Sonde bleibt am Analyten gebunden.

### Herstellung des Stempels:

Es wurde ein mikrostrukturierter Stempel aus PDMS (Polydimethylsiloxan) gefertigt. Die Strukturen bestanden dabei aus Stempelfüßchen von ca. 100 x 100 µm, die durch Vertiefungen von ca. 25 µm Breite und 1 µm Tiefe getrennt wurden. Hierzu wurde ein Ansatz aus einer 1:10 Mischung von Vernetzungsreagenz und Silikonelastomer (Sylgard 184, Dow Corning) nach mehrfachem Entgasen zwischen einen entsprechend strukturierten Siliziumwafer und eine glatte Plexiglasplatte gegossen und senkrecht für 24h bei Raumtemperatur inkubiert. Nach der Polymerisierung wurde die strukturierte Oberfläche des Stempels bei 2 mbar in einem Plasmaofen für 60s einem H₂O-Plasma ausgesetzt.

### Beschichtung des Stempels:

Die oxidierte Oberfläche wurde mit einer Lösung aus 2% Aminopropyldimethylethoxysilan (ABCR, Karlsruhe, Deutschland) in 10% H₂O und 88% Ethanol für 30 min inkubiert. Die silanisierte Oberfläche wurde mit Ethanol und Reinstwasser gewaschen und mit Stickstoff trocken geblasen. An die Aminogruppen des Silans wurde ein bifunktionales PEG angebunden, dessen eines Ende über eine Amino-reaktive NHS-Gruppe (N-hydroxy-succinimid), das andere über eine Carboxygruppe verfügte. 25 µl einer 20 mM NHS-PEG-COOH (Shearwater, Huntsville, USA) Lösung (in H₂O) wurden unter einem runden Deckglas mit 12 mm Durchmesser für 2 h in feuchter Atmosphäre bei RT inkubiert. Es wurde mit Reinstwasser gewaschen und mit Stickstoff trocken geblasen. Noch nicht abreagierte Aminogruppen der Oberfläche wurden mit einer 6 mM Lösung (in H₂O von NHS-PEG-methoxy (Rapp Polymere, Tübingen, Deutschland) geblockt. 25 µl der Lösung wurden dazu 1h unter einem runden Deckglas inkubiert.

Nach diesem Schritt steht eine Oberfläche mit Carboxygruppen für die Kopplung des ersten Referenzkomplex-Oligos zur Verfügung. Dazu wurde der erste Referenzkomplex-Oligo (Sequenz siehe Beispiel 4) mit einer wässrigen EDC Lösung (1-Ethyl-3-(3-Dimethylamino-propyl)carbiimide ; Sigma, Taufkirchen, Deutschland) gemischt. Die Endkonzentration an Oligo war 25 µM, die EDC Konzentration war 50 mM. Die frisch angesetzte Lösung wurde spotförmig ( 5 x 1 µl Spots) auf die Oberfläche aufgetragen und 2 h bei RT inkubiert. Anschließend wurde mit 1x SSC + 0,5 % SDS 2x 30 min gewaschen und über Nacht bei 4 °C mit 1%gew Rinderserumalbumin (BSA) geblockt. Schließlich wurde mit Wasser nachgespült und mit Stickstoff trocken geblasen.

### Aufbau der Sonde:

Zur Hybridisierung des Unzip-Oligos mit Biotin (Sequenz siehe Beispiel 4) wurde eine 2 µM Lösung in 5x SSC hergestellt. 10 µl dieser Hybridisierungslösung wurden unter einem runden Deckglas (12 mm Durchmesser) 1h bei RT in feuchter Atmosphäre inkubiert. Es wurde 3 x 5 min mit 1 x SSC + 0,1 % SDS gewaschen, mit Reinstwasser gespült und mit Stickstoff trocken geblasen.

Zur Anbindung des Streptavidins (Sigma, Taufkirchen, Deutschland) wurden 60 µl einer Streptavidinlösung auf ein PDMS-Stück pipettiert. Die Konzentration an Streptavidin betrug 20 µg/ml in PBS + 0,2 % BSA. Die PDMS-Stücke wurden 45 min bei RT inkubiert und anschließend mit PBS +0,05% Tween 20 gewaschen.

Der biotinylierte Antikörper war ein gegen His-Tags gerichteter Antikörper (Dianova, Hamburg). Dieser Antikörper-wurde auf 20 µg/ml in PBS + 0,2% BSA verdünnt. Die PDMS-Stücke wurden mit 60 µl dieser Lösung 1 h inkubiert, mit PBS + 0,05% Tween gewaschen, mit Wasser gespült und mit Stickstoff trocken geblasen.

### Beschichtung der Unterlage:

Als Unterlage wurden Aldehydsilan beschichtete Glasobjektträger (Typ CSS, Genpak, Brighton, UK) verwendet. An die Aldehydgruppen des Silans wurde ein bifunktionales PEG angebunden, dessen eines Ende über eine Aminogruppe, das andere über eine Carboxygruppe verfügte. 200 µl einer Lösung aus 6 mM HCl-NH₂-PEG-COOH (Shearwater, Huntsville, USA) in H₂O wurden unter einem 24 x 60 mm² Deckglas für 2 h in feuchter Atmosphäre bei 40 °C inkubiert. Es wurde mit Reinstwasser gewaschen und mit Stickstoff trocken geblasen. Anschließend wurden die Schiffschen Basen mit 1%gew Natriumborhydrid in H₂O 30 min reduziert.

Nach diesem Schritt steht eine Oberfläche mit Carboxygruppen für die Kopplung der Antikörper zur Verfügung. Dazu wurden die Carboxygruppen mit EDC und NHS (N-hydroxy-succinimid; Sigma, Taufkirchen, Deutschland) flächig aktiviert. Die Oberfläche wurde mit einer frisch angesetzten Lösung (200 µl) aus 50 mM EDC und 50 mM NHS unter einem 24 x 60 mm² Deckglas für 30 min in feuchter Atmosphäre bei RT inkubiert. Anschließend wurde mit Wasser gewaschen und mit Stickstoff trocken geblasen.

### Immobilisierung des Antikörpers:

Ein Antikörper gegen das Protein p53 (Klon Pab421, Calbiochem, Darmstadt) wurde ausgehend von der Stammlösung auf eine Endkonzentation von 100 µg/ml in 2 Vol.-% Glycerin verdünnt.

Die Spot-Lösung des Antikörpers wurden als 1 µl Spots (insgesamt 4 Spots) von Hand auf die Unterlage pipettiert. Die Spots wurden 1 h bei RT in feuchter Atmosphäre auf der Oberfläche inkubiert und mit PBS abgewaschen. Die restlichen aktivierten Carboxygruppen neben den Spots wurden über Nacht mit 1 %gew BSA in PBS geblockt. Anschließend wurde mit PBS + 0,05% Tween 20 gewaschen.

### Anbindung des Analyten (rekombinantes p53 mit His-Tag) aus Kaninchenserum:

Das Antigen (p53-His, Positivkontrolle für anti-His Antikörper, Dianova, Hamburg) wurde in Kaninchenserum auf eine Konzentration von 2 µg/ml verdünnt. 40 µl dieser Lösung wurden 1 h bei RT auf den Spots inkubiert. Nach der Inkubation wurden die Objektträger in PBS + 0,05% Tween gewaschen, mit Wasser abgespült und mit Stickstoff trocken geblasen.

### Stempeln:

Die Oberflächen wurden unter PBS so einander angenähert, dass die auf dem Stempel spotförmig gebundenen Sonden mit den auf der Unterlage befindlichen Analyten in Kontakt kommen und wechselwirken können. Nach einer Inkubationszeit von 30min wurden die Oberflächen voneinander getrennt.

### Messung:

Die Unterlage wurde mit einem Laser-Scanner nach der fluoreszenzmarkierten übertragenen Sonde abgescannt.

### Auswertung und Ergebnis:

Um die Spezifität des Übertrages zeigen zu können, waren die Spots auf Stempel und Unterlage nach folgendem Schema angeordnet:

Stempel Unterlage

anti-His anti-His anti-p53/ anti-p53/
p53-His p53-His

anti-His

anti-His anti-His anti-p53/ anti-p53/
p53-His p53-His

Bei dieser Anordnung können beim Stempeln 4 Spots der Sonde mit dem Antigen, das vom anti-p53 Antikörper gebunden wurde, wechselwirken. Der mittlere Spot der Sonde dagegen hat nur Kontakt zur Passivierung der Unterlage. Liegt dem Übertrag der Fluorphore der Sonde auf die Unterlage eine spezifische Wechselwirkung zugrunde, muß der Übertrag auf die Antigene signifikant höher sein als der Übertrag auf die Passivierung.

Dies konnte in oben beschriebenem Experiment, bei dem 4 Stempelversuche parallel durchgeführt wurden, bestätigt werden. Ein Fluoreszenz-Scan ist in Figur 22 dargestellt. Für einen Stempelversuch beträgt der Mittelwert (4 Spots) der berechneten Intensitätswerte (siehe Bsp. 6) für den Übertrag auf die Antigene 4624 ± 409. Für diesen Versuch beträgt der Übertrag auf die Passivierung 673. Demzufolge ist der spezifische Übertrag um den Faktor 7 höher als der unspezifische. Der Mittelwert für das Verhältnis aus spezifischem und unspezifischem Übertrag für die 4 durchgeführten Versuche beträgt 7,2 ± 2,1.

## Patentansprüche

1. Verfahren zur Bestimmung eines Analyten in einer Probe, **dadurch gekennzeichnet, daß** man
eine Probe, die möglicherweise den zu bestimmenden Analyten enthält, mit einer ersten Oberfläche (= Unterlage) in Kontakt bringt, so dass der möglicherweise in der Probe enthaltene Analyt an die Unterlage gebunden wird; eine zweite Oberfläche (= Stempel), an die eine Sonde, die an den Analyt binden kann, gebunden ist, an die Unterlage annähert, so dass Sonde und Analyt interagieren können, wobei die Bindung der Sonde an den Analyt und die Bindung des Analyten an die Unterlage unter einer von außen angelegten Zugkraft stabiler ist als die Bindung der Sonde an den Stempel;
Stempel und Unterlage voneinander trennt; und
bestimmt, ob die Sonde an die Unterlage gebunden ist und/oder wie viel Sonde an die Unterlage gebunden ist.

2. Verfahren nach Anspruch 1, **dadurch gekennzeichnet, dass** auf der Unterlage Moleküle, die den Analyten binden können (= Rezeptoren), immobilisiert sind, wobei die Bindung des Analyten an den Rezeptor unter einer von außen angelegten Zugkraft stabiler ist als die Bindung der Sonde an den Stempel.

3. Verfahren nach Anspruch 2, **dadurch gekennzeichnet, dass** die Rezeptoren und/oder Sonden ausgewählt sind aus der Gruppe bestehend aus polyklonalen oder monoklonalen Antikörpern, Antikörperfragmenten oder Antikörperderivaten, die den Analyten erkennen und binden können.

4. Verfahren nach Anspruch 3, **dadurch gekennzeichnet, dass** als Rezeptoren und/oder Sonden Antikörperfragmente oder -derivate ausgewählt aus Fv-, Fab-, Fab'- oder F(ab')₂-Fragmenten, "single-chain antibody fragments", bispezifischen Antikörpern, chimären Antikörpern, humanisierten Antikörpern sowie Fragmenten, die CDRs (complementarity determining regions) enthalten, die ein Epitop des Analyten erkennen, verwendet werden.

5. Verfahren nach Anspruch 1 oder Anspruch 2, **dadurch gekennzeichnet, dass** als Rezeptoren und/oder Sonden Nucleinsäuren wie DNA (Desoxyribonucleinsäure) oder RNA (Ribonucleinsäure) und künstliche Nucleinsäuren wie LNA (Locked Nucleic Acid) und PNA (Peptide Nucleic Acid) und dreidimensionale Strukturen aus Nucleinsäuren, bevorzugt Aptamere verwendet werden.

6. Verfahren nach einem der vorhergehenden Ansprüche, **dadurch gekennzeichnet, dass** der Analyt ausgewählt ist aus der Gruppe bestehend aus Proteinen, Polypeptiden, Peptiden, Nucleinsäuren, modifizierten Nucleinsäuren, Nucleinsäure ähnlichen Molekülen wie Peptide Nucleic Acid (PNA) oder Locked Nucleic Acid (LNA), dreidimensionalen Nucleinsäurestrukturen, Aptameren, Kohlenhydraten und modifizierten Varianten davon.

7. Verfahren nach Anspruch 6, **dadurch gekennzeichnet, dass** der Analyt ein Protein, Polypeptid oder Peptid ist.

8. Verfahren nach einem der vorhergehenden Ansprüche, **dadurch gekennzeichnet, dass** die Sonde spezifisch an den Analyt bindet.

9. Verfahren nach einem der vorhergehenden Ansprüche, **dadurch gekennzeichnet, dass** die Sonde ausgewählt ist aus der Gruppe bestehend aus Antikörpern, Antikörperfragmenten und Antikörperderivaten.

10. Verfahren nach einem der vorhergehenden Ansprüche, **dadurch gekennzeichnet, dass** die Sonde eine Markierung aufweist.

11. Verfahren nach einem der vorhergehenden Ansprüche, **dadurch gekennzeichnet, dass** die Sonde am Stempel über einen Sensorkomplex immobilisiert ist.

12. Verfahren nach Anspruch 11, **dadurch gekennzeichnet, dass** der Sensorkomplex einen ersten Bindungspartner, der mit dem Stempel verbunden ist und einen zweiten Bindungspartner, der fest mit der Sonde verbunden ist, umfasst, wobei der erste Bindungspartner und der zweite Bindungspartner nicht-kovalent aneinander binden können.

13. Verfahren nach Anspruch 11 oder Anspruch 12, **dadurch gekennzeichnet, dass** der erste Bindungspartner und der zweite Bindungspartner Nucleinsäuren sind.

14. Verfahren nach Anspruch 13, **dadurch gekennzeichnet, dass** der erste Bindungspartner und der zweite Bindungspartner ausgewählt sind aus der Gruppe bestehend aus einzelsträngiger DNA und/oder einzelsträngiger RNA.

15. Verfahren nach Anspruch 14, **dadurch gekennzeichnet, dass** das 5'-Ende des ersten Bindungspartners mit dem Stempel verbunden ist und das 3'-Ende des zweiten Bindungspartners mit der Sonde verbunden ist oder dass das 3'-Ende des ersten Bindungspartners mit dem Stempel verbunden ist und das 5'-Ende des zweiten Bindungspartners mit der Sonde verbunden ist.

16. Verfahren nach einem der vorhergehenden Ansprüche, **dadurch gekennzeichnet, dass** der Stempel mehrere voneinander getrennte Bereiche aufweist, in denen Sonden mit unterschiedlicher Spezifität für unterschiedliche Analyten gebunden sind.

17. Verfahren nach einem der Ansprüche 2 bis 16, **dadurch gekennzeichnet, dass** die Unterlage mehrere voneinander getrennte Bereiche aufweist, in denen Rezeptoren mit unterschiedlicher Spezifität für unterschiedliche Analyten gebunden sind.

18. Verfahren nach Anspruch 16 oder 17, **dadurch gekennzeichnet, dass** die Unterlage mehrere voneinander getrennte Bereiche aufweist, in denen Rezeptoren mit unterschiedlicher Spezifität für unterschiedliche Analyten gebunden sind, derart dass sie symmetrisch zu den sich auf dem Stempel befindenden Bereichen sind, so dass bei dem Annähern von Stempel und Unterlage jeweils die Bereiche der Sonden und Rezeptoren, die für den selben Analyten spezifisch sind, zur Deckung kommen.

19. Verfahren nach Anspruch 16 oder 17, **dadurch gekennzeichnet, dass** Sowohl Unterlage als auch Stempel Bereiche aufweisen, in denen Rezeptoren immobilisiert sind als auch Bereiche, in denen Sonden immobilisiert sind, wobei die Bereiche jeweils so angeordnet sind, dass bei Inkontaktbringen von Stempel und Unterlage sich Bereiche mit Rezeptoren für einen Analyten und Bereiche mit Sonden für den gleichen Analyten gegenüberliegen.

20. Verfahren nach einem der vorhergehenden Ansprüche, **dadurch gekennzeichnet, dass** Stempel und/oder Unterlage Flächenabschnitte aufweisen, auf denen mehrere Spots angeordnet sind.

21. Verfahren nach Anspruch 20, **dadurch gekennzeichnet, dass** Stempel und/oder Unterlage Flächenabschnitte mit mehreren Spots aufweisen, wobei die Spots eines Flächenabschnitts jeweils verschiedene mit einem Analyten bindefähige Rezeptoren oder Sonden aufweisen.

22. Verfahren nach Anspruch 20, **dadurch gekennzeichnet, dass** Stempel und/oder Unterlage mindestens einen Spot aufweisen, auf dem Rezeptoren immobilisiert sind, die direkt mit der Sonde bindefähig sind, wobei diese Spots zur Kontrolle der Kopplung verwendet werden.

23. Vorrichtung zur Bestimmung eines Analyten in einer Probe umfassend zwei Oberflächen, die jeweils Flächenabschnitte aufweisen, an die mit unterschiedlichen Analyten bindefähigere Rezeptoren und Sonden gebunden sind, wobei Rezeptoren und Sonden jeweils so angeordnet sind, dass bei einem Inkontaktbringen der beiden Oberflächen die jeweils mit dem selben Analyten bindefähigen Rezeptoren bzw. Sonden aneinander gegenüber liegen, wobei die Sonden jeweils über einen Sensorkomplex immobilisiert sind und eine Markierung tragen, wobei der Sensorkomplex eine Kombination aus mindestens zwei bindefähigen Molekülen ist, die bei Anwendung einer Zugkraft getrennt werden können, wobei die Kombination so ausgewählt wird, dass die Zugkraft, die zu ihrer Trennung notwendig ist, unter den gegebenen Versuchsbedingungen größer ist als die Zugkraft, die zur Trennung von unspezifisch gebundenen Molekülen notwendig ist, aber kleiner ist als die Zugkraft, die zur Trennung des spezifisch gebundenen Analyten von seinen Bindepartnern oder zur Abtrennung der immolisierten Rezeptoren notwendig ist.

24. Vorrichtung nach Anspruch 23, **dadurch gekennzeichnet, dass** die Vorrichtung einen Stempel und eine Unterlage umfasst und dass ein erster Bindungspartner fest mit dem Stempel verbunden ist und ein zweiter Bindungspartner fest mit der Sonde verbunden ist, wobei der erste Bindungspartner und der zweite Bindungspartner nicht-kovalent aneinander binden können.

25. Vorrichtung nach Anspruch 24, **dadurch gekennzeichnet, dass** der erste Bindungspartner und der zweite Bindungspartner Nucleinsäuren sind.

26. Vorrichtung nach Anspruch 25, **dadurch gekennzeichnet, dass** der erste Bindungspartner und der zweite Bindungspartner ausgewählt sind aus der Gruppe bestehend aus einzelsträngiger DNA und/oder einzelsträngiger RNA.

27. Vorrichtung nach Anspruch 26, **dadurch gekennzeichnet, dass** das 5'-Ende des ersten Bindungspartners mit dem Stempel verbunden ist und das 3'-Ende des zweiten Bindungspartners mit der Sonde verbunden ist oder dass das 3'-Ende des ersten Bindungspartners mit dem Stempel verbunden ist und das 5'-Ende des zweiten Bindungspartners mit der Sonde verbunden ist.

28. Vorrichtung nach einem der Ansprüche 23 bis 27, **dadurch gekennzeichnet, dass** die Vorrichtung weiterhin eine Einrichtung zum Annähern und Trennen der beiden Oberflächen umfasst.

29. Vorrichtung nach einem der Ansprüche 23 bis 28, **dadurch gekennzeichnet, dass** die Vorrichtung weiterhin eine Einrichtung zum Nachweis, ob und/oder in welcher Menge die Sonde an die Unterlage gebunden ist, umfasst.

30. Vorrichtung nach Anspruch 29, **dadurch gekennzeichnet, dass** die Vorrichtung eine Einrichtung zum Nachweis der Markierung aufweist.

31. Vorrichtung nach einem der Ansprüche 23 bis 30, **dadurch gekennzeichnet, dass** der Stempel mehrere voneinander getrennte Bereiche aufweist, in denen Sonden mit unterschiedlicher Spezifität für unterschiedliche Analyten gebunden sind.

32. Vorrichtung nach einem der Ansprüche 23 bis 31, **dadurch gekennzeichnet, dass** die Unterlage mehrere voneinander getrennte Bereiche aufweist, in denen Rezeptoren mit unterschiedlicher Spezifität für unterschiedliche Analyten gebunden sind.

33. Vorrichtung nach Anspruch 31 oder 32, **dadurch gekennzeichnet, dass** die Unterlage mehrere voneinander getrennte Bereiche aufweist, in denen Rezeptoren mit unterschiedlicher Spezifität für unterschiedliche Proben gebunden sind, derart, dass sie symmetrisch zu den sich auf dem Stempel befindenden Bereichen sind, so dass bei dem Annähern von Stempel und Unterlage jeweils die Bereiche der Sonden und Rezeptoren, die für die selbe Probe spezifisch sind, zur Deckung kommen.

34. Vorrichtung nach einem der Ansprüche 23 bis 33, **dadurch gekennzeichnet, dass** die Bereiche jeweils mehrere Spots aufweisen, an denen jeweils unterschiedliche mit einem Analyten bindefähige Rezeptoren bzw. Sonden angeordnet sind.

## Claims

1. Method for determining an analyte in a sample,
**characterised in that**
a sample, which possibly contains the analyte to be determined, is brought into contact with a first surface (substrate), so that the analyte possibly contained in the sample is bound to the substrate;
a second surface (stamp), to which a probe capable of binding to the anaylte is bound, is brought together with the substrate, so that the probe and the analyte can interact, wherein the bond of the probe to the analyte and the bond of the analyte to the substrate is more stable under an externally-applied tensile force than the bond of the probe to the stamp;
the stamp and the substrate are separated from one another; and
it is determined, whether the probe is bound to the substrate and/or how much probe is bound to the substrate.

2. Method according to claim 1,
**characterised in that**
molecules (receptors), which can bind the analyte, are immobilised on the substrate, wherein the bond of the analyte to the receptor is more stable under an externally-applied tensile force than the bond of the probe to the stamp.

3. Method according to claim 2,
**characterised in that**
the receptors and/or probe are selected from the group consisting of polyclonal or monoclonal antibodies, antibody fragments or antibody derivatives, which recognise and bind to the analyte.

4. Method according to claim 3,
**characterised in that**
antibody fragments or antibody derivatives are selected from Fv, Fab, Fab' or F(ab')₂-fragments, single-chain antibody fragments, bi-specific antibodies, chimeral antibodies, humanised antibodies and fragments, which contain CDRs (complementarity-determining regions), which recognise an epitope of the analyte, are used as receptors and/or probes.

5. Method according to claim 1 or claim 2,
**characterised in that**
nucleic acids such as DNA (desoxyribonucleic acid) or RNA (ribonucleic acid) and artificial nucleic acids such as LNA (locked nucleic acid) and PNA (peptide nucleic acid) and three-dimensional structures made of nucleic acids, preferably aptamers, are used as receptors and/or probes.

6. Method according to any one of the preceding claims,
**characterised in that**
the analyte is selected from the group consisting of proteins, polypeptides, peptides, nucleic acids, modified nucleic acids, molecules similar to nucleic acids, such as peptide nucleic acid (PNA) or locked nucleic acid (LNA), three-dimensional nucleic acid structures, aptamers, carbohydrates and modified variants thereof.

7. Method according to claim 6,
**characterised in that**
the analyte is a protein, polypeptide or peptide.

8. Method according to any one of the preceding claims,
**characterised in that**
the probe binds specifically to the analyte.

9. Method according to any one of the preceding claims,
**characterised in that**
the probe is selected from the group consisting of antibodies, antibody fragments and antibody derivatives.

10. Method according to any one of the preceding claims,
**characterised in that**
the probe provides a marking.

11. Method according to any one of the preceding claims,
**characterised in that**
the probe is immobilised on the stamp via a sensor complex.

12. Method according to claim 11,
**characterised in that**
the sensor complex comprises a first binding partner, . which is connected to the stamp and a second binding partner, which is firmly connected to the probe,
wherein the first binding partner and the second binding partner can form a non-covalent bond with one another.

13. Method according to claim 11 or claim 12,
**characterised in that**
the first binding partner and the second binding partner are nucleic acids.

14. Method according to claim 13,
**characterised in that**
the first binding partner and the second binding partner are selected from the group consisting of single-strand DNA and/or single-strand RNA.

15. Method according to claim 14,
**characterised in that**
the 5'-end of the first binding partner is connected to the stamp, and the 3'-end of the second binding partner is connected to the probe, or that the 3'-end of the first binding partner is connected to the stamp, and the 5'-end of the second binding partner is connected to the probe.

16. Method according to any one of the preceding claims,
**characterised in that**
the stamp provides several mutually-separated regions, in which probes with different specificity for different analytes are bound.

17. Method according to any one of claims 2 to 16,
**characterised in that**
the substrate provides several mutually-separated regions, in which receptors with different specificity for different analytes are bound.

18. Method according to claim 16 or 17,
**characterised in that**,
the substrate provides several mutually-separated regions, in which receptors with different specificity for different analytes are bound, in such a manner that they are symmetrical to the regions disposed on the stamp, so that when the stamp is brought together with the substrate, the respective regions of the probes and receptors, which are specific for the same analyte come to cover one another.

19. Method according to claim 16 or 17,
**characterised in that**
the substrate and also the stamp provide regions, in which receptors are immobilised and also regions, in which probes are immobilised, wherein the respective regions are arranged in such a manner that when the stamp and the substrate are brought into contact, regions with receptors for an analyte and regions with probes for the same analyte are disposed opposite to one another.

20. Method according to any one of the preceding claims,
**characterised in that**
the stamp and/or substrate provide surface portions, on which several spots are arranged.

21. Method according to claim 20,
**characterised in that**
the stamp and/or substrate provide surface portions with several spots, wherein the spots of one surface portion provide different receptors or probes each capable of binding to an analyte.

22. Method according to claim 20,
**characterised in that**
the stamp and/or the substrate provide at least one spot, on which receptors are immobilised, which are capable of binding directly to the probe, wherein the spots are used for controlling the coupling.

23. Device for determining an analyte in a sample comprising two surfaces, which each provide surface portions, to which receptors and probes capable of binding to different analytes are bound,
wherein the receptors and probes are disposed respectively in such a manner that, when the two surfaces are brought into contact, the receptors or respectively probes capable of binding to the same respective analyte are disposed opposite to one another,
wherein the respective probes are immobilised via a sensor complex and bear a marking,
wherein the sensor complex is a combination of at least two molecules capable of binding, which can be separated upon the application of a tensile force,
wherein the combination is selected in such a manner that the tensile force required to separate them is greater under the given experimental conditions than the tensile force required to separate non-specifically bound molecules, but less than the tensile force required to separate the specifically bound analyte from its binding partner or to separate the immobilised receptors.

24. Device according to claim 23,
**characterised in that**
the device comprises a stamp and a substrate and that a first binding partner is firmly connected to the stamp, and a second binding partner is firmly connected to the probe, wherein the first binding partner and the second binding partner can form a non-covalent bond with one another.

25. Device according to claim 24,
**characterised in that**
the first binding partner and the second binding partner are nucleic acids.

26. Device according to claim 25,
**characterised in that**
the first binding partner and the second binding partner are selected from the group consisting of single-strand DNA and/or single-strand RNA.

27. Device according to claim 26,
**characterised in that**
the 5'-end of the first binding partner is connected to the stamp, and the 3'-end of the second binding partner is connected to the probe, or that the 3'-end of the first binding partner is connected to the stamp, and the 5'-end of the second binding partner is connected to the probe.

28. Device according to any one of claims 23 to 27,
**characterised in that**
the device also comprises a unit for bringing together and separating the two surfaces.

29. Device according to any one of claims 23 to 28,
**characterised in that**
the device also provides a unit for detecting whether and/or in which quantity the probe is bound to the substrate.

30. Device according to claim 29,
**characterised in that**
the device provides a unit for detecting the marking.

31. Device according to any one of claims 23 to 30,
**characterised in that**
the stamp provides several mutually-separated regions, in which probes with different specificity for different analytes are bound.

32. Device according to any one of claims 23 to 31,
**characterised in that**
the substrate provides several mutually-separated regions, in which receptors with different specificity for different analytes are bound.

33. Device according to claim 31 or 32,
**characterised in that**
the substrate provides several mutually-separated regions, in which receptors with different specificity for different samples are bound, in such a manner that they are symmetrical to the regions disposed on the stamp, so that when the stamp and the substrate are brought together, the regions of the probes and receptors, which are respectively specific for the same sample cover one another.

34. Device according to any one of claims 23 to 33,
**characterised in that**
the regions each provide several spots, on which respectively different receptors and probes capable of binding to an analyte are arranged.

## Revendications

1. Procédé de détermination ou de dosage d'un analyte dans un échantillon, **caractérisé en ce que** l'on met en contact un échantillon, qui contient éventuellement l'analyte à déterminer, avec une première surface (= support), de sorte que l'analyte éventuellement présent dans l'échantillon se lie au support ; une deuxième surface (= sceau), sur laquelle une sonde qui peut se lier à l'analyte, est liée, est approchée du support, de sorte que la sonde et l'analyte peuvent interagir, où la liaison de la sonde à l'analyte et la liaison de l'analyte au support sont plus stables sous une force de traction appliquée de l'extérieur que la liaison de la sonde au sceau ; le sceau et le support sont séparés, et on détermine si la sonde est liée sur le support et/ou combien de sonde est liée au support.

2. Procédé selon la revendication 1, **caractérisé en ce que** sur le support, sont immobilisées des molécules, qui peuvent se lier à l'analyte (- récepteurs), où la liaison de l'analyte au récepteur est plus stable sous une force de traction appliquée de l'extérieur que la liaison de la sonde au sceau.

3. Procédé selon la revendication 2, **caractérisé en ce que** les récepteurs et/ou les sondes sont choisis parmi le groupe consistant en des anticorps polyclonaux ou monoclonaux, des fragments d'anticorps ou des dérivés d'anticorps, qui peuvent reconnaître et se lier à l'analyte.

4. Procédé selon la revendication 3, **caractérisé en ce que** l'on utilise comme récepteurs et/ou sondes, des fragments ou dérivés d'anticorps choisis parmi les fragments Fv, Fab, Fab' ou F(ab')₂, les « single chain antibody fragments » (fragments d'anticorps à chaîne unique), les anticorps bispécifiques, les anticorps chimères, les anticorps humanisés ainsi que des fragments, qui contiennent des CDR (complementary determining regions, régions déterminantes complémentaires), qui reconnaissent un épitope de l'analyte.

5. Procédé selon la revendication 1 ou 2, **caractérisé en ce que** l'on utilise comme récepteurs et/ou sondes, des acides nucléiques comme l'ADN (acide désoxyribonucléique) ou l'ARN (acide ribonucléique) et des acides nucléiques synthétiques comme le LNA (locked nucleic acid, acide nucléique bloqué) et le PNA (peptide nucleic acid, acide nucléique peptidique) et des structures tridimensionnelles d'acides nucléiques, de préférence des aptamères.

6. Procédé selon l'une des revendications précédentes, **caractérisé en ce que** l'analyte est choisi parmi le groupe consistant en des protéines, des polypeptides, des peptides, des acides nucléiques, des acides nucléiques modifiés, des molécules similaires aux acides nucléiques comme un peptide nucleic acid (PNA, acide nucléique peptidique) ou un locked nucleic acid (LNA, acide nucléique bloqué), des structures tridimensionnelles d'acides nucléiques, des aptamères, des hydrates de carbone et leurs variantes modifiées.

7. Procédé selon la revendication 6, **caractérisé en ce que** l'analyte est une protéine, un polypeptide ou un peptide.

8. Procédé selon l'une des revendications précédentes, **caractérisé en ce que** la sonde se lie spécifiquement à l'analyte.

9. Procédé selon l'une des revendications précédentes, **caractérisé en ce que** la sonde est choisie parmi le groupe consistant en des anticorps, des fragments d'anticorps et des dérivés d'anticorps.

10. Procédé selon l'une des revendications précédentes, **caractérisé en ce que** la sonde présente un marquage.

11. Procédé selon l'une des revendications précédentes, **caractérisé en ce que** la sonde est immobilisée sur le sceau par un complexe capteur.

12. Procédé selon la revendication 11, **caractérisé en ce que** le complexe capteur comprend un premier partenaire de liaison, qui est relié au sceau, et un deuxième partenaire de liaison, qui est solidement relié à la sonde, où le premier partenaire de liaison et le deuxième partenaire de liaison peuvent être liés l'un à l'autre de manière non covalente.

13. Procédé selon la revendication 11 ou 12, **caractérisé en ce que** le premier partenaire de liaison et le deuxième partenaire de liaison sont des acides nucléiques.

14. Procédé selon la revendication 13, **caractérisé en ce que** le premier partenaire de liaison et le deuxième partenaire de liaison sont choisis parmi le groupe consistant en un ADN simple brin et/ou un ARN simple brin.

15. Procédé selon la revendication 14, **caractérisé en ce que** l'extrémité 5' du premier partenaire de liaison est reliée au sceau et que l'extrémité 3' du deuxième partenaire de liaison est relié à la sonde ou que l'extrémité 3' du premier partenaire de liaison est reliée au sceau et que l'extrémité 5' du deuxième partenaire de liaison est relié à la sonde.

16. Procédé selon l'une des revendications précédentes, **caractérisé en ce que** le sceau présente plusieurs zones séparées les unes des autres, dans lesquelles des sondes avec une spécificité différente pour des analytes différents sont reliées.

17. Procédé selon l'une des revendications 2 à 16, **caractérisé en ce que** le support présente plusieurs zones séparées l'une de l'autre, dans lesquelles des récepteurs avec une spécificité différente pour des analytes différents sont reliés.

18. Procédé selon la revendication 16 ou 17, **caractérisé en ce que** le support présente plusieurs zones séparées les unes des autres, dans lesquelles des récepteurs avec une spécificité différente pour des analytes différents sont reliés, de sorte qu'elles sont symétriques aux zones se trouvant sur le sceau, pour que lors du rapprochement du sceau et du support, chaque fois les zones de sondes et de récepteurs, qui sont spécifiques pour le même analyte, arrivent à se superposer.

19. Procédé selon la revendication 16 ou 17, **caractérisé en ce que** non seulement le support, mais également le sceau présentent des zones, dans lesquelles les récepteurs sont immobilisés, mais également des zones, dans lesquelles les sondes sont immobilisées, où les zones sont agencées chaque fois de sorte que lors de la mise en contact du sceau et du support, les zones avec des récepteurs pour un analyte et les zones avec des sondes pour le même analyte sont opposées ou se font face.

20. Procédé selon l'une des revendications précédentes, **caractérisé en ce que** le sceau et/ou le support présentent des segments de surface, sur lesquels plusieurs taches sont disposées.

21. Procédé selon la revendication 20, **caractérisé en ce que** le sceau et/ou le support présentent des segments de surface avec plusieurs taches, où les taches d'un segment de surface présentent chaque fois, différents récepteurs ou sondes capables de liaison avec un analyte.

22. Procédé selon la revendication 20, **caractérisé en ce que** le sceau et/ou le support présentent au moins une tache, sur lequel des récepteurs sont immobilisés, qui sont capables de liaison directe avec la sonde, où ces taches sont utilisées pour le contrôle du couplage.

23. Dispositif de détermination ou de dosage d'un analyte dans un échantillon, comprenant deux surfaces, qui présentent chaque fois, des segments de surface, sur lesquels sont reliés des récepteurs ou sondes capables de liaison avec différents analytes, où les récepteurs et sondes sont agencés chaque fois, de sorte que lors de la mise en contact des deux surfaces, les récepteurs et respectivement, sondes, capables de liaison avec le même analyte, sont opposés ou se font face, les sondes étant immobilisées chaque fois, par un complexe capteur et portent un marquage, où le complexe capteur est une combinaison d'au moins deux molécules capables de liaison, qui peuvent être séparées lors de l'utilisation d'une force de traction, où la combinaison est choisie de sorte que la force de traction, qui est nécessaire pour leur séparation, dans des conditions déterminées de l'essai, est supérieure à la force de traction, qui est nécessaire pour la séparation des molécules liées de manière non spécifique, mais inférieure à la force de traction, qui est nécessaire pour la séparation des analytes liés spécifiquement depuis son partenaire de liaison ou pour la séparation du récepteur immobilisé.

24. Dispositif selon la revendication 23, **caractérisé en ce que** le dispositif comprend un sceau et un support et **en ce qu'**un premier partenaire de liaison est relié solidement au sceau et qu'une deuxième partenaire de liaison est relié solidement à la sonde, le premier partenaire de liaison et le deuxième partenaire de liaison pouvant être reliés l'un à l'autre de manière non covalente.

25. Dispositif selon la revendication 24, **caractérisé en ce que** le premier partenaire de liaison et le deuxième partenaire de liaison sont des acides nucléiques.

26. Dispositif selon la revendication 25, **caractérisé en ce que** le premier partenaire de liaison et le deuxième partenaire de liaison sont choisis parmi le groupe consistant en un ADN simple brin et/ou un ARN simple brin.

27. Dispositif selon la revendication 26, **caractérisé en ce que** l'extrémité 5' du premier partenaire de liaison est reliée au sceau et que l'extrémité 3' du deuxième partenaire de liaison est relié à la sonde ou que l'extrémité 3' du premier partenaire de liaison est reliée au sceau et que l'extrémité 5' du deuxième partenaire de liaison est relié à la sonde.

28. Dispositif selon l'une des revendications 23 à 27, **caractérisé en ce que** le dispositif comprend en outre, un mécanisme pour le rapprochement et la séparation des deux surfaces.

29. Dispositif selon l'une des revendications 23 à 28, **caractérisé en ce que** le dispositif comprend en outre, un mécanisme pour la détection de la liaison de la sonde au support et/ou de la quantité de liaison de la sonde au support.

30. Dispositif selon la revendication 29, **caractérisé en ce que** le dispositif présente un mécanisme pour la détection du marquage.

31. Dispositif selon l'une des revendications 23 à 30, **caractérisé en ce que** le sceau présente plusieurs zones séparées les unes des autres, dans lesquelles des sondes avec une spécificité différente pour différents analytes sont reliées.

32. Dispositif selon l'une des revendications 23 à 31, **caractérisé en ce que** le support présente plusieurs zones séparées les unes des autres, dans lesquelles des récepteurs avec une spécificité différente pour différents analytes sont reliés.

33. Dispositif selon l'une des revendications 31 ou 32, **caractérisé en ce que** le support présente plusieurs zones séparées les unes des autres, dans lesquelles des récepteurs avec une spécificité différente pour des analytes différents sont reliés, de sorte qu'elles sont symétriques aux zones se trouvant sur le sceau, pour que lors du rapprochement du sceau et du support, chaque fois les zones de sondes et de récepteurs, qui sont spécifiques pour le même analyte, arrivent à se superposer.

34. Dispositif selon l'une des revendications 23 à 33, **caractérisé en ce que** les zones présentent chaque fois, plusieurs taches, sur lesquelles sont disposés chaque fois, différents récepteurs et respectivement, sondes, capables de liaison avec un analyte.


### SEQUENCE LISTING

<110> nanotype
<120> Proteinchip
<130> NM5214
<140> PCT/EP02/06003
   <141> 2002-05-31
<160> 4
<170> Patentin version 3.1
<210> 1
   <211> 50
   <212> DNA
   <213> Artificial
<400> 1
   atacgccgta aagccggaga cagataagac gctacatgaa aaaaaaaaaa 50
<210> 2
   <211> 50
   <212> DNA
   <213> Artificial
<400> 2
   atacgccgta aagccggaga cagataagac gctacatgaa aaaaaaaaaa 50
<210> 3
   <211> 50
   <212> DNA
   <213> Artificial
<400> 3
   atacgccgta aagccggaga cagataagac gctacatgaa aaaaaaaaaa 50
<210> 4
   <211> 27
   <212> DNA
   <213> Artificial
<400> 4
   aaaaaaaaaa tctccggctt tacggcg 27